(19) Europäisches Patentamt
European Patent Office
Office européen des brevets

(11) EP 3 998 083 A1

(12) EUROPEAN PATENT APPLICATION
published in accordance with Art. 153(4) EPC

(43) Date of publication:
**18.05.2022 Bulletin 2022/20**

(21) Application number: **20839700.0**

(22) Date of filing: **10.07.2020**

(51) International Patent Classification (IPC):
***A61K 39/395*** *(2006.01)* ***A61P 35/00*** *(2006.01)*
***C07K 16/28*** *(2006.01)* ***C07K 16/46*** *(2006.01)*
***C12N 5/10*** *(2006.01)* ***C12N 1/15*** *(2006.01)*
***C12N 1/19*** *(2006.01)* ***C12N 1/21*** *(2006.01)*
***C12N 15/13*** *(2006.01)* ***C12N 15/63*** *(2006.01)*

(52) Cooperative Patent Classification (CPC):
**A61K 39/395; A61P 35/00; C07K 16/28; C07K 16/46; C12N 5/10; C12N 15/63**

(86) International application number:
**PCT/JP2020/027043**

(87) International publication number:
**WO 2021/010326 (21.01.2021 Gazette 2021/03)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **12.07.2019 JP 2019130236**

(71) Applicant: **CHUGAI SEIYAKU KABUSHIKI KAISHA**
**Tokyo 115-8543 (JP)**

(72) Inventors:
- **KAMIKAWAJI, Shogo**
  **Kamakura-shi, Kanagawa 247-8530 (JP)**
- **BABA, Takeshi**
  **Kamakura-shi, Kanagawa 247-8530 (JP)**
- **SANO, Yuji**
  **Kamakura-shi, Kanagawa 247-8530 (JP)**
- **NAKANISHI, Yoshito**
  **Kamakura-shi, Kanagawa 247-8530 (JP)**

(74) Representative: **Vossius & Partner**
**Patentanwälte Rechtsanwälte mbB**
**Siebertstraße 3**
**81675 München (DE)**

# (54) ANTI-MUTATION TYPE FGFR3 ANTIBODY AND USE THEREFOR

(57) The present disclosure provides antibodies capable of distinguishing between wild-type FGFR3 and FGFR3 S249C mutants. Using these antibodies, antigen-binding molecules capable of selectively damaging tumor cells expressing an FGFR3 S249C mutant were constructed.



Processed by Luminess, 75001 PARIS (FR)

**Description**

[Technical Field]

The present disclosure relates to anti-Fibroblast Growth Factor Receptor 3 (FGFR3) S249C mutant antibodies and their uses. The present disclosure also relates to bispecific antibodies against FGFR3 S249C mutants and CD3, and the uses of such antibodies, in particular, the use of such antibodies in the treatment of cancer.

[Background Art]

The statements in this section only provide background information regarding this disclosure and do not necessarily constitute prior art.

FGF and FGFR

The Fibroblast Growth Factor (FGFs) family and their receptors control many biological activities such as cell proliferation, cell migration, and cell differentiation. Although 22 types of FGF members have been reported in humans, only 18 types of these ligands bind to the receptors (NPL 1).

In humans, there are four types of single transmembrane receptor tyrosine kinases (FGFR1, FGFR2, FGFR3, FGFR4). The structure of these receptors is composed of three extracellular immunoglobulin regions, a transmembrane region and an intracellular tyrosine kinase region (NPL 1).

Two receptor molecules bind to two ligand molecules and heparan sulfate proteoglycan (HSPG) via the immunoglobulin region to form the FGF-FGFR complex. The ligand specificity of FGFR1, FGFR2, and FGFR3 is determined by alternative splicing in the C-terminal half of immunoglobulin region III, which is the FGF-binding region. This alternative splicing produces a IIIb isoform containing exon 8 and a IIIc isoform containing exon 9. FGF3, 7, 10, and 22 bind exclusively to the IIIb isoform, and FGF1 binds to both the IIIb and IIIc isoforms. On the other hand, the other 13 FGF ligands preferentially bind to the IIIc isoform.

When an FGF ligand binds to FGFR, the intracellular domain is phosphorylated as a result of receptor activation, and the intracellular tyrosine kinase region is activated. The FGFR signal is transmitted to the FGFR substrate (FRS2), the Growth factor receptor bound2 (Grb2) and Son Of Sevenless 1 (SOS) complex, and the MAPK pathway (NPL 2).

FGFR3 and its missense mutations

The FGFR3 gene is located on chromosome 4p16.3. Missense mutations in FGFR3 have been found in urothelial bladder cancer. Missense mutations discovered so far are R248C, S249C, G372C, S373C, Y375C, G382R/E, A393E, K562E/M/Q/T and the like. Of these, the mutation rate of S249C is the highest (NPL 3). The S249C-mutation is a mutation in the extracellular region. The cysteine residue translated by the S249C mutation forms a disulfide bond between the receptors and promotes ligand-independent activation (NPL 4).

When the FGFR3 S249C mutant protein was highly expressed in NIH3T3 cells or normal urothelial cells, the FGFR3 S249C mutant protein was constitutively phosphorylated (NPL 5).

The relationship between the FGFR3 S249C mutant and disease

The FGFR3 S249C mutant is also found in lung squamous cell carcinoma. Five (1.2%) among 503 Chinese lung squamous cell carcinoma samples had an S249C mutation (NPL 6).

The FGFR3 S249C mutant is also found in HPV-positive head and neck squamous cell carcinoma. In 120 head and neck squamous cell carcinoma samples (45% of which were HPV-positive), FGFR2 mutations and FGFR3 mutations including S249C were identified in 17.6%. On the other hand, no mutations in FGFR2 and FGFR3 were observed in the HPV-negative samples (NPL 7).

The FGFR3 S249C mutant is also found in cervical cancer. A comprehensive analysis of several studies showed that the FGFR3 S249C mutant was found in 6 cases (1.7%) among 349 cervical cancer samples (NPL 8).

The FGFR3 S249C mutant is also found in prostate cancer. A comprehensive analysis of several studies showed that the FGFR3 S249C mutant was found in seven cases (6.3%) among 112 prostate cancer samples (NPL 9).

Anti-FGFR3 antibody

A therapeutic antibody targeting FGFR3 known to the inventors is R3Mab, which is a neutralizing antibody that specifically binds to FGFR3 (PTLs 1 and 2). R3Mab suppresses FGF1-induced activation of FGFR3 and phosphorylation of the downstream molecules FGFR substrate 2 and MAPK. Additionally, R3Mab has antibody-dependent cellular cytotoxicity (ADCC) activity. R3MAb showed antitumor activity not only against wild-type FGFR3 strains such as RT112, but also against Ba/F3-FGFR3 S249C and UM-UC-14 tumors having the FGFR3 S249C mutation (NPL 10). A Phase I trial of R3Mab has been carried out, but there are no subsequent reports on development (NPL 11). B-701 is an FGFR3 monoclonal IgG1 antibody, which has an antagonistic action and does not cross-react with other FGFRs (FGFR1, FGFR2 or FGFR4). Development has been discontinued for multiple myeloma, there is no follow-up report on the Phase I study for solid tumors, and it is being developed as an indication for locally advanced or metastatic urothelial cell carcinoma (NPL 12).

Antibodies that selectively bind to the FGFR3 S249C mutant

Meanwhile, an attempt was made to obtain an antibody that does not react with wild-type FGFR3 expressed in normal tissues and selectively binds to tumor-specific FGFR3 S249C mutant (NPL 13), and hybridomas that do not bind to wild-type FGFR3 and exhibit selective binding activity to the FGFR3 S249C mutant were allegedly obtained in primary ELISA screening. However, it is described that these hybridomas were not stable during single cell cloning and expansion and lost the ability to selectively bind to the FGFR3 S249C mutant (NPL 13). So far, there are no reports of isolating selective antibody clones against the FGFR3 S249C mutant.

Multispecific antigen-binding molecules and bispecific antibodies

Multispecific antigen-binding molecules that bind to two or more antigens by one molecule are being studied. Of these, bispecific antibodies are antibodies to which binding activity against two different antigens (first antigen and second antigen) can be imparted by modifying a natural IgG type antibody (NPL 14). Bispecific antibodies are used not only as antibodies that neutralize two antigens by one molecule, but they can also be used as antibodies that enhance antitumor activity by cross-linking cells having cytotoxic activity and cancer cells.

T cell-redirecting antibodies

T cell-redirecting antibodies, which are antibodies whose antitumor effect is based on cytotoxicity in which T cells are recruited as effector cells, have been known since the 1980s as an example of bispecific antibodies (NPLs 15, 16 and 17). Unlike antibodies whose antitumor effect is based on ADCC, in which NK cells and macrophages are recruited as effector cells, T cell-redirecting antibodies are bispecific antibodies containing an antibody against any of the constituent subunits of the T cell receptor (TCR) complex on T cells, especially an antibody that binds to a CD3 epsilon chain, and an antibody that binds to an antigen on a target cancer cell. When a T cell-redirecting antibody binds to the CD3 epsilon chain and the cancer antigen at the same time, the T cell and the cancer cell are cross-linked, and as a result, an antitumor effect is exerted on the cancer cell due to the cytotoxic effect of the T cell.

Among tumor antigens for T cell-redirecting antibodies, it is important to select antigens that are expressed only in cancer cells, because if a tumor antigen is expressed in normal tissue, the normal tissue will get damaged and strong side effects will be induced.

[Citation List]

[Patent Literature]

[PTL 1] WO2010/111367
[PTL 2] WO2016/105503

[Non-Patent Literature]

[NPL 1] Saichaemchan et al., Current molecular medicine 2016;16:40-62.
[NPL 2] Kelleher et al., Carcinogenesis 2013;34:2198-2205.
[NPL 3] Hart et al., Molecular biology of the cell 2001;12:931-942.
[NPL 4] d'Avis et al., the molecular biology journal of the American Association for Cancer Research 1998;9:71-78.
[NPL 5] di Martino et al., 2009 Oncogene. 2009 Dec 3; 28(48): 4306-4316

[NPL 6] Wang et al., Oncotarget 2015;6:34300-34308.
[NPL 7] Seiwert et al., an official journal of the American Association for Cancer Research 2015;21:632-641.
[NPL 8] Rosty et al., Molecular cancer 2005;4:15.
[NPL 9] Hernandez et al., 2009 Mod Pathol. 2009 Jun;22(6):848-56. doi: 10.1038/modpathol.2009.46. Epub 2009 Apr 17
[NPL 10] Qing et al., The Journal of clinical investigation 2009;119:1216-1229.
[NPL 11] Oncotarget 2017 8(9) 16052-74.
[NPL 12] ClinicalTrials.gov NCT02401542
[NPL 13] Gorbenko, 2009 Hybridoma (Larchmt). 2009 Aug;28(4):295-300
[NPL 14] Kontermann, mAbs 2012;4:182-197.
[NPL 15] Mezzanzanica et al., International journal of cancer 1988;41:609-615.
[NPL 16] Staerz and Bevan, Proceedings of the National Academy of Sciences of the United States of America 1986;83:1453-1457.
[NPL 17] Staerz et al., Nature 1985;314:628-631.

[Summary of Invention]

[Technical Problem]

An objective of the present disclosure is to provide antibodies that selectively bind to a Fibroblast Growth Factor Receptor 3 (FGFR3) S249C mutant. More specifically, an objective of the present disclosure is to provide antibodies capable of distinguishing between wild-type FGFR3 and FGFR3 S249C mutant. Furthermore, it is also an objective of the present disclosure to construct antigen-binding molecules capable of selectively damaging tumor cells expressing an FGFR3 S249C mutant, based on antibodies that selectively bind to an FGFR3 S249C mutant.

[Solution to Problem]

In order to achieve the above-mentioned objectives, the present disclosers attempted to prepare a monoclonal antibody that selectively binds to a natural FGFR3 S249C mutant present on the cell membrane surface. Furthermore, they found that antigen-binding molecules comprising a domain of an obtained monoclonal antibody that binds to the FGFR3 S249C mutant and a domain that binds to the T cell receptor (TCR) complex exhibit selective cytotoxic activity against cancer cells expressing the FGFR3 S249C mutant, thereby completing the present disclosure.
Specifically, the present disclosure provides the following:
The present disclosure provides FGFR3 S249C mutant-binding molecules useful for the treatment or prevention of pathological conditions associated with the expression and/or activity of the FGFR3 S249C mutant, such as increased expression and/or activity, or undesired expression and/or activity. In some embodiments, the antigen-binding molecules of the present disclosure are used to treat tumors, cancers, and/or cell proliferative disorders.
In another embodiment, the anti-FGFR3 S249C mutant antibodies of the present disclosure are useful as reagents for the detection and/or isolation of the FGFR3 S249C mutant, such as for the detection of the FGFR3 S249C mutant in various tissues and cell types.
The present disclosure further provides methods for producing and using the FGFR3 S249C mutant-binding molecules, and polynucleotides encoding the FGFR3 S249C mutant-binding molecules.
[1] An isolated antigen-binding molecule having selective binding activity to an FGFR3 S249C mutant as compared with wild-type FGFR3.
[2] The multispecific antigen-binding molecule according to [1], wherein the selective binding activity is such that the binding of the antigen-binding molecule to an FGFR3 S249C mutant-expressing cell line is similar to or greater than the binding of the antigen-binding molecule to a wild-type FGFR3-expressing cell line when the antigen-binding molecule is added to the wild-type FGFR3-expressing cell line at a concentration at least 10 times higher than that added to the FGFR3 S249C mutant-expressing cell line, and wherein the expressing cell lines are cell lines each expressing wild-type FGFR3 or FGFR3 S249C mutant to the same extent.
[3] An isolated antigen-binding molecule, comprising any one of the following sets of six HVRs:

(a)

HVR-H1 containing the amino acid sequence of SEQ ID NO: 17,
HVR-H2 containing the amino acid sequence of SEQ ID NO: 18,

HVR-H3 containing the amino acid sequence of SEQ ID NO: 19,
HVR-L1 containing the amino acid sequence of SEQ ID NO: 20,
HVR-L2 containing the amino acid sequence of SEQ ID NO: 21, and
HVR-L3 containing the amino acid sequence of SEQ ID NO: 22;

(b)

HVR-H1 containing the amino acid sequence of SEQ ID NO: 23,
HVR-H2 containing the amino acid sequence of SEQ ID NO: 24,
HVR-H3 containing the amino acid sequence of SEQ ID NO: 25,
HVR-L1 containing the amino acid sequence of SEQ ID NO: 26,
HVR-L2 containing the amino acid sequence of SEQ ID NO: 27, and
HVR-L3 containing the amino acid sequence of SEQ ID NO: 28; or

(c)

HVR-H1 containing the amino acid sequence of SEQ ID NO: 29,
HVR-H2 containing the amino acid sequence of SEQ ID NO: 30,
HVR-H3 containing the amino acid sequence of SEQ ID NO: 31,
HVR-L1 containing the amino acid sequence of SEQ ID NO: 32,
HVR-L2 containing the amino acid sequence of SEQ ID NO: 33, and
HVR-L3 containing the amino acid sequence of SEQ ID NO: 34;

[4] The antigen-binding molecule according to [3], wherein the heavy chain variable region and the light chain variable region include a mouse, rabbit, humanized or human framework.
[5] The antigen-binding molecule according to [3](a), further comprising: a heavy chain variable domain framework FR1 comprising the amino acid sequence of SEQ ID NO: 35; a heavy chain variable domain framework FR2 comprising the amino acid sequence of SEQ ID NO: 36; a heavy chain variable domain framework FR3 comprising the amino acid sequence of SEQ ID No: 37; and a heavy chain variable domain framework FR4 comprising the amino acid sequence of SEQ ID NO: 38.
[6] The antigen-binding molecule according to [3](b), further comprising: a heavy chain variable domain framework FR1 comprising the amino acid sequence of SEQ ID NO: 39; a heavy chain variable domain framework FR2 comprising the amino acid sequence of SEQ ID NO: 40; a heavy chain variable domain framework FR3 comprising the amino acid sequence of SEQ ID No: 41; and a heavy chain variable domain framework FR4 comprising the amino acid sequence of SEQ ID NO: 42.
[7] The antigen-binding molecule according to [3](c), further comprising: a heavy chain variable domain framework FR1 comprising the amino acid sequence of SEQ ID NO: 43; a heavy chain variable domain framework FR2 comprising the amino acid sequence of SEQ ID NO: 44; a heavy chain variable domain framework FR3 comprising the amino acid sequence of SEQ ID No: 45; and a heavy chain variable domain framework FR4 comprising the amino acid sequence of SEQ ID NO: 46.
[8] The antigen-binding molecule according to [5], further comprising: a heavy chain variable domain framework FR1 comprising the amino acid sequence of SEQ ID NO: 47; a heavy chain variable domain framework FR2 comprising the amino acid sequence of SEQ ID NO: 48; a heavy chain variable domain framework FR3 comprising the amino acid sequence of SEQ ID No: 49; and a heavy chain variable domain framework FR4 comprising the amino acid sequence of SEQ ID NO: 50.
[9] The antigen-binding molecule according to [6], further comprising: a heavy chain variable domain framework FR1 comprising the amino acid sequence of SEQ ID NO: 51; a heavy chain variable domain framework FR2 comprising the amino acid sequence of SEQ ID NO: 52; a heavy chain variable domain framework FR3 comprising the amino acid sequence of SEQ ID No: 53; and a heavy chain variable domain framework FR4 comprising the amino acid sequence of SEQ ID NO: 54.
[10] The antigen-binding molecule according to [7], further comprising: a heavy chain variable domain framework FR1 comprising the amino acid sequence of SEQ ID NO:55; a heavy chain variable domain framework FR2 comprising the amino acid sequence of SEQ ID NO: 56; a heavy chain variable domain framework FR3 comprising the amino acid sequence of SEQ ID No: 57; and a heavy chain variable domain framework FR4 comprising the amino acid sequence of SEQ ID NO: 58.
[11] The antigen-binding molecule according to any one of [3]-[7], which comprises

(a) a VH sequence having at least 95% sequence identity to any one of the amino acid sequences of SEQ ID NO: 5, 7, and 9;
(b) a VL sequence having at least 95% sequence identity to any one of the amino acid sequences of SEQ ID NO: 6, 8 and 10; or
(c) a VH sequence of any one of SEQ ID NOs: 5, 7, and 9 and a VL sequence of any one of SEQ ID NOs: 6, 8, and 10.

[12] The antigen-binding molecule according to [1] to [10], wherein the binding to the FGFR3 S249C mutant competes with an antibody comprising the pair of SEQ ID NO: 5 and SEQ ID NO: 6, SEQ ID NO: 7 and SEQ ID NO: 8, or SEQ ID NO: 9 and SEQ ID NO: 10, or binds to the same epitope as an antibody comprising the pair of SEQ ID NO: 5 and SEQ ID NO: 6, SEQ ID NO: 7 and SEQ ID NO: 8, or SEQ ID NO: 9 and SEQ ID NO: 10.
[13] The antigen-binding molecule according to any one of [3] to [12] further comprising an antibody Fc region.
[14] The antigen-binding molecule according to [13], wherein the antibody Fc region contains an Fc region having at least one amino acid mutation in the Fc region of IgG1, IgG2, IgG3, and IgG4.
[15] The antigen-binding molecule according to any one of [3] to [14], which has cytotoxic activity.
[16] The antigen-binding molecule according to [15], which has an ADCC or CDC cytotoxic activity.
[17] The antigen-binding molecule according to any one of [3] to [12], which is (a) a monoclonal antibody; (b) a humanized or chimeric antibody; (c) a human antibody; (d) an IgG antibody; or (e) an antibody fragment.
[18] The antigen-binding molecule according to any one of [1] to [17], which is conjugated to a cytotoxic agent.
[19] An immunoconjugate comprising the antigen-binding molecule according to any one of [1] to [17] and a cytotoxic agent.
[20] The antigen-binding molecule according to [18], which has an internalizing activity.
[21] A pharmaceutical composition comprising the antigen-binding molecule of any one of [1] to [17] or the immunoconjugate of [19], and a pharmaceutically-acceptable carrier.
[22] The pharmaceutical composition according to [21], which is used for either cancer treatment or prevention, or both.
[23] The pharmaceutical composition according to [22], which is used for the treatment or prevention, or both, of bladder cancer, lung squamous cell carcinoma, head and neck cancer, or cervical cancer.
[24] Use of the antigen-binding molecule according to any one of [1] to [17], or the immunoconjugate of [19], in the manufacture of a medicament.
[25] Use of the antigen-binding molecule according to any one of [1] to [17], or the immunoconjugate of [19], in the manufacture of a medicament for either cancer treatment or prevention, or both.
[26] Use of an immunoconjugate of the antigen-binding molecule according to any one of [1] to [17], or the immunoconjugate of [19], in the manufacture of a medicament for the treatment or prevention, or both, of bladder cancer, lung squamous cell carcinoma, head and neck cancer or cervical cancer.
[27] A method of prevention or treatment, or prevention and treatment, comprising the step of administering to an individual having a cancer an effective amount of the antigen-binding molecule according to any one of [1] to [17], or the immunoconjugate of [19], used in the manufacture of a medicament.
[28] A method of prevention or treatment, or prevention and treatment, comprising the step of administering to an individual having bladder cancer, pulmonary squamous epithelial cancer, head and neck cancer or cervical cancer, an effective amount of the antigen-binding molecule according to any one of [1] to [17], or the immunoconjugate of [19], used in the manufacture of the medicament.
[29] A kit used for the treatment or prevention, or both, of cancer, which comprises at least the antigen-binding molecule according to any one of [1] to [17], or the immunoconjugate of [19], and instructions for use.
[30] A kit used for the treatment or prevention, or prevention and treatment, of bladder cancer, pulmonary squamous epithelial cancer, head and neck cancer or cervical cancer, comprising at least the antigen-binding molecule according to any one of [1] to [17], or the immunoconjugate of [19], and instructions for use.
[31] A method for detecting an FGFR3 S249C mutant from a biological sample, which comprises contacting the biological sample and the antigen-binding molecule according to any one of [1] to [17].
[32] The detection method of [32], which comprises contacting the biological sample with the antigen-binding molecule according to any one of [1] to [17] under a condition that allows the binding of the antigen-binding molecule according to any one of [1] to [17] and FGFR3 S249C mutant and the detection of a complex formed between the antigen-binding molecule according to any one of [1] to [17] and FGFR3 S249C mutant.
[33] A method for diagnosing whether or not a subject has cancer, which comprises contacting a biological sample and the antigen-binding molecule according to any one of [1] to [17] and a biological sample from the subject.
[34] The diagnostic method of [33], which comprises contacting the biological sample with the antigen-binding molecule according to any one of [1] to [17] under a condition that allows the binding of the antigen-binding molecule according to any one of [1] to [17] and FGFR3 S249C mutant and the detection of a complex formed between the antigen-binding molecule according to any one of [1] to [17] and FGFR3 S249C mutant.

[35] The method according to any one of [31] to [34], wherein the cancer is bladder cancer, lung squamous cell carcinoma, head and neck cancer or cervical cancer.
[36] A nucleic acid encoding the antigen-binding molecule according to any one of [3] to [17].
[37] A vector comprising the nucleic acid according to [36].
[38] A cell comprising the nucleic acid according to [36] or the vector according to [37].
[39] A method for producing an antibody, which comprises culturing the cells of [38] that produce the antibody.
[40] The method of [39], which comprises recovering the antibody from the cell culture.
[41] A multispecific antigen-binding molecule comprising: a first antigen-binding domain having selective binding activity to an FGFR3 S249C mutant; and a second antigen-binding domain having T cell receptor complex-binding activity.
[42] The multispecific antigen-binding molecule according to the above [41], wherein with regard to the selective binding activity, the binding affinity of the antigen-binding molecule to the FGFR3 S249C mutant-expressing cell line is similar to or greater than the binding affinity of the antigen-binding molecule to the wild-type FGFR3-expressing cell line, when the antigen-binding molecule is added to the wild-type FGFR3-expressing cell line at a concentration at least 10 times higher than that of the FGFR3 S249C mutant-expressing cell line, and wherein the expression cell line is a cell line that expresses each of the wild-type FGFR3 and FGFR3 S249C mutant to the same extent.
[43] A multispecific antigen-binding molecule, wherein the multispecific antigen-binding molecule is an isolated antigen-binding molecule comprising a first antigen-binding domain and a second antigen-binding domain, which comprises any one of:

(a)
the heavy chain variable region of the first antigen-binding domain of:

HVR-H1 containing the amino acid sequence of SEQ ID NO: 17,
HVR-H2 containing the amino acid sequence of SEQ ID NO: 18,
HVR-H3 containing the amino acid sequence of SEQ ID NO: 19,
and the light chain variable region of the first antigen-binding domain of:

HVR-L1 containing the amino acid sequence of SEQ ID NO: 20,
HVR-L2 containing the amino acid sequence of SEQ ID NO: 21 and
HVR-L3 containing the amino acid sequence of SEQ ID NO: 22;

(b)
the heavy chain variable region of the first antigen-binding domain of:

HVR-H1 containing the amino acid sequence of SEQ ID NO: 23,
HVR-H2 containing the amino acid sequence of SEQ ID NO: 24,
HVR-H3 containing the amino acid sequence of SEQ ID NO: 25,
And the light chain variable region of the first antigen-binding domain of:

HVR-L1 containing the amino acid sequence of SEQ ID NO: 26,
HVR-L2 containing the amino acid sequence of SEQ ID NO: 27, and
HVR-L3 containing the amino acid sequence of SEQ ID NO: 28; or

(c)
the heavy chain variable region of the first antigen-binding domain of:

HVR-H1 containing the amino acid sequence of SEQ ID NO: 29,
HVR-H2 containing the amino acid sequence of SEQ ID NO: 30
HVR-H3 containing the amino acid sequence of SEQ ID NO: 31
and the light chain variable region of the first antigen-binding domain of:

HVR-L1 containing the amino acid sequence of SEQ ID NO: 32,
HVR-L2 containing the amino acid sequence of SEQ ID NO: 33, and
HVR-L3 containing the amino acid sequence of SEQ ID NO: 34;
and which comprises:
the heavy chain variable region of the second antigen-binding domain of:

HVR-H1 containing the amino acid sequence of SEQ ID NO: 59,
HVR-H2 containing the amino acid sequence of SEQ ID NO: 60,
HVR-H3 containing the amino acid sequence of SEQ ID NO: 61,
and the light chain variable region of the second antigen-binding domain of:

HVR-L1 containing the amino acid sequence of SEQ ID NO: 62,
HVR-L2 containing the amino acid sequence of SEQ ID NO: 63, and
HVR-L3 containing the amino acid sequence of SEQ ID NO: 64.

[44] The multispecific antigen binding molecule of [43], wherein the heavy chain variable region and the light chain variable region of either or both of the first antigen-binding domain and the second antigen-binding domain include a mouse, rabbit, humanized or human framework.
[45] The antigen-binding molecule according to [43](a), further comprising: a heavy chain variable domain framework FR1 comprising the amino acid sequence of SEQ ID NO: 35; a heavy chain variable domain framework FR2 comprising the amino acid sequence of SEQ ID NO: 36; a heavy chain variable domain framework FR3 comprising the amino acid sequence of SEQ ID NO: 37; and a heavy chain variable domain framework FR4 comprising the amino acid sequence of SEQ ID NO: 38.
[46] The antigen-binding molecule according to [43](b), further comprising: a heavy chain variable domain framework FR1 comprising the amino acid sequence of SEQ ID NO: 39; a heavy chain variable domain framework FR2 comprising the amino acid sequence of SEQ ID NO: 40; a heavy chain variable domain framework FR3 comprising the amino acid sequence of SEQ ID NO: 41; and a heavy chain variable domain framework FR4 comprising the amino acid sequence of SEQ ID NO: 42.
[47] The antigen-binding molecule according to [43](b), further comprising: a heavy chain variable domain framework FR1 comprising the amino acid sequence of SEQ ID NO: 43; a heavy chain variable domain framework FR2 comprising the amino acid sequence of SEQ ID NO: 44; a heavy chain variable domain framework FR3 comprising the amino acid sequence of SEQ ID NO: 45; and a heavy chain variable domain framework FR4 comprising the amino acid sequence of SEQ ID NO: 46.
[48] A multispecific antigen-binding molecule which is an isolated antigen-binding molecule, wherein the heavy and light chain variable regions of the first antigen-binding domain comprise the amino acid sequences of SEQ ID NO: 5 and SEQ ID NO: 6, SEQ ID NO: 7 and SEQ ID NO: 8, or SEQ ID NO: 9 and SEQ ID NO: 10, and the heavy chain variable region and the light chain variable region of the second antigen-binding domain comprises the amino acid sequences of SEQ ID NO: 13 and SEQ ID NO: 15.
[49] The multi specific antigen-binding molecule of [48], wherein the first antigen-binding domain comprises

(a) a heavy chain variable (VH) domain comprising an amino acid sequence having at least 95% sequence identity to the amino acid sequence of SEQ ID NO: 5 and a light chain variable (VL) domain comprising an amino acid sequence having at least 95% sequence identity to the amino acid sequence of SEQ ID NO: 6, as well as a second antigen-binding domain comprising a heavy chain variable (VH) domain comprising an amino acid sequence having at least 95% sequence identity to the amino acid sequence of SEQ ID NO: 13, and a light chain variable (VL) domain comprising an amino acid sequence having at least 95% sequence identity to the amino acid sequence of SEQ ID NO:15,
(b) a VH domain and VL domain as shown in (a), as well as a second antigen-binding domain comprising a heavy chain variable (VH) domain comprising an amino acid sequence having at least 95% sequence identity to the amino acid sequence of SEQ ID NO: 13, and a light chain variable (VL) domain comprising an amino acid sequence having at least 95% sequence identity to the amino acid sequence of SEQ ID NO:15,
(c) a heavy chain variable (VH) domain comprising an amino acid sequence having at least 95% sequence identity to the amino acid sequence of SEQ ID NO: 7 and a light chain variable (VL) domain comprising an amino acid sequence having at least 95% sequence identity to the amino acid sequence of SEQ ID NO: 8, as well as a second antigen-binding domain comprising a heavy chain variable (VH) domain comprising an amino acid sequence having at least 95% sequence identity to the amino acid sequence of SEQ ID NO: 13, and a light chain variable (VL) domain comprising an amino acid sequence having at least 95% sequence identity to the amino acid sequence of SEQ ID NO:15,
(d) a VH domain and VL domain as shown in (c), as well as a second antigen-binding domain comprising a heavy chain variable (VH) domain comprising an amino acid sequence having at least 95% sequence identity to the amino acid sequence of SEQ ID NO: 13, and a light chain variable (VL) domain comprising an amino acid sequence having at least 95% sequence identity to the amino acid sequence of SEQ ID NO:15,
(e) a heavy chain variable (VH) domain comprising an amino acid sequence having at least 95% sequence identity to the amino acid sequence of SEQ ID NO: 9 and a light chain variable (VL) domain comprising an amino acid sequence having at least 95% sequence identity to the amino acid sequence of SEQ ID NO: 10, as well as a second antigen-binding domain comprising a heavy chain variable (VH) domain comprising an amino acid sequence having at least 95% sequence identity to the amino acid sequence of SEQ ID NO: 13, and a light chain variable (VL) domain comprising an amino acid sequence having at least 95% sequence identity to the amino acid sequence of SEQ ID NO:15, or

(f) a VH domain and VL domain as shown in (e), as well as a second antigen-binding domain comprising a heavy chain variable (VH) domain comprising an amino acid sequence having at least 95% sequence identity to the amino acid sequence of SEQ ID NO: 13, and a light chain variable (VL) domain comprising an amino acid sequence having at least 95% sequence identity to the amino acid sequence of SEQ ID NO:15.

[50] A multispecific antigen binding molecule, wherein the antigen-binding molecule comprises an anti-CD3 arm comprising (a) a VH domain comprising the amino acid sequence of SEQ ID NO: 13 and (b) a second antigen-binding domain comprising a VL domain comprising the amino acid sequence of SEQ ID NO: 15, and an anti-FGFR3 S249C mutant arm comprising (a) a VH domain comprising the amino acid sequence of SEQ ID NO: 5 and (b) a first antigen binding domain comprising the VL domain comprising the amino acid sequence of SEQ ID NO: 6.

[51] The multispecific antigen-binding molecule according to any one of [41] to [50], wherein the antigen-binding molecule has cytotoxic activity.

[52] The multispecific antigen-binding molecule according to [51], wherein the cytotoxic activity is T cell-dependent cytotoxic activity (TDCC).

[53] The multispecific antigen-binding molecule according to [51], wherein the cytotoxic activity is cytotoxic activity against a cell expressing the FGFR3 S249C mutant on the surface.

[54] The multispecific antigen-binding molecule according to any one of [41] to [53], wherein the T cell receptor complex-binding activity is the binding activity to a CD3 epsilon chain.

[55] The multispecific antigen binding molecule according to any one of [41] to [54], wherein the FGFR3 S249C mutant binding activity is the binding activity of the surface of eukaryotic cells to the FGFR3 S249C mutant.

[56] The multispecific antigen-binding molecule according to any one of [41] to [55], wherein the FGFR3 S249C mutant binding activity is human FGFR3 S249C mutant binding activity.

[57] The multispecific antigen-binding molecule according to any one of [41] to [56], wherein the first antigen-binding domain or the second antigen-binding domain is an antibody variable fragment (Fv), or the first antigen-binding domain and the second antibody-binding domain is an antibody variable fragment (Fv).

[58] The multispecific antigen-binding molecule according to any one of [41] to [57], wherein the first antigen-binding domain or the second antigen-binding domain is an antibody-binding fragment (Fab), or the first antigen-binding domain and the second antibody-binding domain are an antibody-binding fragment (Fab).

[59] The multispecific antigen-binding molecule according to any one of [41] to [58], further comprising an Fc region having reduced binding activity to Fcγ receptor.

[60] The multispecific antigen-binding molecule according to [59], which additionally comprises a domain consisting of an Fc region having reduced binding activity compared to the binding activity of a polypeptide having an Fc region of an IgG1, 2, 3 or 4 antibody (SEQ ID NO: 66, 67, 68, 69) to an Fcγ receptor.

[61] The multispecific antigen-binding molecule according to [59] and [60], which additionally comprises an Fc region having at least one amino acid mutation at the following positions in the EU numbering system, wherein the multiple antigen-binding molecule according to [59] and [60] has one or more mutations selected from the group of 220, 226, 229, 231, 232, 233, 234, 235, 236, 237, 238, 239, 240, 264, 265, 266, 267, 269, 270, 295, 296, 297, 298, 299, 300, 325, 327, 328, 329 , 330, 331, and 332, and wherein the mutant antibody binds to an extracellular domain via the potion that contains the above mutation(s).

[62] The multispecific antigen-binding molecule according to any one of [41] to [61], which is a bispecific antibody consisting of a first antibody variable fragment having FGFR3 S249C mutant-binding activity, a second antibody variable fragment having CD3 epsilon chain-binding activity, and an Fc region having reduced binding activity to FcyR.

[63] A nucleic acid encoding the multispecific antigen-binding molecule according to any one of [41] to [61].

[64] A vector into which the nucleic acid according to [63] has been introduced.

[65] A cell comprising the nucleic acid according to [63] or the vector according to [64].

[66] A method for producing the multispecific antigen-binding molecule according to any one of [41] to [62] by culturing the cells of [65].

[67] The multispecific antigen-binding molecule according to any one of [41] to [62] produced by the production method of [66].

[68] A pharmaceutical composition comprising the multispecific antigen-binding molecule according to any one of [41] to [62].

[69] A pharmaceutical composition for inducing cytotoxic activity, which comprises the multispecific antigen-binding molecule according to any one of [41] to [62].

[70] A pharmaceutical composition for either the treatment or prevention of cancer, or both, comprising the multispecific antigen-binding molecule according to any one of [41] to [62].
[71] The pharmaceutical composition according to [70], wherein the cancer is bladder cancer, lung squamous cell carcinoma, head and neck cancer, or cervical cancer.
[72] A method of either preventing or treating cancer, or both, wherein the multispecific antigen-binding molecule according to any one of [41] to [62] is administered to a patient in need thereof.
[73] The method according to [72], wherein the cancer according to [72] is bladder cancer, lung squamous cell carcinoma, head and neck cancer, or cervical cancer.

[Effects of the Invention]

The present disclosure provides antibodies capable of distinguishing an FGFR3 S249C mutant from wild-type FGFR3. In addition to being specifically expressed in cancer cells, the FGFR3 S249C mutant is also involved in cancer cell proliferation. Therefore, a high selectivity for cancer can be expected in cancer treatment strategies targeting the FGFR3 S249C mutant. In fact, it was confirmed that a bispecific antigen-binding molecule constructed using an antibody that binds to the FGFR3 S249C mutant obtained by the present disclosure has selective cytotoxicity for cells expressing the FGFR3 S249C mutant.

[Brief Description of Drawings]

FIG. 1 shows a measurement of the expression level of human FGFR3 in CHO cells forcibly expressing wild-type human FGFR3 (hFGFR3/CHO DXB11s) and CHO cells forcibly expressing human FGFR3 S249C mutant (hFGFR3 S249C/CHO DXB11s). It was confirmed that the expression levels of wild-type human FGFR3 and human FGFR3 S249C mutant on the cell surface in these cells were similar.
FIG. 2a shows a measurement of the binding activity of (a) monospecific human anti-FGFR3 S249C mutant antibodies (antibodies in which the two antigen-binding sites thereof bind to the same antigen; FGA0005 bivalent Ab and FGA0007 bivalent Ab), and (b) bispecific human anti-FGFR3 S249C mutant and CD3 antibodies (antibodies in which the left and right antigen binding sites bind to different antigens; FGA0005/CD3 TRAB and FGA0007/CD3 TRAB, one of which binds to the human FGFR3 S249C mutant, and the other binds to CD3) in hFGFR3/CHO DXB 11s and hFGFR3 S249C/CHO DXB11s cells. Both the monospecific and bispecific FGA0005 and FGA0007 antibodies showed a high binding activity to hFGFR3 S249C/CHO DXB11s as compared to hFGFR3/CHO DXB11s.
FIG. 2b shows the continuation of FIG. 2a.
FIG. 3 shows a measurement of the expression level of human FGFR3 on the cell surface in human lung cancer cell PC10 cell line (PC10), PC10 cells forcibly expressing wild-type human FGFR3 (PC10/FGFR3 WT), and PC10 cells forcibly expressing human FGFR3 S249C mutant (PC10/FGFR3 S249C). It was confirmed that the expression levels of human FGFR3 in PC10, PC10/FGFR3 WT and PC10/FGFR3 S249C were similar.
FIG. 4 shows a measurement of the binding activity of a bispecific antibody in which one arm binds to human FGFR3 S249C mutant and the other arm binds to CD3 (FGA0002/CD3-TRAB), and a bispecific antibody in which one arm does not bind to the human FGFR3 S249C mutant and the other arm binds to CD3 (KLH/CD3-TRAB), in PC10, PC10/FGFR3 WT, and PC10/FGFR3 S249C. FGA0002/CD3-TRAB and KLH/CD3-TRAB showed similar binding activity in PC10 and PC10/FGFR3 WT. On the other hand, in PC10/FGFR3 S249C, FGA0002/CD3-TRAB showed higher binding activity as compared to KLH/CD3-TRAB.
FIG. 5 shows a measurement of T cell-dependent cellular cytotoxicity (TDCC) activity of FGA0002/CD3-TRAB and KLH/CD3-TRAB in PC10, PC10/FGFR3 WT, and PC10/FGFR3 S249C using human peripheral blood mononuclear cells as effector cells. KLH/CD3-TRAB showed no TDCC activity in any of the cells, whereas FGA0002/CD3-TRAB showed TDCC activity only in PC10/FGFR3 S249C.

[Mode for Carrying Out the Invention]

I. Definitions

Unless otherwise defined, the technical and scientific terms used herein have the same meaning as commonly understood by one of ordinary skill in the art to which this disclosure belongs. Singleton et al., Dictionary of Microbiology and Molecular Biology 2nd ed., J. Wiley & Sons (New York, NY 1994), and March, Advanced Organic Chemistry Reactions, Mechanisms and Structure 4th ed., John Wiley & Sons (New York, NY 1992) will provide one of ordinary skill in the art with a general guidance for many of the terms used herein. All references cited herein, including patent applications and publications, are incorporated herein by reference in their entirety.

The following definitions apply for the purpose of interpreting this specification, and whenever applicable, terms used in the singular form also include the plural form and vice versa. It should be understood that the terms used herein are for the purpose of describing particular embodiments only and are not intended to be limiting. In the event that any of the definitions below conflicts with any document incorporated herein by reference, the below definitions shall prevail.

"Amino acids"

In this specification, amino acids are expressed in the one-letter notation, three-letter notation, or both. That is, Ala/A for alanine, Leu/L for leucine, Arg/R for arginine, Lys/K for lysine, Asn/N for aspartic acid, Met/M for methionine, Asp/D for asparagine, and Phe/F for phenylalanine, Cys/C for cysteine, Pro/P for proline, Gln/Q for glutamic acid, Ser/S for serine, Glu/E for glutamine, Thr/T for serine, Gly/G for glycine, Trp/W for tryptophan, His/H for histidine, Tyr/Y for tyrosine, Ile/I for isoleucine, and Val/V for valine.

"Fibroblast Growth Factor Receptor 3" and "FGFR3"

Unless otherwise specified, "fibroblast growth factor receptor 3" and "FGFR3" as used herein indicate any native FGFR3 polypeptide from any vertebrate source including mammals such as primates *(e.g.,* humans) and rodents *(e.g.,* mice and rats) (such as FGFR3-IIIb isoform (SEQ ID NO: 1) or FGFR3-IIIc isoform (SEQ ID NO: 65)).

"Natural sequences"

"Natural sequences" as used herein includes, in particular, naturally-occurring truncated forms (e.g., extracellular domain sequences or transmembrane subunit sequences), naturally-occurring variants (e.g., alternative splicing forms), and naturally-occurring allelic variants.

"Wild type FGFR3"

The "wild-type" proteins or portions thereof are the most common standard form in flora and fauna populations and are one version of the proteins found naturally. The wild type is in a normal state and has its original function. The amino acid sequence found in wild-type proteins, e.g., in normal tissues, is the amino acid sequence of the protein which is found naturally.

"Wild-type FGFR3" in the present specification generally refers to a polypeptide containing the amino acid sequence of a naturally-occurring FGFR3 protein. The term "wild-type FGFR3 sequence" generally refers to the amino acid sequence found in a naturally-occurring FGFR3, and includes full-length, untreated FGFR3, as well as any form of FGFR3 resulting from intracellular processing. The term also includes naturally-occurring variants of FGFR3, such as splice variants or allelic variants. An exemplary wild-type FGFR3 sequence of a human FGFR3 polypeptide comprises the amino acid sequences of P22607-1 (SEQ ID NO: 65) or P22607-2 (SEQ ID NO: 1) from the UniProt database.

"FGFR3 ligands"

An "FGFR3 ligand" (referred to interchangeably as "FGF") as used herein refers to any natural or mutant (even if its natural or synthetic) FGFR3 ligand (e.g., FGF1, FGF2, FGF4, FGF8, FGF9, FGF17, FGF18, FGF23) polypeptide, unless otherwise indicated specifically or contextually. The term "natural sequence" specifically refers to naturally-occurring truncated forms (e.g., extracellular domain sequences or transmembrane subunit sequences), naturally-occurring variants (e.g., alternative splice forms), and naturally-occurring allelic variants. The term "wild-type FGFR3 ligand" generally refers to a polypeptide comprising the amino acid sequence of a naturally-occurring FGFR3 ligand protein. The term "wild-type FGFR3 ligand sequence" generally refers to the amino acid sequence found in naturally-occurring FGFR3 ligands.

"FGFR3 activation"

"FGFR3 activation" in the present specification refers to activation or phosphorylation of an FGFR3 receptor. Normally, FGFR3 activation results in signal transduction (e.g., caused by the intracellular kinase domain of the FGFR3 receptor that phosphorylates the tyrosine residue of the FGFR3 receptor or substrate polypeptide). FGFR3 activation can be mediated by binding of the FGFR ligand to an FGFR3 receptor of interest. Binding of an FGFR3 ligand (e.g., FGF1 or FGF9) to FGFR3 activates the kinase domain of FGFR3, which phosphorylates the tyrosine residue of FGFR3 and/or the tyrosine residue of another substrate polypeptide (one or several).

"Constitutive"

Herein, "constitutive" when used, e.g., for receptor kinase activity, refers to the constitutive signal transduction activity of a receptor that is independent of the presence of a ligand or other activating molecule. Depending on the nature of the receptor, all activity may be constitutive, or the activity of the receptor may be further activated by the binding of another molecule (e.g., a ligand). Cellular phenomena that cause receptor activation are well known to those of skill in the art. For example, activation includes multimerization into higher order receptor complexes such as dimerization, trimerization and the like. The complex may comprise a single protein, *i.e.,* a homomeric complex. Alternatively, the complex may comprise at least two different protein species, *i.e.,* heteromeric complexes. For example, complex formation can result from overexpression of normal or mutant receptors on the surface of the cell. Complex formation can also be caused by specific mutations or multiple mutations within the receptor.

"Ligand independent"

Herein, "ligand-independent" when used, e.g., for receptor signal transduction activity, refers to signal activity that is independent of the presence of a ligand. Receptors with ligand-independent kinase activity do not necessarily prevent ligand binding to its receptor to produce further activation of kinase activity.

Herein, "ligand-dependent" when used, e.g., for receptor signal transduction activity, refers to signal transduction activity that depends on the presence of a ligand.

"FGFR3 antagonists" and "FGFR3 inhibitors"

As used herein, "FGFR3 antagonist" and "FGFR3 inhibitor" refer to any FGFR3 antagonist currently known in the art or will be identified in the future, and includes any chemical substance that results in inhibition of a biological activity through another method, where the biological activity is associated with activation of FGFR3 in patients including any of the downstream biological effects resulting from the binding of its native ligand to FGFR3 upon administration to a patient. Such FGFR3 antagonists include any agent capable of blocking either FGFR3 activation or the downstream biological effects of FGFR3 activation associated with the treatment of cancer in a patient. Such antagonists can act by binding directly to the intracellular domain of the receptor and inhibiting its kinase activity. Alternatively, such antagonists can act by occupying the ligand-binding site of the FGFR3 receptor or a portion thereof, thereby making the receptor inaccessible to its natural ligand and thus preventing or reducing its normal biological activity. Alternatively, such antagonists may act by dimerizing the FGFR3 polypeptide, or by regulating the interaction of the FGFR3 polypeptide with other proteins, or may enhance ubiquitination and endocytosis degradation of FGFR3. FGFR3 antagonists include, but are not limited to, small molecule inhibitors, antibodies or antibody fragments, antisense constructs, small inhibitory RNAs *(i.e.,* RNA interference by dsRNA; RNAi), and ribozymes. In a preferred embodiment, the FGFR3 antagonist is a small molecule or antibody that specifically binds to human FGFR3. An exemplary FGFR3 antagonist antibody is described, for example, in U.S. Pat. No. 8,410,250, which is incorporated herein by reference in its entirety. For example, U.S. Pat. No. 8,410,250 describes FGFR3 antagonist antibody clones 184.6, 184.6.1, and 184.6.1N54S (these clones are also referred to as "R3Mab").

"Mutations"

The term "mutation" means substitutions, deletions, additions or modifications of one or several to multiple amino acids, or any combination thereof. The production of an antibody containing a mutation can be done with the aim of acquiring any desired property, for example, reduction or enhancement and such of the binding to an Fc receptor, improvement of the pharmacokinetics of the antibody, reduction of heterogeneity, improvement of commercial productivity, or recognition by an antigen-binding molecule of the present disclosure. In addition, variants also include molecules altered by conservative substitutions, especially those commonly known, as long as they retain substantially the same function as the original sequence. Deletions and insertions of amino acid sequences include deletions and insertions of amino acids at the amino-terminal and/or carboxy-terminal. A spontaneous mutation of a particular amino acid is a "mutation" as used herein. Mutations also include substitutions with non-native amino acids or naturally-occurring amino acid derivatives of the 20 standard amino acids. Amino acid mutations can be generated using genetic or chemical methods well known in the art. Genetic methods include site-specific mutagenesis, PCR, gene synthesis and the like. In methods other than genetic engineering, chemical modifications such as chemical modifications may also be useful. The number of mutations may be at least one in a region specified in the present disclosure, and may include deletions and/or conservative substitutions of up to 50, 40, 30, 20, 10, and 5 amino acids in other regions. Further, in the present disclosure, a "mutation" includes a "alteration" that intentionally introduces a mutation regardless of whether or not the property is acquired.

"FGFR3 S249C mutant"

"FGFR3 S249C mutant" in the present specification refers to a mutant FGFR3 which has an amino acid sequence different from that of wild-type FGFR3, where the serine at position 249 in the wild-type human FGFR3 precursor (including the secretory signal of 1-22 within the 806 residues) that is referred to by UNIPROT registration number P22607 (SEQ ID NO: 1 or SEQ ID NO: 65) is mutated to cysteine. For example, the amino acid sequence shown in SEQ ID NO: 3 is an example of 806 amino acid residues constituting a precursor of "FGFR3 S249C mutant". In the present disclosure, in addition to S249C, an "FGFR3 S249C mutant" can contain an amino acid sequence different from the amino acid sequence of a wild-type human FGFR3 precursor.

"Anti-FGFR3 S249C mutant antibody"

The "anti-FGFR3 S249C mutant antibody" or "antibody that binds to an FGFR3 S249C mutant" in the present specification is an antibody capable of binding to an FGFR3 S249C mutant with sufficient affinity, and as a result, it implies an antibody useful as a diagnostic and/or therapeutic agent when targeting an FGFR3 S249C mutant. In a particular embodiment, the anti-FGFR3 S249C mutant antibody may be one that binds to an epitope of an FGFR3 S249C mutant conserved between FGFR3 S249C mutants of different species.

Binding activity

The binding activity of an antibody to a specific antigen may also be expressed as the kd of the antibody. The kd of an antibody refers to the dissociation rate constant of an antibody for a particular antigen and is expressed in units of the reciprocal of seconds *(i.e.,* sec-1). The larger the kd value, the weaker the binding of the antibody to the antigen.

Wild-type FGFR3 is also expressed in normal tissues, but the FGFR3 S249C mutant is expressed specifically in cancer. If an antibody that binds to the vicinity of the mutation site sequence or an antibody that selectively binds to the altered structure of the FGFR3 protein caused by the formation of a disulfide bond due to the S249C mutation can be obtained, it would be ideal as an antibody arm for constructing a T cell-redirecting antibody.

KD value and kd value

The KD and Kd values of the present specification can be determined using a biosensor based on surface plasmon resonance to characterize antibody-antigen interactions. The KD and kd values can be determined at 25 °C or 37 °C.

"Selective"

As used herein, "selectivity" or "selective" refers to different binding abilities to wild-type and mutant proteins. In one embodiment, for the degree of binding of an anti-FGFR3 S249C mutant antibody to an FGFR3 S249C mutant protein and a wild-type FGFR3 protein, the term means that these binding activities of the antibody are comparable or higher than that when the antibody is added to cell lines expressing mutant and wild-type FGFR3 at the same level at concentrations differing by at least 10 times, 100 times, or more. For example, when the value indicating the binding activity of the antibody added to the mutant cell line at 0.1 μg/mL is equal to or is greater than the value indicating the binding activity of the antibody added to the wild-type cell line at least 1 μg/mL or 100 μg/mL, it means that the antibody "shows selectivity for the mutant over the wild-type".

Species-conserved anti-FGFR3 S249C mutant antibody

In a certain embodiment, the anti-FGFR3 S249C mutant antibody of the present disclosure binds to FGFR3 S249C mutants from multiple species. In a further embodiment, the anti-FGFR3 S249C mutant antibody binds to an FGFR3 S249C mutant derived from eukaryotic cells. A "eukaryotic cell" is a cell with a clear nucleus surrounded by a nuclear envelope, and it can be said that cells of all living organisms belong to this, except for bacteria and cyanobacteria, which are prokaryotic cells. In a further embodiment, the anti-FGFR3 S249C mutant antibody binds to FGFR3 S249C mutants from human and non-human animals. In a further embodiment, the anti-FGFR3 S249C mutant antibody binds to FGFR3 S249C mutants derived from humans, mice, and monkeys (e.g., cynomolgus monkeys, rhesus monkeys, marmosets, chimpanzees, or baboons).

In a particular embodiment, the anti-FGFR3 S249C mutant antibody of the present disclosure has low binding activity to wild-type FGFR3 derived from multiple species. In a further embodiment, the anti-FGFR3 S249C mutant antibody has low binding activity to wild-type FGFR3 derived from human and non-human animals. In a further embodiment, the anti-FGFR3 S249C mutant antibody has low binding activity to wild-type FGFR3 derived from humans, mice, and monkeys.

In a particular embodiment, the anti-FGFR3 S249C mutant antibody of the present disclosure forms an immune complex *(i.e.,* an antigen-antibody complex) with an FGFR3 S249C mutant.

Epitope

"Epitope" in the present specification means a site on an antigen to which an antigen-binding domain in an antigen-binding molecule disclosed in the present specification binds. Thus, for example, an epitope can be defined by its structure. The epitope can also be defined by the binding activity to the antigen in the antigen-binding molecule to which the epitope binds. When the antigen is a peptide or polypeptide, it is also possible to identify the epitope by the amino acid residues that make up the epitope. When the epitope is a sugar chain, it is also possible to specify the epitope by a specific sugar chain structure.

A linear epitope is an epitope containing an epitope to which an amino acid primary sequence is bound. Linear epitopes typically contain at least 3, and most usually at least 5, for example, about 8 to about 10, 6 to 20 amino acids in a unique sequence.

In contrast to the linear epitope, "conformational epitope" is an epitope in which the primary amino acid sequence containing the epitope is not the only determinant of the bound epitope (for example, the primary amino acid sequence of a conformational epitope is not necessarily bound by an epitope-defining antibody). Conformational epitopes may contain a greater number of amino acids compared to linear epitopes. As for the binding to a conformational epitope, an antibody binds to the three-dimensional structure of a peptide or protein. For example, when a protein molecule folds and forms a three-dimensional structure, amino acids and/or polypeptide main chains that form the conformational epitope become aligned, and makes it possible for the antibody to bind to the epitope. Methods for determining epitope conformations include, for example, X ray crystallography, two-dimensional nuclear magnetic resonance spectroscopy, site-specific spin labeling, and electron paramagnetic resonance spectroscopy, but are not limited thereto. See, for example, Epitope Mapping Protocols in Methods in Molecular Biology (1996), Vol. 66, Morris (ed.).

Competing

Whether an antibody binds to the same epitope as an anti-FGFR3 S249C mutant antibody of the present disclosure, or competes for the binding, can be readily determined by using conventional methods known in the art. For example, an antibody of the present disclosure is bound to an FGFR3 S249C mutant protein or peptide under saturated conditions. Next, the ability of the test antibody to bind to the FGFR3 S249C mutant is evaluated. If the test antibody is capable of binding to the FGFR3 S249C mutant after saturation binding with the anti-FGFR3 S249C mutant antibody of the present disclosure, then it can be concluded that the test antibody binds to an epitope different from the anti-FGFR3 S249C mutant antibody of the present disclosure. On the other hand, if the test antibody cannot bind to the FGFR3 S249C mutant after saturation binding with the anti-FGFR3 S249C mutant antibody of the present disclosure, the test antibody possibly binds to the same epitope as the epitope bound by the anti-FGFR3 S249C mutant antibody of the present disclosure. Therefore, further routine experiments (e.g., peptide mutation and binding analysis) are performed to see if the test antibody binding was not observed due to actual binding to the same epitope as the antibody of the present disclosure, or due to steric hindrance (or another phenomenon).

This type of experiment can be performed using ELISA, RIA, Biacore, flow cytometry or any other quantitative or qualitative antibody binding assay available in the art. According to a particular embodiment of the disclosure, when one antibody present at an excess of 1-fold, 5-folds, 10-folds, 20-folds or 100-folds inhibits the other binding by at least 50%, although preferably 75%, 90% or even 99%, as measured by a competitive binding assay, the two antibodies are bound to the same (or overlapping) epitope (e.g., Junghans et al., Cancer Res. 1990: 50: See 1495-1502). Alternatively, two antibodies are considered to bind to the same epitope if essentially all amino acid mutations in an antigen that reduce or eliminate binding of one antibody, reduce or eliminate binding of the other. If only a subset of amino acid mutations that reduce or eliminate the binding of one antibody reduces or eliminates the other binding, the two antibodies are considered to have "overlapping epitopes".

The above binding methodology can be implemented with one of two strategies to determine whether antibody binding competes with an anti-FGFR3 S249C mutant antibody of the present disclosure. In the first strategy, the antibody of the present disclosure is bound to an FGFR3 S249C mutant under saturated conditions, and then the binding of the test antibody to the FGFR3 S249C mutant is evaluated. The second strategy is to evaluate the binding of an antibody of the present disclosure to the FGFR3 S249C mutant after binding the test antibody to the FGFR3 S249C mutant under saturated conditions. If either strategy allows only the first (saturated) antibody to bind to the FGFR3 S249C mutant, it is concluded that the test antibody and the antibody of the present disclosure compete for binding to the FGFR3 S249C mutant. An antibody that competes with an antibody of the present disclosure for binding does not necessarily have to bind to the same epitope as the antibody of the present disclosure, but by binding overlapping or flanking epitopes, it may sterically interfere with the binding of the antibody of the present disclosure.

"Antigen-binding molecules"

The "antigen-binding molecules" in the present specification are not particularly limited as long as they are molecules containing an antigen-binding domain of the present disclosure, and may further comprise even a peptide or protein having a length of about 5 amino acids or more. They are not limited to peptides or proteins derived from an organism, and may, for example, be polypeptides consisting of an artificially designed sequence. Further, they may be any of natural polypeptides, synthetic polypeptides, recombinant polypeptides and the like.

Although not limited to these, an "antigen-binding molecule" refers to an antibody, an antibody fragment, or a molecule formed by an antibody fragment, as exemplified by a bispecific antibody or a diabody, or chimeric antigen receptor (also referred to as "CAR"), and can also include multispecific binding molecules (bispecific diabodies, bispecific antibodies).

"Multiple specificity"

As used herein, the term "(antigen) specificity" refers to the binding specificity of an immune entity with its binding partner (e.g., antigen), and it refers to the property of binding to a specific binding partner, but not binding to other binding partners, or binding to other partners with a low affinity.

The multispecific antigen-binding molecules described herein include a first antigen-binding site and a second antigen-binding site that can specifically bind to at least two different antigens.

When an "antigen-binding molecule" in the present specification is administered to a human, a gene-recombinant antibody-derived domain that has been artificially altered for the purpose of reducing the heterologous antigenicity to humans, or such, can be suitably adopted as the antigen-binding domain in the antigen-binding molecule. Gene-recombinant antibodies include, for example, humanized antibodies and the like. These altered antibodies are appropriately produced using known methods.

"Antigen binding domain"

The term "antigen-binding domain" in the present specification refers to a part of an antigen-binding molecule that binds to only a part (epitope) of an antigen when the antigen-binding molecule binds to an antigen.

The structure of the antigen-binding domain that binds to an epitope is called a paratope. The epitope and paratope are stably bound by hydrogen bonds, electrostatic forces, Van der Waals forces, hydrophobic bonds, etc., which act between the epitope and the paratope. The binding force between the epitope and the paratope is called affinity. The sum of the binding forces when a plurality of antigens and a plurality of antigen-binding molecules bind to each other is called avidity. When an antibody or the like containing a plurality of antigen-binding domains (that is, polyvalent) binds to a plurality of epitopes, the avidity is higher than the affinity because the binding forces (affinity) act synergistically.

"Antibody fragment"

The term "antibody fragment" in the present specification refers to a molecule other than the complete antibody, which contains a part of the complete antibody that binds to the antigen to which the complete antibody binds. Examples of antibody fragments include, without limitation, Fv (also referred to as "antibody variable fragment"), Fab (also referred to as "antigen binding fragment"), Fab', Fab'-SH, F(ab')2, and single chain antibody molecules (e.g., scFv).

"Antibody"

The term "antibody" is used herein in the broadest sense as long it shows a desired antigen-binding activity, and encompasses without limitation, various antibody structures such as monoclonal antibodies, polyclonal antibodies, multi-specific antibodies (for example, bispecific antibodies), and antibody fragments.

"Isolated" antibody

The term "isolated" antibody as used herein is one that is isolated from its original environmental components. In some embodiments, the antibody is measured, for example, by electrophoresis (e.g., SDS-PAGE, isoelectric focusing (IEF), capillary electrophoresis) or chromatography (e.g., ion exchange or reverse phase HPLC), and purified to a purity of more than 95% or 99%. See, for example, Flatman et al., J. Chromatogr. See B 848: 79-87 (2007) as a review of methods for assessing antibody purity.

"Monoclonal antibody"

The term "monoclonal antibody" in the present specification refers to an antibody obtained from a substantially homogenous population of antibodies. That is, the individual antibodies that make up the population are identical and/or bind to the same epitope, except for possible mutant antibodies (e.g., mutant antibodies containing naturally-occurring mutations, or mutant antibodies that occur during the production of monoclonal antibody preparations). In contrast to polyclonal antibody preparations, which typically contain different antibodies against different determinants (epitopes), each monoclonal antibody in the monoclonal antibody preparation is directed to a single determinant on the antigen. Therefore, the modifier "monoclonal" indicates the antibody characteristic that it is obtained from a substantially homogeneous population of antibodies, and should not be construed as requiring production of the antibody by any particular method. For example, the monoclonal antibodies used according to the present disclosure may be prepared by various methods such as the hybridoma method, the recombinant DNA method, the phage display method, and methods using transgenic animals containing all or part of the human immunoglobulin locus, but are not limited thereto. Such methods and other exemplary methods for preparing monoclonal antibodies are described herein.

"Human antibody"

As used herein, the term "human antibody" refers to an antibody produced by a human or human cell, or an antibody having an amino acid sequence corresponding to the amino acid sequence of an antibody derived from a non-human source using a human antibody repertoire or another human antibody coding sequence. This definition of human antibody clearly excludes humanized antibodies that contain non-human antigen-binding residues.

The "human consensus framework" is a framework that indicates the most commonly occurring amino acid residues in the selection group of human immunoglobulin VL or VH framework sequences. Usually, the selection of human immunoglobulin VL or VH sequences is from a subgroup of variable domain sequences. Usually, the sequence subgroup is a subgroup in Kabat et al., Sequences of Proteins of Immunological Interest, Fifth Edition, NIH Publication 91-3242, Bethesda MD (1991), vols. 1-3. In one embodiment, for VL, the subgroup is the subgroup κI by Kabat et al. above. In one embodiment, for VH, the subgroup is subgroup III by Kabat et al., above.

"Humanized" antibody

The term "humanized" antibody in the present specification refers to a chimeric antibody containing amino acid residues from a non-human HVR and amino acid residues from a human FR. In a certain embodiment, the humanized antibody comprises substantially all of at least one, typically two variable domains, in which all or substantially all HVRs *(e.g.,* CDRs) correspond to those of nonhuman antibodies, and all or substantially all FRs correspond to those of human antibodies. The humanized antibody may optionally include at least a portion of the antibody constant region derived from a human antibody. The "humanized form" of an antibody (e.g., a nonhuman antibody) refers to an antibody that has undergone humanization.

"Chimeric" antibody

A "chimeric" antibody as used herein refers to an antibody where a portion of a heavy chain and/or light chain is derived from a particular source or species, while the rest of the heavy chain and/or light chain is derived from a different source or species.

"Full-length antibody", "complete antibody", and "whole antibody"

The terms "full-length antibody", "complete antibody," and "whole antibody" as used herein are used interchangeably, and refer to an antibody having a structure substantially similar to that of a native antibody or an antibody having a heavy chain containing an Fc region as defined herein.

"Variable region" or "VR"

As used herein, the term "variable region", "variable domain," or "VR" refers to the heavy or light chain domain of an antibody that is involved in the binding of the antibody to an antigen. The heavy and light chain variable domains of native antibodies (VH and VL, respectively) are similar in structure, with each domain usually containing four conserved framework regions (FR) and three hypervariable regions (HVR) (see, for example, Kindt et al., Kuby Immunology, 6th ed., W.H. Freeman and Co., page 91, (2007)). One VH or VL domain will be sufficient to confer antigen binding specificity. In addition, antibodies that bind to a particular antigen may be isolated by screening a complementary library of VL or VH domains using the VH or VL domains from the antibody that binds to that antigen. See, for example, Portolano et al., J. Immunol. 150: 880-887 (1993); Clarkson et al., Nature 352: 624-628 (1991).

"Hypervariable region" or "HVR"

As used herein, the term "hypervariable regions" or "HVRs" refers to each region of a variable domain of an antibody having at least one characteristic selected from the group consisting of: being hypervariable in sequence ("complementarity determining region" or "CDR"), forming a structurally-defined loop ("hypervariable loop"), and including an antigen contact residue ("antigen contact"). Usually, an antibody comprises 6 HVRs: 3 for VH (HI, H2, H3) and 3 for VL (L1, L2, L3). Illustrative HVRs of the present specification include:

(a) A hypervariable loop occurring at amino acid residues 26-32 (L1), 50-52 (L2), 91-96 (L3), 26-32 (H1), 53-55 (H2), and 96-101 (H3) (Chothia and Lesk, J. Mol. Biol. 196: 901-917 (1987));
(b) A CDR occurring at amino acid residues 24-34 (L1), 50-56 (L2), 89-97 (L3), 31-35b (H1), 50-65 (H2), and 95-102 (H3) (Kabat et al., Sequences of Proteins of Immunological Interest, 5th Ed. Public Health Service, National Institutes of Health, Bethesda, MD (1991);
(c) An antigen contact occurring at amino acid residues 27c-36 (L1), 46-55 (L2), 89-96 (L3), 30-35b (H1), 47-58 (H2), and 93-101 (H3) (MacCallum et al. J. Mol. Biol. 262: 732-745 (1996)); and
(d) A combination with at least one selected from the group consisting of (a), (b), and (c), including HVR amino acid residues 46-56 (L2), 47-56 (L2), 48-56 (L2), 49-56 (L2), 26-35 (H1), 26-35b (H1), 49-65 (H2), 93-102 (H3), and 94-102 (H3).

Unless otherwise indicated, HVR residues and other residues in the variable domain (e.g., FR residues) are numbered herein according to Kabat et al. above.

"Framework" or "FR"

"Framework" or "FR" refers to variable domain residues other than hypervariable region (HVR) residues. The variable domain FR usually consists of four FR domains: FR1, FR2, FR3, and FR4. Correspondingly, the sequences of HVR and FR usually appear in VH (or VL) in the following order from the N-terminal side: FR1-H1(L1)-FR2-H2(L2)-FR3-H3(L3)-FR4.

"Percent (%) amino acid sequence identity"

"Percent (%) amino acid sequence identity" as used herein with respect to a reference polypeptide sequence is defined as a percentage of amino acid residues in the candidate sequence that are identical to the amino acid residues in the reference polypeptide sequence, after aligning the sequences for maximum percent sequence identity and, if necessary, introducing a gap, and not considering any conservative substitutions as part of the sequence identity. The alignment for the purpose of determining the percent amino acid sequence identity can be achieved by using various methods within the scope of technology in the art such as BLAST, BLAST-2, ALIGN, and publicly-available computer software such as Megalign (DNASTAR) software.

One of skill in the art can determine the appropriate parameters for sequence alignment, including any algorithm required to achieve maximum alignment over the overall length of the sequences being compared. However, for the purposes herein, percent amino acid sequence identity values are generated using the sequence comparison computer program ALIGN-2. The ALIGN-2 sequence comparison computer program is the work of Genetec, and its source code has been submitted to the United States Copyright Office (US Copyright Office, Washington D.C., 20559) together with user documents and registered under the US copyright registration number TXU510087. The ALIGN-2 program is publicly available from Genentech, Inc. (South San Francisco, California), or may be compiled from the source code. The ALIGN-2 program is compiled for use on UNIX operating systems, including Digital UNIX V4.0D. All sequence comparison parameters are set by the ALIGN-2 program and do not fluctuate.

In situations where ALIGN-2 is used for amino acid sequence comparison, percent amino acid sequence identity of a given amino acid sequence A to a given amino acid sequence B, or with it, or against it (can also be said as a given amino acid sequence A, which comprises or contains a certain percent amino acid sequence identity to, a given amino acid sequence B, or with it, or against it) is calculated as follows: 100 times the fraction X/Y. Here, X is the number of amino acid residues scored by the sequence alignment program ALIGN-2 as the same matches in the alignment of A and B in the program, and Y is the total number of amino acid residues in B. It is understood that if the length of the amino acid sequence A is not equal to the length of the amino acid sequence B, then the percent amino acid sequence identity of A to B is not equal to the percent amino acid sequence identity of B to A. Unless otherwise stated, all percent amino acid sequence identity values used herein are obtained using the ALIGN-2 computer program as described in the previous paragraph.

"Fc region"

The term "Fc region" in the present specification is used to define the C-terminal region of an immunoglobulin heavy chain containing at least a part of a constant region. The term includes the Fc regions of native sequences and mutant Fc regions. In one embodiment, the human IgG heavy chain Fc region extends from Cys226 or from Pro230 to the carboxyl end of the heavy chain. However, the C-terminal lysine (Lys447) of the Fc region may or may not be present. Unless otherwise specified herein, the numbering of amino acid residues in the Fc region or constant region is described according to the EU numbering system (also called EU index) described in Kabat et al., Sequences of Proteins of Immunological Interest, 5th Ed., Public Health Service, National Institutes of Health, Bethesda, MD 1991.
"Mutant Fc region"
The term "mutant Fc region" in the present specification includes amino acid sequences different from that of natural sequence Fc regions by at least one amino acid modification (alteration), preferably one or more amino acid substitutions. Preferably, the mutant Fc region has, compared to the native sequence Fc region or the parent polypeptide Fc region, at least one amino acid substitution in the Fc region of the native sequence Fc region or in the Fc region of the parent peptide. For example, it has about 1 to about 10 amino acid substitutions, preferably about 1 to about 5 amino acid substitutions. The mutant Fc regions herein are preferably at least about 80% homologous to the native sequence Fc region and/or the Fc region of the parent polypeptide, most preferably has at least about 90% homology, more preferably at least about 95% homology with them.
The "mutant Fc region" of an antigen-binding molecule described herein includes an Fc region among the amino acids constituting the Fc region of IgG1, IgG2, IgG3 or IgG4, in which the Fcγ receptor-binding activity is reduced.
There are five major classes of antibodies: IgA, IgD, IgE, IgG, and IgM. Some of them may be further divided into subclasses (isotypes). The antibody isotype is determined by the structure of the constant region. The constant regions of the IgG1, IgG2, IgG3, and IgG4 isotypes are called Cγ1, Cγ2, Cγ3, and Cγ4, respectively. The amino acid sequences of the polypeptides constituting the Fc region of human Cγ1, Cγ2, Cγ3 and Cγ4 are exemplified in SEQ ID NOs: 66, 67, 68 and 69.
The Fc region is a region excluding F(ab')2, containing two light chains and two heavy chains containing a portion of the constant region between the CH1 and CH2 domains such that an inter-chain disulfide bond is formed between the two heavy chains. The Fc region constituting an antigen-binding molecule disclosed in the present specification can be preferably obtained by partially digesting an IgG1, IgG2, IgG3, IgG4 monoclonal antibody, and such, with a proteolytic enzyme such as pepsin, and then re-eluting the fraction adsorbed on a protein A column. The proteolytic enzyme is not particularly limited as long as it can digest the full-length antibody so as to produce F(ab')2 in a restrictive manner by appropriately setting the reaction conditions of the enzyme such as pH, and examples are pepsin, ficin, and the like.

"Fcγ receptor"

Fcγ receptor refers to a receptor capable of binding to the Fc region of an IgG1, IgG2, IgG3, IgG4 monoclonal antibody, and means substantially any member of the family of proteins encoded by the Fcγ receptor gene. In humans, this family includes FcyRI (CD64) containing the isoforms FcyRIa, FcyRIb and FcyRIc; FcyRII (CD32) containing the isoforms FcyRIIa (including allotypes H131 and R131), FcyRIIb (including FcγRIIb-1 and FcyRIIb-2) and FcyRIIc; and FcyRIII (CD16) containing the isoforms FcyRIIIa (including allotypes V158 and F158) and FcγRIIIb (including allotypes FcγRIIIb-NA1 and FcyRIIIb-NA2), and any undiscovered human FcyRs or FcyR isoforms or allotypes, but is not limited thereto. FcyRs may be of any biological origin, including, but not limited to, humans, mice, rats, rabbits and monkeys. Mouse FcyRs include FcyRI (CD64), FcyRII (CD32), FcyRIII (CD16) and FcyRIII-2 (CD 16-2), as well as any undiscovered mouse FcyRs or FcyR isoforms or allotypes, but are not limited thereto.
Preferable examples of such Fcγ receptors include human FcyI (CD64), FcγIIA (CD32), FcγIIB (CD32), FcγIIIA (CD16) and/or FcγIIIB (CD16). The amino acid sequence of FcyI is set forth in RefSeq registration number NP_000557.1, the amino acid sequence of FcγIIA in RefSeq registration number AAH20823.1, the amino acid sequence of FcγIIB in RefSeq registration number AAI46679.1, the amino acid sequence of FcγIIIA in RefSeq registration number AAH33678.1, and the amino acid sequence of FcγIIIB in RefSeq Registration No. AAI28563.1. Whether or not an Fcγ receptor has binding activity in the Fc region of IgG1, IgG2, IgG3, and IgG4 monoclonal antibodies can be confirmed by, for example, FACS and ELISA formats described above, as well as by ALPHAScreen (Amplified Luminescent Proximity Homogenous Assay) and BIACORE method utilizing the surface plasmon resonance (SPR) phenomenon (Proc. Natl. Acad. Sci. USA (2006) 103 (11), 4005-4010).

Further, the term "Fc ligand" or "effector ligand" refers to a molecule derived from any organism, preferably a polypeptide, which binds to the Fc region of an antibody to form an Fc/Fc ligand complex. Binding of the Fc ligand to Fc preferably induces one or more effector functions. Fc ligands include Fc receptors, FcγR, FcaR, FcεR, FcRn, C1q, C3, mannan-binding lectin, mannose receptor, staphylococcus protein A, staphylococcus protein G and viral FcγR, but are not limited thereto. Fc ligands also include Fc receptor homologues (FcRH) (Davis et al., (2002) Immunological Reviews 190, 123-136), which are a family of Fc receptors homologous to FcγR. Fc ligands may also include undiscovered molecules that bind to Fc.

Binding activity to Fcγ receptor

Whether an Fc region has reduced binding activity to an Fcγ receptor of any one of FcγI, FcγIIA, FcγIIB, FcγIIIA and/or FcγIIIB can be confirmed by, for example, FACS and ELISA formats described above, as well as by ALPHAScreen (Amplified Luminescent Proximity Homogenous Assay) and BIACORE method utilizing the surface plasmon resonance (SPR) phenomenon (Proc. Natl. Acad. Sci. USA (2006) 103 (11), 4005-4010).

ALPHAScreen is carried out based on the following principle by ALPHA technology using two beads - a donor and an acceptor. A luminescent signal is detected only when a molecule bound to the donor bead biologically interacts with a molecule bound to the acceptor bead, and the two beads are in close proximity. The photosensitizer in the donor beads excited by the laser converts the surrounding oxygen into excited singlet oxygen. Singlet oxygen diffuses around the donor beads, and when it reaches the adjacent acceptor beads, it causes a chemiluminescent reaction inside the beads, eventually emitting light. When the molecule bound to the donor bead and the molecule bound to the acceptor bead do not interact, the chemiluminescent reaction does not occur because the singlet oxygen produced by the donor bead does not reach the acceptor bead.

When one of the substances (ligand) for observing the interaction is fixed on the thin gold film of the sensor chip and light is applied from the back of the sensor chip so that it is totally reflected at the interface between the thin gold film and the glass, a part with reduced reflection intensity (SPR signal) is formed in a part of the reflected light. When the other substance (analyte) for observing the interaction is injected onto the surface of the sensor chip and the ligand and the analyte are bound, the mass of the immobilized ligand molecule increases and the refraction index of the solvent on the surface of the sensor chip changes. This change in the refraction index causes a positional shift of SPR signal (conversely, the dissociation shifts the signal back to the original position). The Biacore system takes the above-mentioned shift amount, that is, the change in mass on the surface of the sensor chip on the vertical axis, and displays the change in mass over time as measurement data (sensorgram). The kinetics: the binding rate constant (ka) and the dissociation rate constant (kd), are obtained from the curve of the sensorgram and the affinity (KD) is obtained from the ratio of these constants. Inhibition assays are also preferably used in the BIACORE method. Examples of inhibition assays are described in Proc. Natl. Acad. Sci. USA (2006) 103 (11), 4005-4010.

Herein, "Fcγ receptor-binding activity is reduced" refers to, for example, that based on the above-described analysis method, the competitive activity of a test polypeptide complex is 50% or less, preferably 45% or less, 40% or less, 35% or less, 30% or less, 20% or less, or 15% or less, and particularly preferably 10% or less, 9% or less, 8% or less, 7% or less, 6% or less, 5% or less, 4% or less, 3% or less, 2% or less, or 1% or less than the competitive activity of a control polypeptide complex.

Antigen-binding molecules comprising the Fc region of a monoclonal IgG1, IgG2, IgG3, or IgG4 antibody can be appropriately used as control antigen-binding molecules. The Fc region structures are shown in SEQ ID NOs: 66 (A is added to the N terminus of RefSeq accession number AAC82527.1), 67 (A is added to the N terminus of RefSeq accession number AAB59393.1), 68 (A is added to the N terminus of RefSeq accession number CAA27268.1), and 69 (A is added to the N terminus of RefSeq accession number AAB59394.1). Furthermore, when an antigen-binding molecule comprising an Fc region mutant of an antibody of a particular isotype is used as a test substance, the effect of the mutation of the mutant on the Fcγ receptor-binding activity is assessed using as a control an antigen-binding molecule comprising an Fc region of the same particular isotype. Antigen-binding molecules comprising an Fc region mutant whose Fcγ receptor-binding activity has been confirmed to be reduced are appropriately prepared as described above.

Mutants having a deletion of amino acids 231A-238S according to EU numbering (WO 2009/011941), and mutants such as C226S, C229S, P238S, (C220S) (J. Rheumatol (2007) 34, 11); C226S and C229S (Hum. Antibod. Hybridomas (1990) 1(1), 47-54); and C226S, C229S, E233P, L234V, and L235A (Blood (2007) 109, 1185-1192) are known as examples of such mutants.

Namely, preferable examples are antigen-binding molecules having an Fc region in which, among amino acids constituting the Fc region of an antibody of a particular isotype, an amino acid at any of the following positions according to EU numbering: 220, 226, 229, 231, 232, 233, 234, 235, 236, 237, 238, 239, 240, 264, 265, 266, 267, 269, 270, 295, 296, 297, 298, 299, 300, 325, 327, 328, 329, 330, 331, or 332 is substituted. The isotype of the antibody from which the Fc region originates is not particularly limited, and the Fc region derived from an IgG1, IgG2, IgG3, or IgG4 monoclonal antibody, preferably the Fc region derived from IgG1 antibodies, may be appropriately used.

The mutant Fc regions of the antigen-binding molecule described herein may have a mutation that regulates the association of a bispecific antibody composed of, for example, two types of heavy chain variable regions (first heavy chain and second heavy chain) and two types of light chain variable regions (first light chain and second light chain).

For efficient production of bispecific antibodies, mutations may be included that preferentially secrete different combinations of IgG for the H chain by substituting amino acids in the CH3 region of the IgG H chain. Specifically, this is a method conducted by substituting an amino acid side chain present in the CH3 region of one of the H chains with a larger side chain (knob; bulge), and substituting an amino acid side chain present in the CH3 region of the other H chain with a smaller side chain (hole; void), thereby promoting heterologous H chain formation and inhibiting homologous H chain formation by allowing knobs to be placed in the holes.

Furthermore, in the mutant Fc domains of the antigen-binding molecules described herein, mutations in amino acid residues may be introduced at the interface so that the two or more amino acid residues forming the interface have the same charge such that the association between the original polypeptides is inhibited.

For example, in an antigen-binding molecule containing two or more heavy chain CH3 regions, one to three sets of amino acid residues in the first heavy chain CH3 region selected from the set of amino acid residues shown in the following (1) to (3) according to EU numbering may have the same kind of charge.

(i) 356 and 439
(ii) 357 and 370
(iii) 399 and 409

With respect to bispecific antibodies, the above "first heavy chain" is the H chain of one of the two H chains forming the antibody, and the second H chain refers to the other H chain different from the first H chain. That is, one of the two H chains can be arbitrarily designated as the first H chain and the other can be designated as the second H chain.

An "isolated" antibody is one that has been isolated from its original environmental components. In some embodiments, the antibody is assayed, for example, by electrophoresis *(e.g.,* SDS-PAGE, isoelectric focusing (IEF), capillary electrophoresis) or chromatography *(e.g.,* ion exchange or reverse phase HPLC), and purified to a purity greater than 95% or 99%. See for example, Flatman et al., J. Chromatogr. See B 848: 79-87 (2007) for a review of methods for assessing antibody purity.

"Isolated" nucleic acid refers to a nucleic acid molecule isolated from its original environmental components. An isolated nucleic acid contains a nucleic acid molecule contained within a cell that normally contains the nucleic acid molecule, but the nucleic acid molecule exists extrachromosomally, or exists at a position on the chromosome different from the original position on the chromosome.

"Isolated nucleic acid encoding an anti-FGFR3 S249C mutant antibody" refers to one or more nucleic acid molecules encoding the heavy and light chains (or fragments thereof) of the antibody, and includes nucleic acid molecules on one vector or different vectors, and nucleic acid molecules existing at one or more positions in a host cell.

The term "monoclonal antibody" in the present specification refers to an antibody obtained from a substantially uniform population of antibodies. That is, the individual antibodies that make up the population are identical and/or bind to the same epitope, except for possible mutant antibodies (e.g., mutant antibodies that contain naturally-occurring mutations, or mutant antibodies that occur during the production of monoclonal antibody preparations. Such mutants are usually present in small quantities). In contrast to polyclonal antibody preparations that typically contain different antibodies against different determinants (epitopes), each monoclonal antibody in the monoclonal antibody preparation is directed to a single determinant on the antigen. Therefore, the modifier "monoclonal" indicates the antibody characteristic that it is obtained from a substantially homogeneous population of antibodies, and should not be construed as requiring the production of the antibody by any particular method. For example, the monoclonal antibodies used according to the present invention may be prepared by various methods such as, the hybridoma methods, the recombinant DNA method, the phage display method, methods using transgenic animals containing all or a part of the human immunoglobulin locus, but are not limited thereto. Such methods and other exemplary methods for producing monoclonal antibodies are described herein.

"Naked antibody" refers to an antibody that is not conjugated to a heterologous part (e.g., cytotoxic part) or radiolabel. Naked antibodies can be present in pharmaceutical formulations.

"Native antibody" refers to an immunoglobulin molecule with various naturally-occurring structures. For example, a native IgG antibody is a heterotetrameric glycoprotein of approximately 150,000 daltons composed of disulfide-bonded two identical light chains and two identical heavy chains. From the N-terminus to the C-terminus, each heavy chain has a variable region (VH), also called a variable heavy chain domain or heavy chain variable domain, followed by three constant domains (CHI, CH2, and CH3). Similarly, from the N-terminus to the C-terminus, each light chain has a variable region (VL), also called a variable light chain domain or a light chain variable domain, followed by a constant light chain (CL) domain. The light chain of an antibody may be assigned to one of two types, called kappa (κ) and lambda (λ), based on the amino acid sequence of its constant domain.

"Host Cell", "Host Cell Line", and "Host Cell Culture"

Herein, "host cell", "host cell line", and "host cell culture" are interchangeably used and means foreign nucleic acid-introduced cells (including progeny of such cells). Host cells include "transformants" and "transformed cells", which include primary transformed cells and progeny derived from those cells regardless of the number of passages. Progeny do not have to be exactly identical to the parent cell in content of nucleic acid, and may have mutations. Mutant progeny having the same function or biological activity which is used to screen or select the original transformed cells are also included herein.

"Vector"

The term "vector" in the present specification refers to a nucleic acid molecule capable of amplifying another nucleic acid to which it is linked. The term includes vectors as self-replicating nucleic acid structures and vectors incorporated into the genome of host cells into which they have been introduced. Certain vectors can result in the expression of nucleic acids to which they are operably linked. Such vectors are also referred to herein as "expression vectors". An expression vector can be introduced into a host cell by a method using a virus or an electroporation method, but the introduction of the expression vector is not limited to the introduction *in vitro,* and can also be directly introduced into the living body.

"Chimeric Antigen Receptor (CAR)"

The term "chimeric antigen receptor" or "CAR" in the present specification means an antigen receptor designed to bind to a cell surface antigen in a manner independent of a human leukocyte antigen. Gene alteration of T cells to target antigens expressed on tumor cells through expression of a chimeric antigen receptor (CAR) is one approach for treating patients with malignant tumors. For example, see Brentjens et al., 2010, Molecular Therapy, 18: 4, 666-668; Morgan et al., 2010, Molecular Therapy, published online February 23, 2010, pages 1-9; and Till et al., 2008, Blood, 112: 2261-2271. Most commonly, CAR comprises an antigen binding domain, a transmembrane domain, a co-stimulatory signaling region, and a CD3ζ signaling domain. The extracellular binding domain of CAR is derived from a murine monoclonal antibody or a humanized monoclonal antibody. See, for example, Patent No. 5840837 and Patent No. 6293194.

"Co-stimulatory ligand"

The "co-stimulatory ligands" of the present specification include molecules on antigen-presenting cells *(e.g.,* aAPCs, dendritic cells, B cells, etc.) that can specifically bind to a cognate co-stimulatory molecule on a T cell, and can thereby provide signals that mediate T cell responses including, without limitation, growth, activation, differentiation, and such signals, in addition to, for example, the primary signal given by the binding of a peptide-loaded MHC molecule to the TCR/CD3 complex. Co-stimulatory ligands include, without limitation, CD7, B7-1 (CD80), B7-2 (CD86), PD-L1, PD-L2, 4-1BBL, OX40L, inducible co-stimulatory ligand (ICOS-L), intracellular adhesion molecule (ICAM), CD30L, CD40, CD70, CD83, HLA-G, MICA, MICB, HVEM, phosphotoxin β receptor, 3/TR6, ILT3, ILT4, HVEM, agonists or antibodies that bind to the Toll ligand receptor, and ligands that specifically binds to B7-H3. Co-stimulatory ligands also include, without limitation, antibodies that specifically bind to co-stimulatory molecules present on T cells such as CD27, CD28, 4-1BB, OX40, CD30, CD40, PD-1, ICOS, lymphocyte function-related antigen-1 (LFA-1), CD2, CD7, LIGHT, NKG2C, and B7-H3, and also ligands that specifically bind to CD83.

"Co-stimulatory molecule"

A "co-stimulatory molecule" is a cognate-binding partner on a T cell that specifically binds to a co-stimulatory ligand, thereby mediating a co-stimulatory response by the T cell, such as, but not limited to, proliferation. Co-stimulatory molecules include, but are not limited to, MHC class I molecules, BTLA and Toll ligand receptors.

"Co-stimulatory signal"

As used herein, a "co-stimulatory signal" is a molecule that, in combination with a primary signal such as TCR/CD3 linkage, induces T cell proliferation and/or key molecule upregulation or downregulation.

Definitions relating to pharmaceuticals

"Pharmaceutical formulation"

A drug/agent (e.g., a pharmaceutical formulation) refers to a preparation which is in a form such that the biological activity of the active ingredient contained therein can exert its effect, and which does not include additional elements that are unacceptably toxic to a subject to whom the formulation is administered.
The "individual" or "subject" is a mammal. Mammals are, without limitation, domestic animals *(e.g.,* cows, sheep, cats, dogs, horses), primates *(e.g.,* humans and non-human primates such as monkeys), rabbits, as well as rodents (e.g., mice and rats). In certain embodiments, the individual or subject is a human.

"Pharmaceutically-acceptable carrier"

The term "pharmaceutically-acceptable carrier" as used herein refers to an ingredient other than the active ingredient in a pharmaceutical formulation that is nontoxic to a subject. Pharmaceutically-acceptable carriers include, but are not limited to, buffers, excipients, stabilizers, or preservatives.

"Treatment" (and its grammatical derivatives thereof, such as "treating", "to treat", etc.)

The term "treatment" (and its grammatical derivatives thereof, such as "treating", "to treat", etc.) as used herein is a clinical intervention intended to alter the natural course of the individual being treated, and can be performed for prevention and also performed during the course of a clinical condition. The desired effects of treatment include, but are not limited to, prevention of the onset or recurrence of the disease, alleviation of symptoms, reduction of any direct or indirect pathological effects of the disease, prevention of metastasis, reduced rate of progression, recovery or alleviation of disease state, and ameliorated or improved prognosis. In some embodiments, the antibodies of the present disclosure are used to delay the onset of a disease or slow the progression of a disease.

"Therapeutically-effective amount"

The expression "therapeutically-effective amount" in the present specification refers to the amount of a therapeutic agent for treating or preventing a disease or disorder in a mammal. In the case of cancer, a therapeutically-effective amount of a drug either reduces the number of cancer cells; reduces the size of the primary tumor; inhibits the infiltration of cancer cells into surrounding organs *(i.e.,* causes some delay, and preferably suspension); inhibits tumor metastasis *(i.e.,* causes some delay, and preferably suspension); causes some inhibition of tumor growth; or causes some relief of one or more disease-related symptoms, or both. To some extent, the drug can either prevent proliferation or kill existing cancer cells, or both, and is either mitotic or cytotoxic, or both. In cancer treatment, *in vivo* efficacy is measured by assessing at least one indicator selected from the group consisting of, for example, survival time, time to progression (TTP), response rate (RR), response period, and quality of life.

"Cancer" and "cancerous"

As used herein, "cancer" and "cancerous" refer to or describe a mammalian physiological condition that is generally characterized by uncontrolled cell proliferation. Benign and malignant cancers are included in this definition. An "early-stage cancer" or "early-stage tumor" means a cancer that is not invasive or metastatic or is classified as a stage 0, I, or II cancer. Examples of cancers include carcinoma, lymphoma, blastoma (including medulloblastoma and retinoblastoma), sarcoma (including liposarcoma and synovial sarcoma), neuroendocrine tumors (including carcinoid tumors, gastrinoma and pancreatic islet cell carcinoma), mesothelioma, Schwannoma (including acoustic neuroma), meningitis, adenocarcinoma, melanoma, and leukemia or lymphocytic malignancies, but are not limited thereto. More specific examples of such cancers include squamous cell carcinoma (e.g., lung squamous cell carcinoma or HPV (human papillomavirus)-positive head and neck squamous carcinoma), small cell lung cancer (SCLC), non-small cell lung cancer (NSCLC), lung cancer including lung adenocarcinoma and lung squamous cell carcinoma, peritoneal cancer, hepatocellular carcinoma, gastric/stomach cancer, pancreatic cancer including digestive system cancer, glioblastoma, cervical cancer, ovarian cancer, liver cancer, bladder cancer, liver tumor, breast cancer (including metastatic breast cancer), colon cancer, rectal cancer, colorectal cancer, endometrial or uterine carcinoma, salivary gland carcinoma, kidney or renal cancer, prostate cancer, vulvar cancer, thyroid cancer, liver carcinoma, anal carcinoma, penile carcinoma, testicular cancer, esophageal cancer, biliary tract tumor, and head and neck cancer, and multiple myeloma.
The term "package insert" is used to refer to instructions for use of therapeutic products, which is commonly included in commercial packages of the therapeutic products and includes information on indications, dosage form, dosage, administration method, concomitant therapies, contraindications, and/or warnings.

II. Compositions and methods

The present disclosure encompasses compositions comprising a pharmaceutical composition, which include either or both of an anti-FGFR3 S249C mutant antibody and a polynucleotide comprising a sequence encoding an anti-FGFR3 S249C mutant antibody. A composition as used herein encompasses one or several antibodies that bind to an FGFR3 S249C mutant and/or one or several polynucleotides that comprise a sequence/sequences encoding one or several antibodies that bind to an FGFR3 S249C mutant. These compositions may further include suitable carriers, for example, pharmaceutically-acceptable excipients such as buffers. These are well known in the art.

The present disclosure also encompasses embodiments of isolated antibodies and polynucleotides. Also encompassed are embodiments of substantially pure antibodies and polynucleotides.

The disclosure also encompasses methods of treating diseases using anti-FGFR3 S249C mutant antibodies (as described herein or known in the art).

Compositions

The anti-FGFR3 S249C mutant antibodies of the present disclosure are preferably monoclonal. Also encompassed within the scope of the present disclosure are the Fab, Fab', Fab'-SH and F(ab')2 fragments of anti-FGFR3 S249C mutant antibodies provided herein. These antibody fragments may be prepared by conventional methods, such as enzymatic digestion, or by recombinant techniques. Such antibody fragments may be chimeric or humanized. These fragments are useful for diagnostic and therapeutic purposes as described below.

Monoclonal antibodies refer to antibodies obtained from a population of substantially the same type of antibody, *i.e.,* the individual antibodies that make up the population are identical except for a small amount of naturally-occurring mutants that may be present. Thus, the modifier "monoclonal" implies a characteristic of the antibodies, not a mixture of separate antibodies.

The anti-FGFR3 S249C mutant monoclonal antibodies of the present disclosure can be prepared using the hybridoma method first described by Kohler et al., Nature, 256: 495 (1975), or the recombinant DNA method (U.S. Pat. No. 4,816,567).

Antibody production methods

Hybridoma method

Immunity

In the hybridoma method, a mouse or other suitable host animal such as a hamster is immunized against induce lymphocytes producing or capable of producing an antibody that specifically binds to the protein used for immunization. Generally, an antibody to an FGFR3 S249C mutant is produced within animals by multiple subcutaneous (sc) or intraperitoneal (ip) injections of an FGFR3 S249C mutant and an adjuvant. The FGFR3 S249C mutant can be prepared using methods known in the art. Some of the methods are described further herein. For example, recombinant production of human and mouse FGFR3 S249C mutants is described below. In one embodiment, an animal is immunized with an FGFR3 S249C mutant fused to the Fc site of an immunoglobulin heavy chain. In a preferred embodiment, the animal is immunized with a FGFR3 S249C mutant-IgG1 fusion protein. Normally, animals are immunized against an immunogenic conjugate of an FGFR3 S249C mutant or a derivative, using Monophosphorylated Lipid A (MPL)/trehalose dicrynomycolate (TDM) (Ribi Immunochem. Research, Inc., Hamilton, MT), and the solution is injected subcutaneously at multiple sites. Two weeks later, the animals are boosted. After 7-14 days, blood is collected from the animals and the serum is tested for antibody titer. The animals are further boosted until the antibody titer reaches a plateau.

An alternative method may be immunizing lymphocytes *in vitro.* Then the lymphocytes are fused with myeloma cells using a suitable fusing agent such as polyethylene glycol to form hybridomas (Goding, Monoclonal Antibodies: Principles and Practice, pp. 59-103 (Academic Press, 1986)). Alternatively, the method of DNA immunization of animals to be immunized with a cDNA encoding an FGFR3 mutant in which an expression control region is operably linked is also known. DNA immunization does not require purification of the immunogen.

Hybridomas

Hybridomas thus prepared are seeded and grown in a suitable medium preferably containing one or more substances that inhibit the growth or survival of unfused parental myeloma cells. For example, if the parental myeloma cells are deficient in the enzyme hypoxanthine-guanine phosphoribosyltransferase (HGPRT or HPRT), the hybridoma medium will typically contain hypoxanthine, aminopterin and thymidine (HAT medium), which are substances that prevent the growth of HGPRT-deficient cells.

Preferred myeloma cells

Preferred myeloma cells are cells that fuse efficiently, support stable, high-level production of antibodies by selected antibody-producing cells, and are sensitive to media such as the HAT medium. Of these, preferred myeloma cell lines are murine myeloma lineage cells, for example, MOPC-21 and MPC-11 mouse tumors available from the Salk Institute Cell Distribution Center, San Diego, California, USA, and those derived from SP-2 or X63-Ag8-653 cells available from the American Type Culture Collection, Rockville, Maryland, USA. Human myeloma and mouse-human hetero myeloma cell lines have also been disclosed for the production of human monoclonal antibodies (Kozbor, J. Immunol., 133: 3001 (1984); Brodeur et al., Monoclonal Antibody Production Techniques and Applications, Pages 51-63 (Marcel Dekker, Inc., New York, 1987)).

Alternatively, cDNA encoding the antibody variable region can be obtained directly from antibody-producing cells of an immunized animal. For example, the method of conducting limiting dilution of B cells from immunized rabbits, and culturing them together with supporting cells is known. The specificity of the antibody produced in the culture medium can be compared in advance, and cDNA encoding the antibody variable region can be recovered from the cells producing the target antibody by a method such as PCR. By incorporating the recovered cDNA into an appropriate expression system, it becomes possible to obtain a monoclonal antibody without going via hybridoma.

Assaying the produced monoclonal antibody

The medium in which the hybridomas are growing is assayed for the production of a monoclonal antibody against the FGFR3 S249C mutant. Preferably, the binding specificity of the monoclonal antibody produced by the hybridomas is determined by immunoprecipitation or an *in vitro* binding assay such as radioimmunoassay (RIA) or enzyme-linked immunosorbent assay (ELISA).

Methods for measuring the binding affinity of a monoclonal antibody

The binding affinity of a monoclonal antibody is determined by, for example, the Scatchard analysis method of Munson et al., Anal. Biochem., 107: 220 (1980).

Hybridoma proliferation

Once a hybridoma producing an antibody of the desired specificity, affinity, and/or activity has been identified, the clone can be subcloned by limiting dilution and proliferated by standard methods (Goding, Monoclonal Antibodies: Principles and Practice, pp. 59-103 (Academic Press, 1986)). Media suitable for this purpose include, for example, D-MEM or RPMI-1640 medium. In addition, the hybridomas can be grown *in vivo* as ascites tumors in animals.

Monoclonal antibodies secreted by subclones can be suitably separated from media, ascites, or serum by conventional immunoglobulin purification methods such as protein A-SEPHAROSE, hydroxyapatite chromatography, gel electrophoresis, dialysis, or affinity chromatography.

Screening for anti-FGFR3 S249C mutant antibody clones

An anti-FGFR3 S249C mutant antibody of the present disclosure can be identified using a combinatorial library to screen for synthetic antibody clones with the desired activity. In principle, synthetic antibody clones are selected by screening phage libraries having phage that display various fragments of antibody variable regions (Fv) fused to phage coat protein. Such phage libraries are sorted by affinity chromatography for the desired antigen. Clones expressing Fv fragments capable of binding to the desired antigen are absorbed onto the antigen, thereby separating them from the non-binding clones of the library. The binding clones can then be eluted from the antigen and further enriched by an additional cycle of antigen absorption/elution. Any anti-FGFR3 S249C mutant antibody of the present disclosure can be obtained by designing a suitable antigen screening procedure for selecting the phage clone of interest, followed by constructing a full-length anti-FGFR3 S249C mutant antibody clone using the Fv sequences from the phage clone of interest and an appropriate constant region (Fc) sequences described in Kabat et al., Sequences of Proteins of Immunological Interest, Fifth Edition, NIH Publication 91-3242, Bethesda MD (1991), vols.1-3.

Fv clones

The antigen-binding domain of an antibody is formed by two variable (V) regions of about 110 amino acids - the light (VL) and heavy (VH) chains. Both of these have three hypervariable loops or complementary strand determination regions (CDR). As described in Winter et al., Ann. Rev. Immunol., 12: 433-455 (1994), variable regions can be functionally displayed on phages either as single-chain Fv (scFv) fragments in which VH and VL are covalently linked via a short and flexible peptide, or as Fab fragments, in which they are each fused to a constant domain and interact non-covalently. As used herein, the scFv-coding phage clones and the Fab-coding phage clones are collectively referred to as "Fv phage clones" or "Fv clones".

Random recombination of VH and VL genes in the phage library

Repertoires of VH and VL genes can be isolated and cloned by the polymerase chain reaction (PCR), randomly recombined in a phage library, and screened for antigen-binding clones as described in Winter et al., Ann. Rev. Immunol., 12: 433-455 (1994) and such. Libraries from immunized sources do not need hybridoma construction and provide high affinity antibodies against the immunogen. Alternatively, a naive repertoire can be cloned to provide a single source of human antibodies against a wide range of non-self and also self-antigens without any immunization as described in Griffiths et al., EMBO J, 12: 725-734 (1993), and such. Finally, a naive library can be synthetically prepared by cloning unrearranged V gene segments from stem cells, utilizing PCR primers with random sequences to encode the highly variable CDR3 regions, and completing the rearrangement *in vitro,* as described in Hoogenboom and Winter, J. et al. Mol. Biol. 227: 381-388 (1992).

Filamentous phages

Filamentous phages are used to display antibody fragments by fusion with the minor coat protein pIII. These antibody fragments can be displayed as single-chain Fv fragments where VH and VL domains are connected on the same polypeptide chain by a flexible polypeptide spacer as described, for example, in Marks et al., J. Mol. Biol. 222: 581-597 (1991), or as Fab fragments where one strand is fused with pIII and the other is secreted into the periplasm of bacterial host cells, where assembly of a Fab-coat protein structure is displayed on the phage surface by substituting some wild-type coat proteins, as described in Hoogenboom et al., Nucl. Acids. Res., 19: 4133-4137 (1991).

Nucleic acid encoding an antibody gene fragment

Generally, a nucleic acid encoding an antibody gene fragment is obtained from immune cells collected from humans or animals. If a library biased in favor of an anti-FGFR3 S249C mutant clones is desired, the individual is immunized with an FGFR3 S249C mutant to generate an antibody response, and spleen cells and/or circulating B cells, which are other peripheral blood lymphocytes (PBL), are collected for library construction. Anti-FGFR3 S249C mutant antibody

Further enrichment of anti-FGFR3 S249C mutant-reactive cell populations

Further enrichment of anti-FGFR3 S249C mutant-reactive cell populations can be accomplished by using appropriate screening techniques to isolate B cells expressing an FGFR3 S249C mutant-specific membrane-binding antibody, for example, by cell separation using FGFR3 S249C mutant affinity chromatography or by fluorescent dye labeling, adsorption of cells to the FGFR3 S249C mutant, and subsequently, a fluorescence-activated cell sorter

(FACS).

Construction of an antibody library using an arbitrary animal (human or non-human) species

Alternatively, utilization of spleen cells and/or B cells or other PBLs from non-immunized donors provides a better indication of the possible antibody repertoire, and enables construction of an antibody library using any animal (human or non-human) species in which an FGFR3 S249C mutant is not an immunogen. For libraries that incorporate *in vitro* antibody gene constructs, stem cells are collected from individuals to provide nucleic acids encoding non-rearranged antibody gene segments. The immune cells of interest can be obtained from various animal species such as humans, mice, rats, lagomorphs, wolves, canines, felines, pigs, cows, horses, birds and the like.

Amplification of nucleic acids encoding antibody variable gene segments (including VH and VL segments)

Nucleic acids encoding antibody variable gene segments (including VH and VL segments) are recovered from cells of interest and amplified. In the case of rearranged VH and VL gene libraries, as described in Orlandi et al, Proc. Natl. Acad. Sci. (USA), 86: 3833-3837(1989), the desired DNA can be obtained by isolating genomic DNA or mRNA from lymphocytes and performing a polymerase chain reaction (PCR) with primers that match the 5' and 3' ends of the rearranged VH and VL genes, thus preparing a diverse V gene repertoire for expression. These V genes can be amplified from cDNA and genomic DNA by a back primer at the 5' end of the exon encoding the mature V domain and a forward primer based in the J segment, as described in Orlandi et al, (1989) and Ward et al, Nature, 341:544-546(1979). However, for amplification from cDNA, the back primer can be based on the leader exon as described in Jones et al., Biotechnol., 9: 88-89 (1991), and the forward primer can be based in the constant region as described in Sastry et al., Proc. Natl. Acad. Sci. (USA) 86: 5728-5732 (1989). It is possible to incorporate degenerates into the primer to maximize complementarity as described in Orlandi et al. (1989) or Sastry et al. (1989). Preferably, to amplify all available VH and VL sequences present in the nucleic acid sample of immune cells, the diversity of the library is maximized by using PCR primers targeting each V gene family, as described in, *e.g.,* the method of Marks et al., J. Mol. Biol., 222: 581-597 (1991), or in the method of Orum et al., Nucleic Acids Res., 21: 4491-4498 (1993). For cloning of amplified DNA into expression vectors, rare restriction sites can be introduced as a tag into one end within the PCR primer by further PCR amplification with a tagged primer, as described in Orlandi et al. (1989) or as described in Clackson et al., Nature, 352: 624-628 (1991).

*In vivo* induction of a repertoire of synthetically-rearranged V genes

A synthetically-rearranged V gene repertoire can be induced *in vivo* from the V gene segments. Most human VH gene segments have been cloned, sequenced (reported by Tomlinson et al., J. Mol. Biol. 227: 776-798 (1992)), and mapped (Matsuda et al., Nature Genet., 3: 88-94 (1993)); these cloned segments (including all major conformations of the H1 and H2 loops) are used to generate a diverse VH gene repertoire by PCR primers that code H3 loops of varying sequences and lengths, as described in Hoogenboom and Winter, J. Mol. Biol. 227: 381-388 (1992). A VH repertoire can also be prepared with all the sequence diversity focused on a single long H3 loop, as described in Barbas et al., Proc. Natl. Acad. Sci. USA, 89: 4457- 4461 (1992). Human $V_\kappa$ and $V\lambda$ segments have been cloned and sequenced (Williams and Winter, Eur. J. Immunol., 23: 1456-1461 (1993)) and can be used to generate a synthetic light chain repertoire. A synthetic V gene repertoire, which is based on the range of VH and VL folds and L3 and H3 lengths, encodes antibodies with considerable structural diversity. Following the amplification of the V gene encoding DNA, germline V gene segments can be rearranged *in vitro* according to the method of Hoogenboom and Winter, J. Mol. Biol. 227: 381-388 (1992).

Construction of antibody fragment repertoires

Antibody fragment repertoires can be constructed by combining the VH and VL gene repertoires together in several ways. Each repertoire is made with a different vector, and the vectors can be made *in vitro* as described in, for example, Hogrefe et al., Gene, 128: 119-126 (1993), or *in vivo* by combinatorial infection, for example, by the loxP system described in Waterhouse, Nucl. Acids Res., 21: 2265-2266 (1993) and such. This *in vivo* recombination technique utilizes two-chain Fab fragments to overcome library size limitations imposed by E. *coli* transformation efficiency. Naive VH and VL repertoires are individually cloned, one into a phagemid and the other into a phage vector. The two libraries are then combined by phage infection of phagemid-containing bacteria such that each cell has a different combination and the size of the library is limited only by the number of cells present (about 1012 clones). Both vectors have an *in vivo* recombination signal such that the VH and VL genes are recombined into a single replicon and packaged together into a phage virion. These huge libraries provide many diverse antibodies with good affinity (Kd-1 of about 10-8M).

Construction of antibody fragment repertoires-2

Alternatively, the repertoires can be sequentially cloned into the same vector as described, for example, in Barbas et al., Proc. Natl. Acad. Sci. USA 88: 7978-7982 (1991), or assembled by PCR and then cloned as described, for example, in Clackson et al., Nature, 352: 624-628 (1991). The PCR assembly can also be utilized to ligate DNA encoding a flexible peptide spacer with the VH and VL DNAs to form a single chain Fv (scFv) repertoire. In yet another technique, an "intracellular PCR assembly" is used to combine the VH and VL genes in lymphocytes by PCR and then clone the repertoire of the ligated genes, as described in Embleton et al., Nucl. Acids Res., 20: 3831-3837 (1992), and such.

Naive libraries

Antibodies produced by a naive library (either natural or synthetic) may have moderate affinity ($Kd^{-1}$ of about $10^6$ to $10^7 M^{-1}$), but by constructing and separating from a second library, affinity maturation also can be mimicked *in vitro* as described above in Winter et al. (1994). For example, in the method of Hawkins et al., J. Mol. Biol. 226: 889-896 (1992), or in the method of Gram et al., Proc. Natl. Acad. Sci USA, 89: 3576-3580 (1992), mutations can be randomly introduced *in vitro* by utilizing an error-prone polymerase (reported in Leung et al., Technique, 1: 11-15 (1989)). Furthermore, by randomly mutating one or more CDRs, for example, in selected individual Fv clones, affinity maturation can be achieved by using PCR with a primer having a random sequence extending to the CDR of interest, and screening clones with a higher affinity. WO 9607754 (published March 14, 1996) describes how to induce mutations in the complementarity determining regions of immunoglobulin light chains to create a library of light chain genes. Another effective technique includes recombining VH or VL domains selected by a phage display with a repertoire of naturally-occurring V domain variants obtained from unimmunized donors, and screening for higher affinity by multiple chain shuffling, as described in Marks et al., Biotechnol. 10: 779-783 (1992). This technique allows the production of antibodies and antibody fragments with an affinity in the range of 10-9M.

Nucleic acid and amino acid sequences of FGFR3 S249C mutants

Nucleic acid and amino acid sequences of FGFR3 S249C mutants are known in the art. A nucleic acid sequence encoding an FGFR3 S249C mutant can be designed using the amino acid sequence of the desired region of the FGFR3 S249C mutant. As is well known, FGFR3 has two major splice isoforms, namely, FGFR3 IIIb and FGFR3 IIIc. The FGFR3 sequence is well known in the art and may include the sequence of UniProKB/Swiss-Prot Accession No. P22607 (FGFR3 IIIc) or P22607_2 (FGFR3 IIIb). FGFR3 mutations have been identified and are well known in the art, and include the following mutations (with respect to the sequence shown in UniProKB/Swiss-Prot Accession No. P22607 (FGFR3 IIIc) or P22607_2 (FGFR3 IIIb)):

| FGFR3-IIIb | FGFR3 IIIc |
|---|---|
| R248C | |
| S249C | |
| G372C | G370C |
| Y375C | Y373C |
| G382R | G380R |
| K652E | K650E |

Nucleic acids encoding FGFR3 S249C mutants

Nucleic acids encoding FGFR3 S249C mutants can be prepared by various methods known in the art. These methods include any of the methods described in Engels et al., Agnew. Chem. Int. Ed. Engl., 28: 716-734 (1989), including, but not limited to, chemical synthesis methods using, for example, triester, phosphite, phosphoramidite and H-phosphonate methods. In one embodiment, the preferred codons for the expression host cell are used to design the FGFR3 S249C mutant encoding DNA. Alternatively, DNA encoding an FGFR3 S249C mutant can be isolated from a genomic or cDNA library.

Linkage with an expression control sequence of an expression vector such as a plasmid of a DNA molecule encoding the FGFR3 S249C mutant

Following the construction of a DNA molecule encoding the FGFR3 S249C mutant, the DNA molecule is operably linked to an expression control sequence of an expression vector such as a plasmid, and this control sequence is bound by host cells transformed with that vector. Generally, a plasmid vector has replication and control sequences induced from a species compatible with the host cell. This vector usually has a replication site as well as a sequence encoding a protein capable of providing phenotypic selection in transformed cells. Vectors suitable for expression in prokaryotic and eukaryotic host cells are known in the art, some of which are described herein. Cells derived from prokaryotes such as yeast or multicellular organisms such as mammals can be used.

In some cases, the DNA encoding the FGFR3 S249C mutant is operably linked by the host cell to a secretory leader sequence that causes the secretion of the expression product into the medium. Examples of secretory leader sequences include stII, ecotin, lamB, herpes GD, 1pp, alkaline phosphatase, invertase, and alpha factor. Also suitable for use here is the 36 amino acid leader sequence of protein A (Abrahmsen et al., EMBO J., 4: 3901 (1985)).

Host cells

Host cells are transfected with the above-mentioned expression or cloning vectors of the invention, and preferably transformed and cultured in a general culture solution modified to be suitable for inducing promoters, selecting transformants, or amplifying genes encoding the desired sequence.

Transfection

Transfection means the uptake of an expression vector by a host cell for which it is unknown whether an arbitrary coding sequence is actually expressed. Many methods of transfection are known to ordinary skilled artisans, such as $CaPO_4$ sedimentation and electroporation. Generally, successful transfections are bound when signs of vector action appear within the host cell.

Transformation

Transformation means introducing a DNA into an organism such that it can replicate either as an extrachromosomal component or chromosomal integrant. Depending on the host cell used, transformation is performed using basic techniques suitable for the cell. Transformation methods are known in the art, some of which are further described herein.

Prokaryotic host cells used to produce the FGFR3 S249C mutant

The prokaryotic host cells used to produce the FGFR3 S249C mutant can generally be cultured as described in Sambrook and the like described above.

The mammalian host cells used to produce the FGFR3 S249C mutant can be cultured in a variety of media. The media are well known in the art and some of them are described herein.

The host cells referred to in this disclosure include not only cells in the host animal, but also cells in an *in vitro* culture.

Purification of the FGFR3 S249C mutant

Purification of the FGFR3 S249C mutant is performed using methods bound in the art. Some of them are described herein.

Affinity chromatographic separation of phage display clones

For use in affinity chromatographic separation of phage display clones, the purified FGFR3 S249C mutant can be attached to a suitable matrix such as agarose beads, acrylamide beads, glass beads, cellulose, various acrylic copolymers, hydroxyl methacrylic gels, polyacrylic and poly methacrylic copolymers, nylon, and neutral and ionic carriers. Attachment of the FGFR3 S249C mutant protein to the substrate can be accomplished by methods described in Methods in Enzymology, Vol. 44 (1976). Widely used techniques for attaching protein ligands to polysaccharide matrices such as agarose, dextran or cellulose include carrier activation by halogenated cyan, followed by coupling of peptide ligands to the activating substrate with primary aliphatic or aromatic amines.

Alternatively, the FGFR3 S249C mutant can be used to coat the wells of an absorption plate, expressed on host cells attached to an absorption plate or used in cell sorting, or conjugated to biotin for capture by streptavidin-coated beads, or utilized in any other method of the art for panning phage display libraries.

The phage library samples are contacted with the immobilized FGFR3 S249C mutant under conditions suitable for binding at least a portion of the phage particles to the adsorbent. Usually, conditions including pH, ionic strength, temperature, and such are selected to mimic physiological conditions. The phages bound to the solid phase are washed, and then eluted with an acid as described in, for example, Barbas et al., Proc. Natl. Acad. Sci. USA, 88: 7978-7982 (1991), or with an alkaline as described in, for example, Marks et al., J. Mol. Biol. 222: 581-597 (1991), or by S249C mutant FGFR3 antigenic competition, which is a technique similar to the antigenic competition method of, for example, Clackson et al., Nature, 352: 624-628 (1991). Phages can be concentrated 20 to 1,000 times in the first selection. Furthermore, the concentrated phages can be grown in bacterial cultures for further selection.

Phage selection

The efficiency of selection depends on many factors, including the kinetics of dissociation during washing and whether multiple antibody fragments on a single phage can be simultaneously associated with the antigen. Antibodies with a primary dissociation constant (and weak binding affinity) can be retained by utilizing short washes, polyvalent phage displays and high coating densities of solid phase antigens. High densities not only stabilize phages through multivalent interactions, but also favor rebinding of dissociated phages. Selection of antibodies with slow dissociation kinetics (and good binding affinity) can be enhanced by long washes and the use of monovalent phage displays as described in Bass et al., Proteins, 8: 309-314 (1990) and WO 92/09690, and by a low antigen coating density as described in Marks et al., Biotechnol., 10: 779-783 (1992).

Even if there is a slight difference in affinity, it is possible to select from phage antibodies having different affinities for the FGFR3 S249C mutant. However, random mutations (e.g., as performed in some of the affinity maturation techniques described above) make the selected antibody prone to many mutations, and most of the mutants bind to antigens and only a few of them have a higher affinity. Restricting the FGFR3 S249C mutants allows rare, high-affinity phages to be competitively eliminated. To retain all higher affinity mutations, the phages can be incubated with an excess of biotinylated FGFR3 S249C mutant, but incubated with a biotinylated FGFR3 S249C mutant with a lower molar concentration than the target molar affinity constant for FGFR3 S249C mutant. High affinity binding phages can then be captured by paramagnetic beads coated with streptavidin. Such an "equilibrium capture" enables the selection of antibodies with a sensitivity that allows the isolation of mutant clones with a slightly two-fold higher affinity from excessive phages with a low affinity, based on the binding affinity. It is also possible to manipulate the conditions used to wash the phages bound to the solid phase to identify based on the dissociation constant.

Synthesis of monoclonal antibodies/Fv clones in recombinant host cells

The DNAs encoding the hybridoma-derived monoclonal antibodies or the phage display Fv clones of the present disclosure are immediately isolated and sequenced using conventional methods (for example, by using an oligonucleotide primer that is configured to specifically amplify heavy and light chain or phage DNA templates that code for the region of interest in the hybridoma). Once isolated, DNA is placed in an expression vector, which is then transfected into host cells such as *E. coli* cells, monkey COS cells, Chinese Hamster Ovary (CHO) cells, or myeloma cells, which otherwise would not produce antibody proteins, to acquire synthesis of monoclonal antibodies in recombinant host cells. Review articles on the recombinant expression of antibody-encoding DNA in bacteria include Skerra et al., Curr. Opinion in Immunol., 5: 256-262 (1993) and Pluckthun, Immunol. Revs. 130: 151-188 (1992).

Synthesis of Fv clones

The DNAs that encode the Fv clones of the present disclosure can be combined with known DNA sequences (for example, a suitable DNA sequence can be obtained from the above-mentioned Kabat, etc.) that encode heavy and/or light chain constant regions to form clones that encode full-length or partial heavy and/or light chains. It will be understood that constant regions of any isotype, such as IgG, IgM, IgA, IgD and IgE constant regions, can be used for this. Such constant regions can be obtained from any human or animal species. An Fv clone obtained from a variable domain DNA of one animal species (e.g., human) and then fused to a constant region DNA of another animal species to form a "hybrid" full-length heavy and/or light chain coding sequence, is included in the definitions of "chimeric" and "hybrid" antibodies used herein. In a preferred embodiment, an Fv clone obtained from a human variable DNA is fused to a human constant region DNA to form the coding sequence for all human, full-length or partial heavy and/or light chains.

Synthesis of "chimeric" or "hybrid" antibodies

In addition, a DNA encoding an anti-FGFR3 S249C mutant antibody derived from a hybridoma of the present disclosure can be modified by substituting the homologous mouse sequence derived from the hybridoma clone with the coding sequence of human heavy chain and light chain constant domains (by, for example, the method of Morrison et al., Proc. Nat. Acad. Sci. USA, 81: 6851 (1984)). DNA encoding an antibody or antibody fragment derived from a hybridoma or Fv clone can be further modified by covalently binding all or part of the non-immunoglobulin polypeptide coding sequence to an immunoglobulin coding sequence. As such, "chimeric" or "hybrid" antibodies are prepared to have a binding specificity for antibodies derived from the Fv clones or hybridoma clones of the present disclosure.

In a preferred embodiment, the amino acid sequence of the variable region in the hybridoma-derived anti-FGFR3 S249C mutant antibody of the present disclosure can be, for example, a combination of the following heavy chains (VH) and light chains (VL).

| Clone name | VH | VL |
|---|---|---|
| FGA0002 | SEQ ID NO: 5 | SEQ ID NO: 6 |
| FGA0005 | SEQ ID NO: 7 | SEQ ID NO: 8 and |
| FGA0007 | SEQ ID NO: 9 | SEQ ID NO: 10 |

The amino acid sequences of each VH and VL are amino acid sequences constituting the variable domain of a rabbit antibody. Therefore, a rabbit-human chimeric antibody can be constructed when the human antibody constant domain is conjugated. As constant domains of globulin in human medical practice, for example, the human heavy chain constant region (F760 mnN17; SEQ ID NO: 11) and the human light chain constant region (k0MTC; SEQ ID NO: 12) are known.

Antibody fragments

This disclosure includes antibody fragments. In certain cases, there are advantages to using antibody fragments over whole antibodies. Smaller sized fragments may provide faster clearance and improved access to solid tumors.

Production of antibody fragments

Various techniques have been developed to produce antibody fragments. Traditionally, these fragments have been induced via proteolytic digestion of complete antibodies (e.g., Morimoto et al., Journal of Biochemical and Biophysical Methods 24: 107-117 (1992) and Brennan et al., Science, 229: 81 (1985)). However, these fragments can now be produced directly by recombinant host cells. For example, Fab, Fv and ScFv antibody fragments are all expressed and secreted in *E. coli,* facilitating large-scale production of these fragments. Antibody fragments can be separated from the antibody phage libraries described above. Alternatively, Fab'-SH fragments can be recovered directly from *E. coli,* and F(ab')2 fragments can be formed by chemical coupling (Carter et al., Bio/Technology 10: 163-167 (1992)).

Another approach allows F(ab')2 fragments to be separated directly from recombinant host cell culture. Fab and F(ab')2 fragments with increased *in vivo* half-life containing salvage receptor binding epitope residues are described in U.S. Pat. No. 5,869,046. Other methods for producing antibody fragments should be apparent to those of skill in the art. In another embodiment, the selected antibody is a single chain Fv fragment (scFv). See WO 93/16185; U.S. Pat. No. 5,571,894; and U.S. Pat. No. 5,587,458. Fv and sFv are the only species with complete binding sites lacking constant regions; therefore, they are suitable for reducing nonspecific binding during *in vivo* use. The sFv fusion protein can be constructed at either the amino or carboxy terminus of sFv to obtain a fusion of effector proteins. See the above-mentioned Antibody Engineering, ed. Borrebaeck. Antibody fragments may also be, for example, "linear antibodies" as described in U.S. Pat. No. 5,641,870. Such linear fragments may be monospecific or bispecific.

Production of humanized antibodies by humanization of non-human antibodies

This disclosure includes humanized antibodies. Various methods for humanizing non-human antibodies are conventionally well known. For example, a humanized antibody has one or more amino acid residues of non-human origin introduced into it. These non-human amino acid residues are often referred to as "import" residues, typically obtained from "import" variable domains. Humanization can be essentially carried out using the method of Winter and Collaborators by substituting the relevant hypervariable region sequences of human antibodies (Jones et al., Nature, 321: 522-525 (1986), Riechmann et al., Nature, 332: 323-327 (1988), Verhoeyen et al., Science, 239: 1534-1536 (1988)). Thus, such a "humanized" antibody is a chimeric antibody in which substantially less than the complete human variable domain has been replaced with the corresponding sequence from a non-human species (U.S. Pat. No. 4,816,567). In practice, humanized antibodies are typically human antibodies in which some hypervariable region residues, and, in some cases, some FR residues, are replaced with residues from similar sites in rodent antibodies.

For example, in some embodiments, the amino acid sequences of the variable regions in a hybridoma-derived anti-FGFR3 S249C mutant antibody of the present disclosure can be, for example, a combination of the following heavy chains (VH) and light chains (VL):

| Clone name | VH | VL |
|---|---|---|
| FGA0002 | SEQ ID NO: 5 | SEQ ID NO: 6 |
| FGA0005 | SEQ ID NO: 7 | SEQ ID NO: 8 and |
| FGA0007 | SEQ ID NO: 9 | SEQ ID NO: 10 |

Moreover, the following amino acid sequences were identified as the hypervariable regions (HVR) of each VH and VL.

| | | | |
|---|---|---|---|
| FGA0002 | | SEQ ID NO: 5 | SEQ ID NO: 6 |
| | HVR1 | SEQ ID NO: 17 | SEQ ID NO: 20 |
| | HVR2 | SEQ ID NO: 18 | SEQ ID NO: 21 |
| | HVR3 | SEQ ID NO: 19 | SEQ ID NO: 22 |
| FGA0005 | | SEQ ID NO: 7 | SEQ ID NO: 8 and |
| | HVR1 | SEQ ID NO: 23 | SEQ ID NO: 26 |
| | HVR2 | SEQ ID NO: 24 | SEQ ID NO: 27 |
| | HVR3 | SEQ ID NO: 25 | SEQ ID NO: 28 |
| FGA0007 | | SEQ ID NO: 9 | SEQ ID NO: 10 |
| | HVR1 | SEQ ID NO: 29 | SEQ ID NO: 32 |
| | HVR2 | SEQ ID NO: 30 | SEQ ID NO: 33 |
| | HVR3 | SEQ ID NO: 31 | SEQ ID NO: 34 |

Therefore, a humanized antibody can be constructed by selecting either one or a plurality of each of the above HVRs 1-3 and transferring them into human FR. In a preferred embodiment, the HVRs to be transferred are all of the HVRs 1-3 of VH and VL. The amino acid sequence of human FR for obtaining a humanized antibody can be appropriately selected from known amino acid sequences. Alternatively, a new FR can be obtained from any human antibody library. In one embodiment, when each of the above HVR amino acid sequences are transplanted into human FR as a CDR to construct a humanized antibody, combinations with the following FR amino acid sequences can be exemplified:

| | | | |
|---|---|---|---|
| FGA0002 | | SEQ ID NO: 5 | SEQ ID NO: 6 |
| | CDR1 | SEQ ID NO: 17 | SEQ ID NO: 20 |
| | CDR2 | SEQ ID NO: 18 | SEQ ID NO: 21 |
| | CDR3 | SEQ ID NO: 19 | SEQ ID NO: 22 |
| | FR1 | SEQ ID NO: 35 | SEQ ID NO: 47 |
| | FR2 | SEQ ID NO: 36 | SEQ ID NO: 48 |
| | FR3 | SEQ ID NO: 37 | SEQ ID NO: 39 |
| | FR4 | SEQ ID NO: 38 | SEQ ID NO: 50 |
| FGA0005 | | SEQ ID NO: 7 | SEQ ID NO: 8 and |
| | CDR1 | SEQ ID NO: 23 | SEQ ID NO: 26 |
| | CDR2 | SEQ ID NO: 24 | SEQ ID NO: 27 |
| | CDR3 | SEQ ID NO: 25 | SEQ ID NO: 28 |
| | FR1 | SEQ ID NO: 39 | SEQ ID NO: 51 |
| | FR2 | SEQ ID NO: 40 | SEQ ID NO: 52 |
| | FR3 | SEQ ID NO: 41 | SEQ ID NO: 53 |
| | FR4 | SEQ ID NO: 42 | SEQ ID NO: 54 |
| FGA0007 | | SEQ ID NO: 9 | SEQ ID NO: 10 |
| | CDR1 | SEQ ID NO: 29 | SEQ ID NO: 32 |
| | CDR2 | SEQ ID NO: 30 | SEQ ID NO: 33 |
| | CDR3 | SEQ ID NO: 31 | SEQ ID NO: 34 |
| | FR1 | SEQ ID NO: 43 | SEQ ID NO: 55 |
| | FR2 | SEQ ID NO: 44 | SEQ ID NO: 56 |
| | FR3 | SEQ ID NO: 45 | SEQ ID NO: 57 |
| | FR4 | SEQ ID NO: 46 | SEQ ID NO: 58 |

Humanized antibodies with reduced antigenicity

In order to reduce antigenicity, the choice of both light and heavy human variable domains used in producing humanized antibodies is very important. The "best fit method" screens a variable domain sequence of a rodent antibody against the entire library of known human variable domain sequences. Next, the human sequence closest to that of rodent is accepted as the human framework region of the humanized antibody (Sims et al., J. Immunol., 151: 2296 (1993); Chothia et al., J. Mol. Biol.., 196: 901 (1987)). Another method uses a specific framework region derived from the consensus sequence of all human antibodies in a specific light or heavy chain subgroup. The same framework can be used for several different humanized antibodies (Carter et al., Proc. Natl. Acad. Sci., USA, 89: 4285 (1992); Presta et al., J. Immunol., 151: 2623 (1993)).

Humanized antibodies with high affinity and other favorable biological properties

Furthermore, it is important to humanize the antibody while retaining a high antigen affinity and other favorable biological properties. To achieve this objective, humanized antibodies are prepared by a certain method that uses a three-dimensional model of parental and humanized sequences via steps of analyzing the parental sequence and various conceptual humanized products. Three-dimensional immunoglobulin models are commonly available and are well known to those of skill in the art. Computer programs that illustrate and display the assumed three-dimensional conformational structure of the selected candidate immunoglobulin sequences are available for purchase. These displays allow the analysis of the likely role of residues in the function of candidate immunoglobulin sequences, *i.e.,* analysis of residues that affect the ability of a candidate immunoglobulin to bind to its antigen. In this way, FR residues can be selected from the recipient and import sequences and combined, so that the desired antibody properties such as increased affinity for the target antigen are achieved. In general, hypervariable region residues directly and most substantially affect antigen binding activity.

Production of human antibodies

The human anti-FGFR3 S249C mutant antibodies of the present disclosure can be constructed by binding Fv clone variable domain sequences selected from a human-derived phage display library to known human constant domain sequences as described above. Alternatively, the human monoclonal anti-FGFR3 S249C mutant antibodies of the present disclosure can be prepared by the hybridoma method. Human myeloma and mouse-human heteromyeloma cell lines for the production of human monoclonal antibodies are described, for example, in Kozbor, J., Immunol. 133, 3001 (1984); Brodeur et al., Monoclonal Antibody Production Techniques and Applications, pp. 51-63 (Marcel Dekker, Inc., New York, 1987); and Boerner et al., J. Immunol., 147: 86 (1991).

Production of transgenic animals (e.g., mice)

It is now possible to produce transgenic animals (e.g., mice) capable of producing a complete repertoire of human antibodies without the production of endogenous immunoglobulins, through immunization. For example, homozygous deletion of the antibody heavy chain joining region (JH) gene in chimeric and germline mutant mice has been described to result in complete inhibition of endogenous antibody production. Transfer of the human germline immunoglobulin gene sequence into such germline mutant mice causes the production of human antibodies upon antigen challenge. For example, see Jakobovits et al., Proc. Natl. Acad. Sci., USA 90, 2551-255 (1993); Jakobovits et al., Nature 362, 255-258 (1993).

Gene shuffling

Gene shuffling can also be used to obtain human antibodies from non-human, e.g., rodent antibodies, if the human antibody has similar affinities and properties to the starting non-human, e.g., rodent, antibody. By this method, also referred to as "epitope imprinting," either the heavy chain variable region gene or the light chain variable region gene of the non-human antibody fragment obtained by the above-mentioned phage display technique is replaced with a repertoire of human V domain genes to create a population of non-human/human chain scFv or Fab chimera. By selection with an antigen, a non-human chain/human chain chimeric scFv or Fab is isolated, in which the human chain restores the antigen binding site disrupted by the removal of the corresponding non-human chain in the primary phage display clone, *i.e.,* the epitope governs (imprints) the selection of the human chain partner. Repeating this step to replace the remaining non-human chain yields a human antibody (see PCT patent application WO 93/06213 published April 1, 1993). Unlike humanization of non-human antibodies by traditional CDR grafting, this technique provides fully human antibodies with no FR or CDR residues of non-human origin.

Bispecific antibodies

Bispecific antibodies are monoclonal antibodies, preferably human antibodies or humanized antibodies, which have a binding specificity for at least two different epitopes. In this case, one of the binding specificities is for an FGFR3 S249C mutant and the other is for any other antigen. An exemplary bispecific antibody can bind to two different epitopes of an FGFR3 S249C mutant. Bispecific antibodies can also be used to localize cytotoxic activity in cells expressing an FGFR3 S249C mutant. These antibodies have an FGFR3 S249C mutant-binding arm and an arm that binds to CD3 to exert T cell-dependent cytotoxic activity. A bispecific antibody can be prepared as a full-length antibody or antibody fragment (e.g., F(ab')2 bispecific antibody).

In some embodiments of the present disclosure, the following amino acid sequences can be exemplified as CDRs for constructing the variable region domains of the FGFR3 S249C mutant-binding arm:

| | | |
|---|---|---|
| FGA0002 | SEQ ID NO: 5 | SEQ ID NO: 6 |
| CDR1 | SEQ ID NO: 17 | SEQ ID NO: 20 |
| CDR2 | SEQ ID NO: 18 | SEQ ID NO: 21 |
| CDR3 | SEQ ID NO: 19 | SEQ ID NO: 22 |
| FGA0005 | SEQ ID NO: 7 | SEQ ID NO: 8 and |
| CDR1 | SEQ ID NO: 23 | SEQ ID NO: 26 |
| CDR2 | SEQ ID NO: 24 | SEQ ID NO: 27 |
| CDR3 | SEQ ID NO: 25 | SEQ ID NO: 28 |
| FGA0007 | SEQ ID NO: 9 | SEQ ID NO: 10 |
| CDR1 | SEQ ID NO: 29 | SEQ ID NO: 32 |
| CDR2 | SEQ ID NO: 30 | SEQ ID NO: 33 |
| CDR3 | SEQ ID NO: 31 | SEQ ID NO: 34 |

Antigen-binding domain having T cell receptor complex-binding activity

As used herein, the expression "antigen-binding domain having T cell receptor complex binding activity" refers to a portion of a T cell receptor complex antibody which comprises a region that specifically binds to and is complementary to a part of or the whole T cell receptor complex. The T cell receptor complex may be the T cell receptor itself, or may be an adapter molecule that constitutes the T cell receptor complex together with the T cell receptor. A suitable adapter is CD3.

Antigen binding domain having T cell receptor binding activity

As used herein, the expression "antigen-binding domain having T cell receptor binding activity" refers to part of a T cell receptor antibody which comprises a region that specifically binds to and is complementary to a part of or the whole T cell receptor.

The portion of the T cell receptor to which the domain of the present disclosure binds may be a variable region or a constant region, but is preferably an epitope present in the constant region. The sequences of the constant region include, for example, T cell receptor $\alpha$ chain of RefSeq registration number CAA26636.1, T cell receptor $\beta$ chain of RefSeq registration number C25777, T cell receptor $\gamma$1 chain of RefSeq registration number A26659, T cell receptor $\gamma$2 chain of RefSeq registration number AAB63312.1, and T cell receptor $\delta$ chain of RefSeq registration number AAA61033.1.

Antigen-binding domain having CD3 binding activity

As used herein, the expression "antigen-binding domain having CD3 binding activity" refers to a portion of a CD3 antibody which comprises a region that specifically binds to and is complementary to a part of or all of CD3. Preferably, the domain comprises a light chain variable region (VL) of an anti-CD3 antibody and a heavy chain variable region (VH) of an anti-CD3 antibody. Preferable examples of such domains are "scFv (single chain Fv)", "single chain antibody", "Fv", "scFv2 (single chain Fv 2)", "Fab" or "F (ab'))2", and the like.

The antigen-binding domain having CD3 binding activity according to the present disclosure may bind to any epitope present in the $\gamma$-chain, $\delta$-chain, or $\varepsilon$-chain sequence constituting human CD3. In the present disclosure, a domain comprising the light chain variable region (VL) and the heavy chain variable region (VH) of the anti-CD3 antibody that binds to an epitope present in the extracellular region of the $\varepsilon$ chain of the human CD3 complex is preferably used. As such domains, in addition to the light chain variable region (VL) and the heavy chain variable region (VH) of the anti-CD3 antibody described in the Examples, a CD3 binding domain comprising the light chain variable region (VL) and the heavy chain variable region (VH) of the OKT3 antibody (Proc. Natl. Acad. Sci., USA (1980) 77, 4914-4917) or various known CD3 antibodies is also preferably used. In addition, it is possible to appropriately use an antigen-binding domain derived from an anti-CD3 antibody having desired properties obtained by immunizing a desired animal with the γ chain, δ chain or ε chain constituting human CD3 by an above method. An appropriately humanized antibody or a human antibody as described above is appropriately used as the anti-CD3 antibody that is the origin of the antigen-binding domain having CD3 binding activity. As to the structure of the γ chain, δ chain, or ε chain constituting CD3, the polynucleotide sequence is described in RefSeq registration number NM_00007S.2, RefSeq registration number NM_0007S2.4, and RefSeq registration number NM_000733.3, and the polynucleotide sequence is described in RefSeq registration number NP_000064.1, RefSeq registration number NP_000723.1 and RefSeq registration number NP_000724.1.

The antigen-binding domains in the antigen-binding molecules of the present disclosure can bind to the same epitope. Here, the same epitope can be present in the protein consisting of the amino acid sequence set forth in SEQ ID NO: 3. Alternatively, the antigen-binding domains in the antigen-binding molecules of the present disclosure can bind to different epitopes. Here, different epitopes can be present in the protein consisting of the amino acid sequence set forth in SEQ ID NO: 3.

In one embodiment of the present disclosure, the following amino acid sequences can be exemplified as CDR amino acids for constructing an antigen-binding domain having CD3 binding activity:

| | VH | VL |
|---|---|---|
| CDR1 | SEQ ID NO: 59 | SEQ ID NO: 62 |
| CDR2 | SEQ ID NO: 60 | SEQ ID NO: 63 |
| CDR3 | SEQ ID NO: 61 | SEQ ID NO: 64 |

Method for producing a bispecific antibody

Methods for producing bispecific antibodies are known in the art. Traditional recombinant production of bispecific antibodies is based on the co-expression of two pairs of immunoglobulin heavy chain-light chain, where the two heavy chains have different specificities (Milstein et al., Nature, 305: 537-539 (1983)). Due to the random assortment of immunoglobulin heavy and light chains, these hybridomas (quadromas) produce a possible mixture of 10 different antibody molecules, only one of which has the correct bispecific structure. Purification of the correct molecule, usually performed by affinity chromatography steps, is rather cumbersome and the product yield is low. A similar method is disclosed in WO 93/08829 published on May 13, 1993, Traunecker et al., EMBO J. 10: 3655-3459 (1991).

Another more preferred approach

Another more preferred approach is to fuse antibody variable domains having the desired binding specificities (antigen-antibody binding sites) to immunoglobulin constant domain sequences. The fusion is preferably a fusion with an immunoglobulin heavy chain constant domain comprising at least a portion of the hinge, and CH2 and CH3 regions. It is desirable that the first heavy chain constant region (CHI) containing the site required for light chain binding is present in at least one of the fusions. DNAs encoding the immunoglobulin heavy chain fusions and, if desired, the immunoglobulin light chain, are inserted into separate expression vectors, and co-transfected into a suitable host organism. This provides great flexibility in adjusting the mutual proportions of the three polypeptide fragments, in embodiments where optimal yields are provided by unequal proportions of the three polypeptide chains used in the construct. However, when expression of at least two polypeptide chains in equal proportions results in high yields, or when the proportions are of no particular importance, the coding sequences for two or all three polypeptide chains can be inserted into one expression vector.

In a preferred embodiment of this approach, the bispecific antibody consists of a hybrid immunoglobulin heavy chain for one arm, which has a first binding specificity, and a hybrid immunoglobulin heavy chain-light chain pair for the other arm (providing a second binding specificity). It turned out that this asymmetric structure makes it easier to separate the desired bispecific compound from unwanted immunoglobulin chain combinations, since an easy separation method is provided when only half of the bispecific molecule has an immunoglobulin light chain. This approach is disclosed in WO 94/04690. For further details on producing bispecific antibodies, see, for example, Suresh et al., Methods in Enzymology, 121: 210 (1986).

Other approaches

According to other approaches, the interface between a pair of antibody molecules can be manipulated to maximize the percentage of heterodimers recovered from a recombinant cell culture. Suitable interfaces include at least a portion of the CH3 domain of the antibody constant domain. In this method, one or more small amino acid side chains from the interface of the first antibody molecule are replaced with larger side chains (e.g., tyrosine or tryptophan). Complementary "cavities" of the same or similar size as the large side chains are created at the interface of the second antibody molecule by replacing the large amino acid side chains with smaller ones (*e.g.*, alanine or threonine). This provides a mechanism for increasing the yield of heterodimers over other unwanted end products such as homodimers.

Cross-linked antibodies or "heteroconjugated antibodies" and their manufacturing methods

Bispecific antibodies include cross-linked antibodies and "heteroconjugated antibodies". For example, one antibody of the heteroconjugate may bind to avidin and the other to biotin. Such antibodies have been proposed for, *e.g.*, targeting immune system cells to unwanted cells (U.S. Pat. No. 4676980), and treating HIV infection (WO 91/00360, WO 92/00373 and European Patent No. 03089). Heteroconjugated antibodies can be produced by suitable cross-linking methods. Suitable cross-linking agents are well known in the art and are described in U.S. Pat. No. 4676980 and such, along with multiple cross-linking methods.

Technologies for producing chimeric antigen receptors from antibody fragments

Chimeric antigen receptors

The CAR of the present disclosure can be engineered to include an extracellular domain having an antigen-binding domain fused to the intracellular signaling domain of the T cell antigen receptor complex ζ chain (*e.g.*, CD3ζ). The CAR of the present disclosure can re-induce antigen binding based on antigen binding-specificity, when expressed in T cells. The antigen of the present disclosure is an FGFR3 S249C mutant, and the CAR of the present disclosure includes any antigen-binding domain that, upon binding of the CAR of the present disclosure to the FGFR3 S249C mutant, affects tumor cells making them unable to grow, prompt them to die, or affects in other ways by which the total tumor burden in the patient gradually decreases or disappears. The antigen-binding domain is preferably fused with the intracellular domain from one or more of the co-stimulatory molecules and the ζ chain. The antigen-binding domain is preferably fused with one or more intracellular domains selected from the group of CD137 (4-1BB) signaling domain, CD28 signaling domain, CD3ζ signaling domain, and any combination thereof.

Transmembrane domain

With respect to the transmembrane domain, CAR can be designed to include a transmembrane domain fused with the extracellular domain of CAR. In one embodiment, a transmembrane domain that is naturally attached to one of the domains in CAR is used. In some cases, the transmembrane domain can be selected to avoid binding of such domains to transmembrane domains of the same or different surface membrane proteins such that the interaction with other members of the receptor complex is minimized, or can also be selected or altered by amino acid substitutions for that purpose.

The transmembrane domain may be derived from either a natural or synthetic source. If the source is natural, the domain can be derived from any membrane-binding or transmembrane protein. Transmembrane regions that are particularly useful in the present disclosure can be derived from the α, β or ζ chains of T cell receptors, CD28, CD3ε, CD45, CD4, CD5, CD8, CD9, CD16, CD22, CD33, CD37, CD64, CD80, CD86, CD134, CD137, and CD154 (*i.e.*, including at least their transmembrane regions). Alternatively, the transmembrane domain may be synthetic, in which case it is believed to primarily contain hydrophobic residues such as leucine and valine. Preferably, a triplet of phenylalanine, tryptophan and valine will be found at each end of the synthetic transmembrane domain. Optionally, a short oligopeptide or polypeptide linker, preferably 2-10 amino acids in length, may form a chain between the transmembrane domain of CAR and the cytoplasmic signaling domain. Glycine-serine doublets are particularly suitable linkers.

Cytoplasmic domain

The cytoplasmic domain of the CAR of the present disclosure, or in other words the intracellular signaling domain, is responsible for the activation of at least one of the normal effector functions of immune cells introduced with CAR. The term "effector function" refers to a specialized function of a cell. For example, the effector function of T cells may be cytolytic activity, or helper activity including cytokine secretion. Therefore, the term "intracellular signal transduction domain" refers to the part of the protein that transmits the effector function signal and guides the cell to perform its specialized function. Usually, the entire intracellular signaling domain can be used, but in many cases, it is not necessary to use the entire chain. To the extent that truncated portions of the intracellular signaling domain are used, such shortened portions can be used in place of intact strands as long as they transmit effector function signals. The term intracellular signaling domain shall therefore include any shortened portion of the intracellular signaling domain that is sufficient to transmit an effector function signal.

Preferred examples of intracellular signaling domains for use in the CARs of the present disclosure include T cell receptors (TCRs) and cytoplasmic sequences of co-receptors that act cooperatively to elicit signaling after engagement of an antigen with a receptor, as well as any derivatives or variants of these sequences and any synthetic sequences that have the same functional capacity.

It is known that the signals produced only through the TCR are insufficient for complete activation of T cells, and that secondary or co-stimulatory signals are also required. Thus, it can be said that T cell activation is mediated by two distinct classes of cytoplasmic signaling sequences: those that elicit antigen-dependent primary activation through the TCR (primary cytoplasmic signaling sequences), and those that act in an antigen-independent manner to produce a secondary or co-stimulatory signal (secondary cytoplasmic signaling sequences).

Source of T cells

Prior to T cell growth and genetic alterations of the present disclosure, a source of T cells is obtained from the subject. T cells are available from a number of sources, including peripheral blood mononuclear cells, bone marrow, lymph node tissue, cord blood, thymic tissue, tissue from a site of infection, ascites, pleural effusion, spleen tissue, and tumors. In certain embodiments of the disclosure, any variety of T cell lines available in the art can be used. In certain embodiments of the disclosure, T cells are obtained from blood units collected from a subject using any of a variety of techniques known to those of skill in the art, such as Ficoll™ separation. In one preferred embodiment, cells from blood extracted from an individual are obtained by apheresis. Apheresis products typically include T cells, monocytes, granulocytes, lymphocytes including B cells, other nucleated white blood cells, red blood cells, and platelets. In one embodiment, the cells collected by apheresis can be washed to remove the plasma fraction and then placed in a suitable buffer or medium for subsequent processing steps. In one embodiment of the disclosure, these cells are washed with phosphate buffered saline (PBS). In one alternative embodiment, the washing solution is calcium-free and magnesium-free, or free of, not all, but many bivalent cations. Again, surprisingly, the initial activation step in the absence of calcium results in enhanced activation. As will be readily understood by those skilled in the art, the washing step can be achieved by using methods known in the art, for example, by using a semi-automated "flow-through" centrifuge according to the manufacturer's instructions (e.g., Cobe 2991 cell processor, Baxter CytoMate, or Haemonetics Cell Saver 5). After washing, the cells can be resuspended in various biocompatible buffers, such as $Ca^{2+}$-free, $Mg^{2+}$-free PBS, PlasmaLyte A, or other saline solutions with or without a buffer. Alternatively, the cells may be resuspended directly in the culture medium after removing unwanted components of the apheresis sample.

Activation and proliferation of T cells

Either before or after genetic alteration of T cells to express the desired CAR, T cells can be activated and multiplied generally using methods set forth in, for example, U.S. Pat. Nos. 6,352,694; 6,534,055; 6,905,680; 6,692,964; 5,858,358; 6,887,466; 6,905,681; 7,144,575; 7,067,318; 7,172,869; 7,232,566; 7,175,843; 5,883,223; 6,905,874; 6,797,514; 6, 867,041; and the U.S. Patent Application Publication No. 20060121005.

Generally, the T cells of the present disclosure are multiplied by contact with an agent that stimulates a CD3/TCR complex-related signal and by contact with a surface bound to a ligand that stimulates co-stimulatory molecules on the T cell surface. In particular, as described herein, T cell populations can be stimulated by contact with an anti-CD3 antibody immobilized on the surface, or an antigen-binding fragment thereof, or an anti-CD2 antibody, or by contact with a protein kinase C activator (*e.g.*, bryostatin) with calcium ionophore. A ligand that binds to an accessory molecule on the T cell surface is used for co-stimulation of the accessory molecule. For example, a population of T cells can be contacted with anti-CD3 and anti-CD28 antibodies under conditions suitable for stimulating T cell proliferation. Anti-CD3 antibody and anti-CD28 antibody to stimulate the proliferation of either $CD4^+$ T cells or $CD8^+$ T cells. Examples of anti-CD28 antibodies include 9.3, B-T3, XR-CD28 (Diaclone, Besancon, France) which can be used in the same manner as other methods generally known in the art (Berg et al., Transplant Proc. 30 (8): 3975-3977, 1998; Haanen et al., J. Exp. Med. 190 (9): 13191328, 1999; Garland et al., J. Immunol Meth. 227 (1-2): 53-63, 1999).

Treatment applications

The present disclosure includes within its scope, cells transduced by a lentiviral vector (LV) *(e.g.,* T cells). For example, LV encodes CAR in which the antigen-binding domain of a specific antibody is combined with the intracellular domain of CD3-ζ, CD28, 4-1BB, or any combination thereof. Thus, in some cases, transduced T cells can elicit a CAR-mediated T cell response.

The present disclosure provides the use of CAR to re-induce the specificity of primary T cells towards tumor antigens. Accordingly, the present disclosure provides a method for stimulating a T cell-mediated immune response against a target cell population or tissue in a mammal, comprising the step of administering CAR-expressing T cells to the mammal, wherein said CAR has a binding moiety that specifically interacts with a given target, *e.g.*, a ζ chain portion containing the intracellular domain of human CD3ζ, and a co-stimulating signal transduction region.

In one embodiment, the disclosure comprises a type of cell therapy in which T cells are genetically altered to express CAR, and the CAR T cells are infused into a recipient in need thereof. The infused cells can kill tumor cells in the recipient. Unlike antibody therapy, CAR T cells can replicate *in vivo* to provide long-term persistence that can lead to sustained tumor control.

Techniques for producing bispecific antibodies from antibody fragments

Techniques for producing bispecific antibodies from antibody fragments are also described in literature. For example, chemical bonds can be used to prepare bispecific antibodies. Brennan et al., Science, 229: 81 (1985) describe a procedure for proteolytically cleaving a complete antibody to produce an F(ab')2 fragment. These fragments are reduced in the presence of the dithiol complex-forming agent, sodium arsenate to stabilize adjacent dithiol and prevent intermolecular disulfide formation. The Fab' fragment produced is then converted to a thionitrobenzoate (TNB) derivative. One of the Fab'-TNB derivatives is then reconverted to Fab'-thiol by reduction with mercaptoethylamine and mixed with an equimolar amount of another Fab'-TNB derivative to form a bispecific antibody. The bispecific antibody produced can be used as a drug for selective immobilization of the enzyme.

Recovering Fab'-SH fragments directly from *E. coli*

Recent advances have made it easier to recover Fab'-SH fragments directly from *E. coli,* whereby bispecific antibodies are formed through chemical coupling. Shalaby, et al., J.M. Exp. Med., 175: 217-225 (1992) describe the production of fully humanized bispecific antibody F(ab')2 molecules. Each Fab' fragment is secreted separately from *E. coli* and chemically coupled *in vitro* to form a bispecific antibody. Thus, the bispecific antibody formed was able to not only bind to HER2-overexpressing cells and normal human T cells, but also induce lytic activity of human cytotoxic lymphocytes against of human thoracic tumor targets.

Methods for creating and separating bispecific antibody fragments

Various methods for creating and separating bispecific antibody fragments directly from recombinant cell cultures have also been described. For example, bispecific antibodies have been produced using leucine zippers (Kostelny et al., J. Immunol., 148 (5): 1547-1553 (1992)). Leucine zipper peptides from the Fos and Jun proteins were genetically fused to Fab' portions of two different antibodies. The antibody homodimer was reduced in the hinge region to form a monomer, and then reoxidized to form an antibody heterodimer. This method can also be used for the production of antibody homodimers. The "diabody" technique described by Hollinger et al., Proc. Natl. Acad. Sci. USA, 90: 6444-6448 (1993) provided another mechanism for producing bispecific antibody fragments. The fragments are formed by binding a heavy chain variable domain (VH) to a light chain variable domain (VL) with a linker short enough to allow pairing between two domains on the same chain. Thus, the VH and VL domains of one fragment are forcibly paired with the complementary VL and VH domains of the other fragment to form two antigen binding sites. Other bispecific antibody fragment production strategies using single chain Fv (sFv) dimers have also been reported. See Gruber et al., J. Immunol., 152: 5368 (1994).

Antibodies that are more than bivalent are also conceivable. For example, a tri-specific antibody can be prepared (Tutt et al., J. Immunol. 147: 60 (1991)).

Multivalent antibodies

A multivalent antibody can be internalized (and/or catabolized) faster than a bivalent antibody by cells expressing the antigens to which the antibody binds. The antibodies of the present disclosure can be multivalent antibodies (which are other than those of the IgM class) with three or more binding sites (e.g., tetravalent antibodies) and can be easily produced by recombinant expression of nucleic acids encoding the polypeptide chains of the antibody. Multivalent antibodies have a dimerization domain and three or more antigen binding sites. Preferred dimerization domains comprise an Fc region or a hinge region (or consist of them).

In this scenario, the antibody would have an Fc region and three or more antigen binding sites at the amino terminus of the Fc region. The preferred multivalent antibodies herein comprise (or consist of) three to eight, preferably four, antigen binding sites. Multivalent antibodies have at least one polypeptide chain (preferably two polypeptide chains), and the polypeptide chain(s) have two or more variable domains.

For example, the polypeptide chain(s) comprises VD1-(X1)n-VD2-(X2)n-Fc, where VD1 is the first variable domain and VD2 is the second variable domain, Fc is one of the polypeptide chains of the Fc region, X1 and X2 represent amino acids or polypeptides, and n is 0 or 1. For example, the polypeptide chain(s) can comprise: VH-CH1-flexible linker-VH-CH1-Fc region chain; or VH-CH1-VH-CH1-Fc region chain. The multivalent antibody herein preferably further comprises at least two (preferably four) light chain variable domain polypeptides. The multivalent antibody herein has, for example, about two to about eight light chain variable domain polypeptides. The light chain variable domain polypeptides discussed herein have a light chain variable domain and, in some cases, an additional CL domain.

Antibody variants

In some embodiments, amino acid sequence modifications of the antibodies disclosed herein is contemplated. For example, it may be desirable to improve the binding affinity and/or biological properties of the antibody. Amino acid sequence variants of an antibody are prepared by introducing appropriate nucleotide changes into the nucleic acid of the antibody or by peptide synthesis. Such modifications include, for example, deletions, insertions or substitutions of residues within the amino acid sequence of the antibody. Deletions, insertions or substitutions may be combined in any way as long as the final composition has the desired characteristics. Amino acid changes can be introduced into the antibody amino acid sequence of the subject when the sequence is made.

"Alanine scanning mutagenesis"

A useful method for identifying a particular residue or region of an antibody that is a preferred position for mutagenesis is "alanine scanning mutagenesis" as disclosed in Science, 244: 1081-1085 (1989) by Cunningham and Wells. Here, the target residue or set of residues is identified (e.g., charged residues such as arg, asp, his, lys, and glu) and substituted with neutral or negatively charged amino acids (most preferably, alanine or polyalanine), to influence the interaction of amino acids with antigens. Those amino acid positions exhibiting functional susceptibility to the substitution are then purified by introducing additional or other variants at, or for, the site of substitution. As described above, the site for introducing the amino acid sequence variant is predetermined, but the nature of the mutation itself does not need to be determined in advance. For example, to analyze the performance of the mutation at any site, ala scanning or random mutagenesis is performed at the target codon or region, and the expressed immunoglobulin is screened for the desired activity.

Amino acid sequence insertion includes amino-terminal fusions and/or carboxy-terminal fusions ranging in length from one residue to polypeptides having 100 or more residues, as well as intrasequence insertions of single or multiple amino acid residues. Examples of terminal insertions include antibodies with N-terminal methionyl residues, or antibodies fused to cytotoxic polypeptides. Other insertional variants of the antibody molecule include fusion of polypeptides that increase the serum half-life of antibodies, or *(e.g.,* for ADEPT) fusion of enzymes to the N-terminus or C-terminus of the antibody.

Glycosylation of polypeptides

Glycosylation of a polypeptide is typically either N-linked or O-linked. N-linked means the binding of the carbohydrate moiety to the side chain of an asparagine residue. The tripeptide sequences of asparagine-X-serine and asparagine-X-threonine (where X is any amino acid except proline) are binding sequences for the enzymatic binding of the sugar chain moiety to the asparagine side chain. Thus, the presence of any of these tripeptide sequences in a polypeptide creates a potential glycosylation site. O-linked glycosylation means that one of the sugars N-acetyl galactosamine, galactose, or xylose binds to a hydroxyamino acid, most commonly serine or threonine, although 5-hydroxyproline or 5-hydroxylysine is also used.

Addition of a glycosylation site to an antibody

Addition of a glycosylation site to an antibody is conveniently accomplished by altering the amino acid sequence such that it contains one or more of the above-mentioned tripeptide sequences (for N-linked glycosylation sites). The alteration may also be made by the addition of or substitution with one or more serine or threonine residues to the original antibody sequence (for O-linked glycosylation sites).

If the antibody contains an Fc region, the carbohydrate attached thereto may be altered. For example, antibodies having a mature carbohydrate structure lacking fucose attached to an Fc region of the antibody is described in U.S. Patent Application No. 2003/0157108 (Presta, L.). See also U.S. Patent Application No. 2004/093621 (Kyowa Hakko Kogyo Co., Ltd.). An antibody with a bisecting N-acetylglucosamine (GlcNAc) within the carbohydrate attached to an Fc region of the antibody is referred to in WO 03/011878, Jean-Mairet et al., and U.S. Pat. No. 6,602,684, Umana et al. Antibodies with at least one galactose residue in the oligosaccharide attached to an Fc region of the antibody are reported in WO 97/30087, Patel et al. See also WO 98/58964 (Raju, S.) and WO 99/22764 (Raju, S.) for antibodies with altered carbohydrates attached to an Fc region of the antibody. For antigen-binding molecules with modified glycosylation, see, U.S. Patent Application No. 2005/0123546 (Umana et al.).

Preferred glycosylation variants in the present specification contain an Fc region, and the carbohydrate structure attached to the Fc region lacks fucose. Such variants have improved ADCC function. In some cases, the Fc region further comprises one or more amino acid substitutions that further improve ADCC, such as substitutions at positions 298, 333 and/or 334 of the Fc region (Eu numbering of residues). Examples of literature relating to "defucosylated" or "fucose-deficient" antibodies include: US 2003/0157108; WO 2000/61739; WO 2001/29246; US 2003/0115614; US 2002/0164328; 2004/0093621; US 2004/0132140; US 2004/0110704; US 2004/0110282; US 2004/0109865; WO 2003/085119; WO 2003/084570; WO 2005/035586; WO 2005/035778; WO 2005/053742; Okazaki et al. Mol. Biol. 336: 1239-1249 (2004); and Yamane-Ohnuki et al. Biotech. Bioeng. 87: 614 (2004). Examples of cell lines producing defucosylated antibodies include Lec13 CHO cells deficient in protein fucosylation (Ripka et al. Arch. Biochem. Biophys. 249:533-545 (1986); U.S. Pat Appl No US 2003/0157108, Presta, L; and WO 2004/056312, Adams et al., especially at Example 11), and knockout cell lines, such as alpha-1,6-fucosyltransferase gene, FUT8,-knockout CHO cells (Yamane-Ohnuki et al. Biotech. Bioeng. 87: 614 (2004)) and the like.

Amino acid substitution variants

Other types of variants are amino acid substitution variants. These variants have at least one amino acid (at least two, at least three, at least 4 or more) residue in the antibody molecule replaced by a different residue. The sites of greatest interest for substitutional mutagenesis include hypervariable regions, but FR alternations are also considered. Conservative substitutions are shown in Table 1. If these substitutions result in altered biological activity, further substantial modifications may be introduced, and the products may be screened.

[Table 1]

| Conservative substitutions | |
|---|---|
| Acidic residues | Asp (D) and G 1 u (E) |
| Basic residues | Lys (K), Arg (R), and His (H) |
| Hydrophilic uncharged residues | Ser (S), Thr (T), Asn (N), and Gln (Q) |
| Aliphatic uncharged residues | Gly (G), Ala (A), Val (V), Leu (L), and Ile (I) |
| Non-polar uncharged residues | Cys (C), Met (M), and Pro (P) |
| Aromatic residues | Phe (F), Tyr (Y), and Trp (W) |

Substantial modifications in the biological properties of an antibody

Substantial modifications in the biological properties of an antibody are achieved by choosing substitutions that substantially differ in their effect on maintaining (a) the structure of the polypeptide backbone in the area of substitution, e.g., as a sheet or helical conformation, (b) the charge or hydrophobicity of the molecule at the target site, or (c) the bulk of the side chain. Naturally-occurring residues can be grouped based on common side chain properties:

(1) Hydrophobic: norleucine, met, ala, val, leu, ile;
(2) Neutral and hydrophilic: cys, ser, thr, asn, gin;
(3) Acidic: asp, glu;
(4) Basic: his, lys, arg;
(5) Residues affecting chain orientation: gly, pro; and
(6) Aromatic: trp, tyr, phe.

Non-conservative substitutions would require exchanging one member of one of these classifications for another.

Substitution of one or more hypervariable region residues of a parent antibody (*e.g.*, humanized or human antibody)

Certain types of substitutional variants involve the substitution of one or more hypervariable region residues of a parent antibody (*e.g.*, humanized or human antibody). In general, the resulting variants selected for further development have improved biological properties compared to the parent antibody from which they were made. Convenient methods for making such substitutional variants include affinity mutation using phage display. Briefly, several hypervariable region sites *(e.g.,* 6-7 sites) are mutated to produce all possible amino acid substitutions at each site. The multivalent antibodies thus produced are displayed from filamentous phage particles as fusions to the gene III product of M13 packed within each particle. Phage-displayed variants are then screened for their biological activity (*e.g*., binding affinity) as disclosed herein. To identify candidate hypervariable region sites for modification, alanine scanning mutagenesis can be performed to identify hypervariable region residues that significantly contribute to antigen binding. Alternatively, or in addition, it may be advantageous to analyze the crystal structure of the antigen-antibody complex to identify the point of contact between the antibody and the antigen. Such contact and flanking residues are candidates for substitution according to the techniques described herein. Once such variants are generated, the variants panel is screened as described herein and antibodies with superior properties in one or more related assays are selected for further development.

Nucleic acid molecules encoding amino acid sequence variants of antibodies

Nucleic acid molecules encoding amino acid sequence variants of antibodies are prepared by various methods known in the art. These methods include, without limitation, isolation from a natural source (in the case of naturally occurring amino acid sequence variants) or preparation by oligonucleotide-mediated (or site-directed) mutagenesis, PCR mutagenesis, and cassette mutagenesis of an earlier prepared variant or a non-variant version of the antibody.

Fc region variants

It is desirable to introduce one or more amino acid modifications into an Fc region of the immunoglobulin polypeptides of the present disclosure to generate Fc region variants. Fc region variants can include human Fc region sequences (*e.g*., human IgG1, IgG2, IgG3 or IgG4 Fc regions) that have an amino acid modification (*e.g*., substitution) at one or more amino acid positions, including hinge cysteine modifications.

In accordance with the description and teachings in the art, in certain embodiments, it is considered that an antibody prepared using a method of the present disclosure has one or more mutations, for example, in the Fc region, as compared to the corresponding wild-type antibody. Nevertheless, this antibody retains substantially the same characteristics required for therapeutic utility compared to its wild-type counterpart. For example, as described in WO 99/51642 and such, it is conceivable to cause a specific mutation in the Fc region that results in altering (*i.e*, improving or reducing) C1q binding and/or complement-dependent cytotoxicity (CDC). See also Duncan & Winter Nature 322: 738-40 (1988); U.S. Pat. No. 5,648,260; U.S. Pat. No. 5,624,821; and WO 94/29351 for other examples of Fc region variants. WO 00/42072 (Presta) and WO 2004/056312 (Lowman) disclose antibody variants with improved or diminished binding to FcRs. The contents of these patent documents are particularly incorporated herein by reference. See also, Shields et al. J. Biol. Chem. 9 (2): See 6591-6604 (2001). Antibodies with increased half-life and improved binding to the neonatal Fc receptor (FcRn), which is responsible for the transfer of maternal IgGs to the fetus (Guyer et al., J. Immunol. 117: 587 (1976) and Kim et al., J. Immunol. 24: 249 (1994)) are disclosed in US 2005/0014934A1 (Hinton et al.). These antibodies comprise an Fc region with one or more substitutions that enhance the binding of the Fc region to FcRn. Polypeptide variants in which the Fc region amino acid sequence has been altered to increase or decrease C1q binding capacity are disclosed in U.S. Pat. No. 6,194,551 B1 and WO 99/51642. The contents of these patent documents are particularly incorporated herein by reference. See also, Idusogie et al. J. Immunol. 164: See 4178-4184 (2000).

Antibody derivatives

The antibodies of the present disclosure can be further modified to include additional non-proteinaceous moieties known in the art that are readily available. Preferably, the moieties suitable for derivatization of the antibody are water-soluble polymers. Non-limiting examples of water-soluble polymers include, but are not limited to, polyethylene glycol (PEG), ethylene glycol/propylene glycol copolymers, carboxymethyl cellulose, dextran, polyvinyl alcohol, polyvinylpyrrolidone, poly-1,3-dioxolan, poly-1,3,6-trioxane, ethylene/maleic anhydride copolymers, polyamino acids (homopolymers or random copolymers), and dextran or poly (n-vinylpyrrolidone) polyethylene glycols, propylene glycol homopolymers, polypropylene oxide/ethylene oxide copolymers, polyoxyethylene polyol (e.g., glycerol), polyvinyl alcohol, and mixtures thereof. Polyethylene glycol propionaldehyde will be advantageous in production due to its stability in water. The polymer may have any molecular weight and may be branched or unbranched. The number of polymers attached to the antibody may vary, and if more than one polymer is attached, they may be the same or different molecules. In general, the number and/or type of polymers used for derivatization can be determined based on considerations including, but not limited to, the particular properties or functions of the antibody to be improved, whether the antibody derivative will be used in a therapy under defined conditions, etc.

Screening for antibodies with the desired properties

The antibodies of the present disclosure can be characterized for their physical/chemical properties and biological functions by various assays known in the art (some of which are disclosed herein). In some embodiments, the antibody has either one or several characteristics of reducing or blocking FGF *(e.g.,* FGF1 and/or FGF9) binding, reducing or blocking FGFR3 activation, reducing or blocking signaling of FGFR3 downstream molecules, disrupting or blocking FGFR3 binding to ligands (e.g., FGF1, FGF9), reducing or blocking FGFR3 dimerization, promoting the formation of monomeric FGFR3, binding to monomeric FGFR3, preventing and/or treating a tumor, cell proliferative disorder or cancer; and/or preventing or treating a disease associated with FGFR3 expression and/or activity of FGFR3 (e.g., increased expression and/or activity of FGFR3). In some embodiments, antibodies are screened for increased FGFR3 activation, increased signaling of FGFR3 downstream molecules, apoptotic activity, FGFR3 downregulation, and effector function (*e.g.*, ADCC activity).

Antibody production and structural analysis

Purified antibodies can be further charactered by a series of assay including, but not limited to, N-terminal sequencing, amino acid analysis, non-denaturing size exclusion high pressure liquid chromatography (HPLC), mass spectrometry, ion exchange chromatography and papain digestion.

Biological activity analysis of antibodies

In certain embodiments of the present disclosure, the antibodies produced herein are analyzed for their biological activity. In certain embodiments, the antibodies of the present disclosure are tested for their antigen-binding activity. Antigen binding assays known in the art and used herein include, without limitation, any direct or competitive binding assay using techniques such as Western blot, radioimmunoassay, ELISA (enzyme-linked immunosorbent assay), "sandwich" immunoassay, immunoprecipitation assay, fluorescent immunoassay, and protein A immunoassay. Antigen binding and other assays are described in the Examples section below.

Measurement of antibody cell growth inhibitory activity

If an anti-FGFR3 S249C mutant antibody that inhibits cell growth is desired, candidate antibodies may be tested in *in vitro* and/or *in vivo* assays that measure inhibition of cell growth. If an anti-FGFR3 S249C mutant antibody that promotes or does not promote apoptosis is desired, the candidate antibody may be tested in an assay that measures apoptosis. Methods for examining the growth and/or proliferation of cancer cells or for determining apoptosis of cancer cells are well known in the art and some are described and exemplified herein. Illustrative methods for determining cell growth and/or proliferation or apoptosis include, for example, BrdU uptake assay, MTT, [3H]-thymidine uptake (e.g., TopCount assay (PerkinElmer)), cell viability assay (e.g., CellTiter-Glo (Promega)), DNA fragmentation assay, caspase activation assay, trypan blue exclusion, chromatin morphology assay, and the like.

Fc activity of antibodies with effector function

"Effector function" refers to the biological activity caused by the Fc region of an antibody, which differs depending on the isotype of the antibody. Examples of antibody effector function include: C1q binding and complement-dependent cytotoxicity (CDC); Fc receptor binding; antibody-dependent cell-mediated cytotoxicity: ADCC); phagocytosis; downregulation of cell surface receptors (e.g., B cell receptors); and B cell activation. In one embodiment, the present disclosure is intended for an antibody having an effector function. In certain embodiments, the Fc activity of the antibody is measured. *In vivo* and/or *in vitro* cytotoxicity assays can be performed to confirm reduced/depleted CDC and/or ADCC activity. For example, an Fc receptor (FcR) binding assay can be performed to confirm that the antibody is deficient in FcyR binding *(i.e.,* almost deficient in ADCC activity) but maintains FcRn binding capacity. NK cells, which are the first cells involved in ADCC, express only FcyRIII, while mononuclear cells express FcyRI, FcyRII and FcyRIII. FcR expression in hematopoietic cells, is summarized in Table 3 on page 464 of Ravetch and Kinet, Annu. Rev. Immunol 9: 457-92 (1991). Examples of *in vitro* assays for assessing ADCC activity of molecules of interest are described in U.S. Pat. No. 5,500,362 or 5,821,337. Assays for detecting ADCC activity are also exemplified herein. Effector cells useful for such assays include peripheral blood mononuclear cells (PBMC) and natural killer (NK) cells. Alternatively, or in addition, the ADCC activity of the molecule of interest can be evaluated *in vivo* within an animal model as disclosed in, for example, Clynes et al. PNAS (USA) 95: 652-656 (1998). A C1q binding assay may also be performed to confirm that the antibody cannot bind to C1q, that is, it lacks CDC activity. To evaluate complement activation, a CDC assay may be performed as described in, for example, Gazzano-Santoro et al., J. Immunol. Methods 202: 163 (1996). In addition, FcRn binding and *in vivo* clearance/half-life measurements can be performed using methods known in the art.

Vectors, host cells and recombination methods

For recombinant production of the antibodies of the present disclosure, the encoding nucleic acid is isolated and inserted into a replicable vector for further cloning (amplification of DNA) or for expression. The DNA encoding the antibody is easily isolated and sequenced by conventional procedures (e.g., using oligonucleotide probes capable of specifically binding to the genes encoding the heavy and light chains of the antibody). Many vectors are available for use. The vector is selected depending to some extent on the host cell used. In general, suitable host cells are cells from prokaryotes or eukaryotes (generally mammals). Constant regions of any isotype may be used for this purpose, including IgG, IgM, IgA, IgD and IgE constant regions, and it will be understood that such constant regions can be obtained from either human or animal species.

a. Antibody production using prokaryotic host cells

i. Vector construction

The polynucleotide sequence encoding the polypeptide component of an antibody of the present disclosure can be obtained using standard recombinant techniques. The desired polynucleotide sequence can be isolated and sequenced from antibody-producing cells such as hybridomas. Alternatively, the polynucleotide can be synthesized using a nucleotide synthesizer or PCR method. Once obtained, the polypeptide-encoding sequence is inserted into a recombinant vector capable of replicating and expressing a heterologous polynucleotide in a prokaryotic host. Many vectors available and known in the art can be used for the purposes of this disclosure. The choice of a suitable vector depends primarily on the size of the nucleic acid inserted into the vector and the particular host transformed with the vector. Each vector contains a variety of components, depending on its function (amplification and/or expression of heterologous polynucleotides) and compatibility with the particular host cell to which it belongs. In general, but without limitation, vector components include an origin of replication, a selectable marker gene, a promoter, a ribosome binding site (RBS), a signal sequence, the heterologous nucleic acid insert and a transcription termination sequence.

Generally, plasmid vectors containing a replicon and control sequences derived from species compatible with the host cell is used in connection with these hosts. The vector usually has a replication origin as well as a marking sequence capable of providing phenotypic selection in transformed cells. For example, *E. coli* is generally transformed with pBR322, a plasmid derived from an *E. coli* species. Since pBR322 contains ampicillin (Amp) and tetracycline (Tet) resistance coding genes, transformed cells can be easily identified. pBR322, its derivatives, or other microbial plasmids or bacteriophages also include or are modified to include promoters that can be used by the microorganism for expressing exogenous proteins. Examples of pBR322 derivatives used to express certain antibodies are described in detail in Carter et al., U.S. Pat. No. 5,648,237.

In addition, phage vectors containing replicon and control sequences that are compatible with the host microorganism can be used as transforming vectors in connection with these hosts. For example, λGEM.TM-11 and such bacteriophages can be utilized in making recombinant vectors that can be used to transform susceptible host cells such as *E. coli* LE392.

An expression vector of the present disclosure may contain two or more promoter-cistron (translation unit) pairs encoding each polypeptide component. The promoter is an untranslated sequence located upstream (5') to a cistron that regulates its expression. Typically, there are two classes of prokaryotic promoters, inducible and constitutive. An inducible promoter is a promoter that induces an increased level of transcription of a cistron under its control in response to changes in culture conditions, such as the presence or absence of a nutrient or a change in temperature.

Promoters

Various promoters that are bound by various potential host cells are known. The promoter of choice can be operably linked to the cistron DNA encoding the light or heavy chain by removing the promoter from the source DNA by restriction enzyme digestion and inserting the isolated promoter into a vector of the present disclosure. Both native promoter sequences and many heterologous promoters can be used to cause amplification and/or expression of the target gene. In certain embodiments, heterologous promoters are useful because they transcribe more commonly expressed target genes and improve efficiency as compared to native target polypeptide promoters.

Promoters suitable for use in prokaryotic hosts

Promoters suitable for use in prokaryotic hosts include PhoA promoters, β-galactamase and lactose promoter systems, tryptophan (trp) promoter systems and hybrid promoters such as tac or trc promoters. However, other promoters that are functional in bacteria (e.g., other known bacterial or phage promoters) are also suitable. These nucleotide sequences have been published, and one of ordinary skill in the art can operably ligate them to cistrons encoding the target light and heavy chains using linkers or adapters that supply any required restriction sites.
(Siebenlist et al. (1980) Cell 20: 269).

Cistrons in recombinant vectors

In one embodiment of the present disclosure, each cistron in the recombinant vector comprises a secretion signal sequence component that induces transcription of the polypeptide expressed across the membrane. In general, the signal sequence can be a component of the vector or part of the target polypeptide DNA inserted into the vector. The signal sequence selected for the purposes of the present invention must be one that is bound and processed by the host cell *(i.e.,* cleaved by a signal peptidase). For prokaryotic host cells that do not bind and process signal sequences native to the heterologous polypeptides, the signal sequence can be replaced by a prokaryotic signal sequence selected from the group consisting of, for example, alkaline phosphatase, penicillinase, Ipp or heat-stable enterotoxin II (STII) leaders, LamB, PhoE, PelB, OmpA and MBP. In one embodiment, the signal sequence used in both cistrons in the expression system are the STII signal sequences or variants thereof.

In another embodiment, the presence of a secretion signal sequence in each cistron is not required because the immunoglobulins according to the present disclosure are produced in the cytoplasm of the host cell. In this regard, immunoglobulin light and heavy chains are expressed, folded and assembled to form functional immunoglobulins in the cytoplasm. There are host systems that exhibit favorable cytoplasmic conditions for disulfide bond formation and enable proper folding and assembling of expressed protein subunits (*e.g.*, *E. coli* trxB-system). Proba and Pluckthun Gene, 159: 203 (1995).

Prokaryotic host cells

Prokaryotic host cells suitable for expressing the antibodies of the present disclosure include archaebacteria and eubacteria, such as Gram-negative or Gram-positive organisms. Examples of useful bacteria include Escherichia (*e.g.*, *E. coli*), Bacillus (*e.g.*, *Bacillus subtilis*), Enterobacteria, Pseudomonas ((*e.g.*, *Pseudomonas aeruginosa),* Salmonella typhimurium, Serratia (*Serratia marcescens*)*,* Klebsiella, Proteus, Shigella, Rhizobia, Vitreoscilla or Paracoccus. In one embodiment, gram-negative bacteria are used. In one embodiment, *E. coli* cells are used as the host of the present disclosure. As an example of an *E. coli* strain, the W3110 strain (Bachmann, Cellular and Molecular Biology, vol. 2 (Washington, D.C. American Society for Microbiology, 1987), pp. 1190-1219; ATCC Deposit No. 27,325) and its derivatives, including the 33D3 strain having the genotype W3110 ΔfhuA (ΔtonA) ptr3 lac Iq lacL8 ΔompTΔ (nmpc-fepE) degP41 kanR (U.S. Pat. No. 5,639,635) are preferable. Further, other strains and derivatives thereof such as Escherichia coli e , Escherichia coli B, Escherichia coli λ 1776 (ATCC 31,537) and Escherichia coli RV308 (ATCC 31,608) are also suitable. These are mere examples and are not supposed to be limiting. Methods of constructing derivatives of any of the above bacteria having defined genotypes are known to those of skill in the art and are described, for example, in Bass et al., Proteins, 8: 309-314 (1990). In general, it is necessary to select a suitable bacterium considering the replication ability of replicon in bacterial cells. When supplying a replicon using a well-known plasmid such as pBR322, pBR325, pACYC177, or pKN410, for example, *Escherichia coli,* Serratia, or Salmonella species can be preferably used as the host. Typically, the host cell must secrete a minimal amount of proteolytic enzyme, and preferably, additional protease inhibitors can be introduced into the cell culture.

ii. Antibody production

The host cells are transformed or transfected with an above-described expression vector, and grown in a conventional nutrient media modified to be suitable for inducing promoters, selecting transformants, or amplifying genes that encoding the desired sequences.

Transformation means introducing DNA into a prokaryotic host to make the DNA replicable as an extrachromosomal element or by chromosomal integrant. Depending on the host cell used, transformation is done using standard techniques suitable for such cells. Calcium treatment with calcium chloride is commonly used for bacterial cells with substantial cell wall barriers. Another method for transformation uses polyethylene glycol/DMSO. Yet another method is electroporation.

Prokaryotic cells used to produce the polypeptides of the present disclosure

The prokaryotic cells used to produce the polypeptides of the present disclosure are known in the art and are grown in media suitable for culturing selected host cells. Examples of suitable media include Luria broth (LB) plus essential nutrient supplements. In certain embodiments, the medium also comprises a selection agent selected based on the composition of the expression vector to selectively allow the growth of prokaryotic cells containing the expression vector. For example, ampicillin is added to the growth medium of cells expressing the ampicillin resistant gene.

Any required supplement in addition to carbon, nitrogen and inorganic phosphate sources can be contained at appropriate concentrations introduced alone or as a mixture with other supplements or media such as composite nitrogen sources. In some cases, the culture medium may contain one or more reducing agents selected from the group consisting of glutathione, cysteine, cystamine, thioglycolate, dithioerythritol and dithiothreitol.

Culture temperature of prokaryotic host cells

Prokaryotic host cells are cultured at an appropriate temperature. For example, for the growth of *E. coli,* the preferred temperature is in the range of about 20 °C to about 39 °C, more preferably about 25 °C to about 37 °C, and even more preferably about 30 °C. The pH of the medium can be any pH in the range of about 5 to about 9, depending primarily on the host organism. For *E. coli,* the pH is preferably from about 6.8 to about 7.4, more preferably about 7.0.

Promoters

When an inducible promoter is used in an expression vector of the present disclosure, protein expression is induced under conditions suitable for promoter activation. In one embodiment of the disclosure, a PhoA promoter is used for transcriptional regulation of the polypeptide. Therefore, transformed host cells are cultured in phosphate-limiting medium for induction. Preferably, the phosphate-limiting medium is the C.R.A.P medium (see, for example, Simmons et al., J. Immunol Methods (2002), 263: 133-147). Various other inducing factors may be used as known to those of skill in the art depending on the vector construct used.

Protein recovery and sequence identification

In one embodiment, the expressed polypeptide of the present disclosure is secreted into and recovered from the periplasm of host cells. Protein recovery generally involves disrupting the microorganism by means such as osmotic shock, sonication, or lysis. Once the cells are disrupted, the cell debris or whole cells can be removed by centrifugation or filtration. The proteins can be further purified, for example, by affinity resin chromatography. Alternatively, the proteins can be transported to culture medium where it can be isolated. Cells can be removed from the culture and the culture supernatant is filtered and concentrated for further purification of the proteins produced. The expressed polypeptide can be further isolated, and identified using commonly known methods such as polyacrylamide gel electrophoresis (PAGE) and Western blot assay.

Large-scale fed-batch fermentation process for the production of recombinant proteins

In one aspect of the disclosure, antibody production is taken over by fermentation processes in large quantities. Various large-scale fed-batch fermentation processes can be used for the production of recombinant proteins. Large-scale fermentation has a volume of at least 1000 liters, preferably about 1000 to 100,000 liters. The fermenters use stirring blades that disperse oxygen and nutrients, especially glucose (a preferred carbon/energy source). Small-scale fermentation generally means fermentation in a fermenter with a volume of about 100 liters or less, which can range from about 1 liter to about 100 liters.

Fermentation process

In fermentation, the induction of protein expression is typically started when the cells have grown to the desired density under appropriate conditions, e.g., about 180-220 OD550, at the stage where the cells are in the early stationary phase. As known and described above in the art, various inducers can be used depending on the vector construct used. Cells may be grown for a short period of time prior to induction. Cells are usually induced for about 12-50 hours, but this may be longer or shorter.

Fermentation conditions

Various fermentation conditions can be modified to improve the production yield and quality of the polypeptides disclosed in the present disclosure. For example, to improve the proper assembly and folding of secreted antibody polypeptides, further vectors that overexpress the chaperone proteins such as Dsb proteins (DsbA, DsbB, DsbC, DsbD and/or DsbG) or FkpA (peptidyl propyl cis/trans-isomerase with chaperone activity) can be used to co-transform host prokaryotic cells. Chaperone proteins have been demonstrated to facilitate proper folding and solubility of heterologous proteins produced in bacterial host cells. See Chen et al. (1999) J Bio Chem 274: 19601-1605; Georgiou et al., U.S. Pat. No. 6083715; Georgiou et al., U.S. Pat. No. 6027888; Bothmann and Pluckthun (2000) J. et al. Biol. Chem. 275: 17100-17105; Ramm and Pluckthun (2000) J. et al. Biol. Chem. 275: 17106-17113; Arie et al. (2001) Mol. Microbiol. 39: 199-210.

Host strain

Certain host strains lacking proteolytic enzymes can be used in the present disclosure in order to minimize proteolysis of expressed heterologous proteins (particularly those susceptible to proteolysis). For example, a prokaryotic host cell line may be altered to cause mutations in a gene encoding a known bacterial protease such as Protease III, OmpT, DegP, Tsp, Protease I, Protease Mi, Protease V, Protease VI, and combinations thereof. Several *E. coli* protease-deficient strains are available, for example, see Joly et al. (1998); Georgio et al., U.S. Pat. No. 5264365; Georgiou et al., U.S. Pat. No. 5508192; Hara et al., (1996), Microbial Drug Resistance 2: 63-72.

In one embodiment, an *E. coli* strain lacking proteolytic enzymes and transformed with a plasmid that overexpresses one or more chaperone proteins is used as the host cell of the expression system of the present disclosure.

iii. Antibody purification

A standard protein purification method known in the art can be used. The following methods are examples of suitable purification procedures: fractionation using immunoaffinity or ion exchange columns, ethanol precipitation, reverse phase HPLC, chromatography with positive exchange resins such as silica or DEAE, chromatofocusing, SDS-PAGE, ammonium sulfate precipitation method, and gel filtration method using, for example, Sephadex G-75.

Immunoaffinity purification method for full-length antibody products of the disclosure using Protein A

In one embodiment, protein A immobilized on a solid phase is used in the immunoaffinity purification method of a full-length antibody product of the present disclosure. Protein A is a 41 kD cell wall protein isolated from *Staphylococcus aureus* that binds to an Fc region of an antibody with a high affinity (Lindmark et al. (1983) J. Immunol., Meth. 62: 1-13). The solid phase on which the protein A is immobilized is preferably a column containing a glass or silica surface, more preferably a controlled pore glass column or a silicic acid column. In some methods, the column is coated with a reagent such as glycerol to prevent non-specific adhesion of contaminants.

In the first step of purification, a cell culture preparation is applied to the protein A immobilized solid phase as described above, and the target antibody is specifically bound to protein A. The solid phase is then washed to remove contaminants that are non-specifically bound to the solid phase. Finally, the antibody of interest is recovered from the solid phase by elution.

b. Production of antibodies using eukaryotic host cells

In general, a vector includes, without limitation, one or more of the followings: a signal sequence, an origin of replication, one or more marker genes, an enhancer element, a promoter, and a transcription termination sequence.

(i) Signal sequence component

Vectors used in eukaryotic host cells may contain a signal sequence or another polypeptide having a specific cleavage site at the N-terminus of the mature protein or target polypeptide. A preferably selected heterologous signal sequence is one that is bound and processed by the host cell *(i.e.,* cleaved by a signal peptidase). For expression in mammalian cells, mammalian signal sequences as well as viral secretory leader sequences such as the herpes simplex gD signal are available.

The DNA of such a precursor region is ligated in reading frame to the DNA encoding the multivalent antibody.

(ii) Origin of replication

In general, a mammalian expression vector does not require a replication origin component. For example, the SV40 origin is typically used simply because it has an early promoter.

(iii) Selectable marker

Expression and cloning vectors contain a selection gene, also referred to as a selectable marker. Typical selection genes encode proteins that (a) confer resistance to antibiotics or other toxins such as ampicillin, neomycin, methotrexate or tetracycline, (b) complement auxotrophic deficiencies if necessary, or (c) provide important nutrients not available from complex media.

In one example of the selection method, a drug that suppresses the growth of host cells is used. Cells successfully transformed with a heterologous gene produce proteins that confer drug resistance and thus survive the selection process. Examples of such dominant selection use the drugs neomycin, mycophenolic acid and hygromycin.

Selection marker

Other examples of selectable markers suitable for mammalian cells are those that allow the identification of cellular components capable of capturing antibody nucleic acids, such as DHFR, thymidine kinase, metallothionein I and II, preferably, primate metallothionein genes, adenosine deaminase, ornithine decarboxylase, etc.

For example, cells transformed with a DHFR selection gene are first identified by culturing all of the transformants in a medium containing methotrexate (Mtx), a competitive antagonist of DHFR. Suitable host cells when using wild-type DHFR are Chinese hamster ovary (CHO) cell line deficient in DHFR activity (e.g., ATCC CRL-9096).

Alternatively, host cells transformed or co-transformed with a DNA sequence encoding an antibody, wild DHFR protein, and other selectable markers such as aminoglycoside 3'-phosphotransferase (APH) (particularly, wild-type hosts containing endogenous DHFR) can be selected by cell growth in a medium containing a selection agent for selectable markers such as kanamycin, neomycin or aminoglycosidic antibiotics such as G418. See U.S. Pat. No. 4,965,199.

(iv) Promoter component

Expression and cloning vectors usually contain a promoter that is bound by the host organism and operably linked to the antibody polypeptide nucleic acid. Eukaryotic promoter sequences are known. Virtually all eukaryotic genes have an AT-rich region found approximately 25 to 30 bases upstream from the transcription initiation site. Another sequence found 70 to 80 bases upstream from the transcription initiation site of many genes is the CNCAAT region where N is any nucleotide. At the 3'end of most eukaryotic genes is the AATAAA sequence, which is a signal for the addition of a polyA tail to the 3'end of the coding sequence. All of these sequences are suitably inserted into eukaryotic expression vectors.

Transcription of antibody polypeptides from vectors in mammalian host cells is regulated by, for example, promoters obtained from the genomes of viruses such as polyomavirus, fowlpox virus, adenovirus (*e.g.*, adenovirus 2), bovine papillomavirus, avian sarcoma virus, cytomegalovirus, retrovirus, Hepatitis B virus, and simian virus 40 (SV40); heterologous mammalian promoters such as the actin promoter or immunoglobulin promoter; and heat shock promoters, as long as such promoters are compatible with host cell systems.

The early and late promoters of the SV40 virus can be conveniently obtained as an SV40 restriction fragment further containing the origin of replication of the SV40 virus. The immediate early promoter of the human cytomegalovirus is conveniently obtained as a Hind III E restriction fragment. A system for expressing DNA in a mammalian host using bovine papillomavirus as a vector is disclosed in U.S. Pat. No. 4,419,446. A variation of this system is disclosed in U.S. Pat. No. 4,660,1978. Alternatively, the Rous sarcoma virus long-terminal repeat can be used as the promoter.

(v) Enhancer element component

Transcription of DNA encoding an antibody polypeptide of the invention by higher eukaryotes is often enhanced by inserting an enhancer sequence into the vector. Many enhancer sequences are now known from mammalian genes (globin, elastase, albumin, alpha-fetoprotein and insulin). However, typically, an enhancer derived from a eukaryotic cell virus will be used. Examples include the SV40 enhancer on the late side of the replication origin (100-270 base pairs), the cytomegalovirus early promoter enhancer, the polyoma enhancer on the late side of the replication origin, and the adenovirus enhancer. See also Yaniv, Nature, 297: 17-18 (1982) for enhancing elements for the activation of eukaryotic promoters. The enhancer can be spliced into the vector at a 5'or 3'position of the antibody polypeptide encoding sequence, but is preferably located at a 5'position from the promoter.

(vi) Transcription termination component

Furthermore, expression vectors used in eukaryotic host cells typically contain sequences required for transcription termination and mRNA stabilization. Such sequences can generally be obtained from the 5' and sometimes 3' untranslated regions of eukaryotic or viral DNA or cDNA. These regions contain nucleotide segments that are transcribed as polyadenylated fragments in the untranslated portion of the antibody-encoding mRNA. One useful transcription termination component is the bovine growth hormone polyadenylation region. See WO 94/11026 and the expression vector disclosed therein.

(vii) Host cell selection and transformation

Suitable host cells for cloning or expressing the DNA in the vectors described herein include the higher eukaryotic cells described herein, including vertebrate host cells. Proliferation of vertebrate cells in culture (tissue culture) has become a routine procedure. Examples of useful mammalian host established cell lines are SV40-transformed monkey kidney CV1 strain (COS-7, ATCC CRL1651); human embryonic kidney line (293 or 293 cells subcloned for growth in suspension culture, Graham et al., J. Gen Virol., 36:59 (1977)); baby Hamster kidney cells (BHK, ATCC CCL10); Chinese hamster ovary cells/-DHFR (CHO, Urlaub et al., Proc. Natl. Acad. Sci., USA, 77: 4216 (1980)); Mouse Sertoli cells (TM4, Mather, Biol. Reprod., 23: 243-251 (1980)); Monkey kidney cells (CV1 ATCC CCL70); African green monkey kidney cells (VERO-76, ATCC CRL-1587); human cervical carcinoma cells (HELA, ATCC CCL2); canine kidney cells (MDCK, ATCC CCL34); buffalo rat liver cells (BRL3A, ATCC CRL1442); human lung cells (W138, ATCC CCL75); human hepatocytes (Hep G2, HB8065); mouse mammary tumor cells (MMT0605622, ATCC CCL51); TRI cells (Mather et al., Annals N.Y. Acad. Sci., 383: 44-68) 1982)); MRC5 cells; FS4 cells; and human hepatoma line (HepG2). Moreover, the example of the host cell is not limited to animal cell lines. Of course, it is also possible to use various cells such as effector cells extracted from a living body and mesenchymal stem cells (MSC) as host cells.

Host cells are transformed with the expression or cloning vectors described above for antibody production, and cultured in the conventional nutrient medium appropriately modified to induce promoters, select transformants, or amplify genes encoding the desired sequence..

(viii) Host cell culture

The host cells used to produce the antibodies of the present disclosure can be cultured in various media. Examples of commercially available media suitable for host cell culture are Ham's F10 (Sigma), Minimal Essential Medium ((MEM), (Sigma), RPMI-1640 (Sigma), and Dulbecco's Modified Eagle's medium ((DMEM), (Sigma). Also, any of the media described in Ham et al., Meth. Enz. 58:44 (1979), Barnes et al., Anal. Biochem. 102: 255 (1980), U.S. Pat. No. 4767704; 4657866; 4927762; 4560655; or 5122469; WO 90/03430; WO 87/00195; or US Reissue Patent No. 30985 can be used as a culture medium for host cells. All of these media can be supplemented with hormones and/or other growth factors (*e.g*., insulin, transferrin, or epidermal growth factors), salts (*e.g*., sodium chloride, calcium, magnesium and phosphate), buffers *(e.g.,* HEPES), nucleotides *(e.g.,* adenosine and thymidine), antibiotics (*e.g*., GENTAMYCIN™ drug), trace elements (defined as inorganic compounds whose final concentration is usually present in the micromolar range), and glucose or equivalent energy sources as needed. Any other required supplement can also be included in suitable concentrations known to those skilled in the art. Culture conditions, such as temperature, pH, etc., are those that have been used in the past for the host cells selected for expression and will be apparent to those skilled in the art.

(ix) Antibody purification

When using recombinant techniques, the antibody is produced intracellularly or secreted directly into the medium. When the antibody is generated intracellularly, the first step is to remove the particulate debris by, for example, centrifugation or ultrafiltration, whether it is host cells or lysed fragments. If the antibody is secreted into the medium, the supernatant from such an expression system is generally first concentrated using a commercially available protein concentration filter, such as an Amicon or Pellicon ultrafiltration device. Protease inhibitors such as PMSF may be included in any of the above steps to inhibit proteolysis, or antibiotics may be included to prevent the growth of exogenous contaminants.

The antibody composition prepared from cells can be purified by using, for example, hydroxyapatite chromatography, gel electrophoresis, dialysis, and affinity chromatography, with affinity chromatography being the preferred purification technique. The suitability of protein A as an affinity ligand depends on the species and isotype of the immunoglobulin Fc region present in the antibody. Protein A can be used to purify antibodies based on human $\gamma 1$, $\gamma 2$, or $\gamma 4$ heavy chain (Lindmark et al., J. Immunol Meth. 62: 1-13 (1983)). Protein G is recommended for all mouse isotypes and human $\gamma 3$ (Guss et al., EMBO J. 5: 165715575 (1986)). The matrix to which the affinity ligand is attached is most often agarose, but other materials can also be used. Mechanically stable matrices such as controlled pore glass and poly (styrene divinyl) benzene allow for faster flow rates and shorter treatment times than those which can be achieved with agarose. If the antibody contains a CH3 domain, Bakerbond ABx™ resin (J.T. Baker, Phillipsburg, NJ) (polyaspartic acid column), chromatofocusing, SDS-PAGE, and ammonium sulfate precipitation methods can also be used according to the recovered multivalent antibody.

Following the preliminary purification step, the mixture containing the antibody of interest and contaminants is subjected to low pH hydrophobic interaction chromatography using an elution buffer with a pH of about 2.5-4.5, preferably at a low salt concentration (e.g., about 0-0.25 M salt).

Immunoconjugate

An "immunoconjugate" is an antibody conjugated to one or more heterologous molecules (heterologous molecules include, but are not limited to, cytotoxic agents). The present disclosure provides immunoconjugates containing any of the anti-FGFR3 antibodies described herein that are conjugated to cytotoxic agents, for example, agents such as chemotherapeutic agents, cell injury agents, growth inhibitors (including "antibody-drug conjugates" or "ADCs"), toxins (*e.g.*, bacteria, filamentous fungi, plant or animal-derived enzymatically-active toxins, or fragments thereof), or radioisotopes *(i.e.,* radioconjugates)

The term "cytotoxic agent" as used herein refers to a substance that inhibits or impedes cell function and/or causes cell death or destruction. Cell injury agents are, without limitation, radioisotopes (*e.g.*, $^{211}At$, $^{131}I$, $^{125}I$, $^{90}Y$, $^{186}Re$, $^{188}Re$, $^{153}Sm$, $^{212}Bi$, $^{32}P$, $^{212}Pb$ and Lu radioisotopes); chemotherapeutic agents or chemotherapeutic drugs (*e.g.* methotrexate, adriamycin, vinca alkaloids (vincristine, vinblastine, etoposide), doxorubicin, melphalan, mitomycin C, chlorambucil, daunorubicin, or other intercalating agents)); growth inhibitors; enzymes such as nucleic acid degradation enzymes and fragments thereof; antibiotics; toxins such as small molecule toxins or enzymatically-active toxins of bacterial, fungal, plant, or animal origin (including fragments and/or variants thereof); and various antitumor or anticancer agents disclosed below.

The use of antibody-drug conjugates for the local delivery of cytotoxic or cytostatic agents, *i.e.,* drugs for killing or inhibiting tumor cells in cancer treatment (Syrigos and Epenethos (1999) Anticancer Research 19: 605- 614; Niculescu-Duvaz and Springer (1997) Adv. Drg Del. Rev. 26: 151-172; U.S. Pat. No. 4,975,278), enables targeted transport of drug components to tumors and intracellular accumulation therein, whereas systemic administration of unconjugated agents with drug effects can result in unacceptable levels of toxicity to normal cells as well as tumor cells sought to be removed (Baldwin et al., (1986) Lancet pp. (Mar. 15, 1986): 603-05; Thorpe, (1985) "Antibody Carriers Of Cytotoxic Agents In Cancer Therapy: A Review", in Monoclonal Antibodies ’84: Biological And Clinical Applications, A. Pinchera et al. (ed.s), pp. 475-506). Accordingly, maximum efficacy with minimal toxicity is desired. Polyclonal and monoclonal antibodies have been reported to be useful in this strategy (Rowland et al. (1986) Cancer Immunol. Immunother., 21: 183-87). Drugs used in this method include daunomycin, doxorubicin, methotrexate and vindesine (Rowland et al., (1986), supra). Toxins used for the antibody-drug conjugates include bacterial toxins such as diphtheria toxin, plant toxins such as ricin, small molecule toxins, such as geldanamycin (Mandler et al. (2000) Jour. of the Nat. Cancer Inst. 92 (19): 1573-1581; Mandler et al. (2000). Bioorganic & Med. Chem. Letters 10: 1025-1028; Mandler et al. (2002) Bioconjugate Chem. 13: 786-791), maytansinoids (EP 1391213; Liu et al., (1996) Proc. Natl. Acad. Sci. USA 93: 8618-8623) and calicheamicin (Lode et al., (1998) Cancer Res. 58: 2928; Hinman et al., (1993) Cancer Res. 53: 3336-3342). The toxins can affect cytotoxic and cytostatic effects by mechanisms including tubulin binding, DNA binding or topoisomerase inhibition. Certain cytotoxic agents tend to get inactivated or have reduced activity when conjugated to large antibodies or protein receptor ligands.

Zevalin® (ibritumomab tiuxetan, Biogen/Idec) is an antibody-radioisotope conjugate where a murine IgG1κ monoclonal antibody against the CD20 antigen found on the cell surface of normal and malignant B lymphocytes is bound to an 111In or 90Y radioisotope with a thiourea linker-chelating agent (Wiseman et al., (2000) Eur. Jour. Nucl. Med. 27 (7): 766-77; Wiseman et al., (2002) Blood 99 (12): 4336-42; Witzig et al., (2002) J. Clin. Oncol. 20 (10): 2453-63; Witzig et al., (2002) J. Clin. Oncol. 20 (15): 3262-69). Zevalin is active against B-cell non-Hodgkin’s lymphocytes (NHL), but administration causes severe and long-term hematopenia in most patients. Mylotarg® (gemtuzumab ozogamicin; Wyeth Pharmaceuticals), which is an antibody-drug conjugate consisting of huCD33 antibody linked to calicheamicin, was approved in 2000 as an injection for the treatment of acute myeloid leukemia (Drugs of the Future (2000) 25 (7): 686; U.S. Pat. No. 4,970,198; No. 5079233; No. 5585089; No. 5606040; No. 5693762; No. 5739116; No. 5767285; No. 5773001). Cantuzumab mertansine (Immunogen, Inc.), which is an antibody-drug conjugate consisting of a huC242 antibody linked to the maytansinoid drug molecule DM1 via a disulfide linker SPP, is undergoing phase II trials for the treatment of CanAg-expressing cancers, such as cancers of the large intestine, pancreas, and stomach. MLN-2704 (Millennium Pharm., BZL Biologics, Immunogen Inc.), which is an antibody-drug conjugate consisting of an anti-prostate-specific membrane antigen (PSMA) monoclonal antibody linked to the maytansinoid drug molecule DM1, is in the development stage of potential treatment for prostate cancer. The synthetic analogs of dolastatin, the auristatin peptides, auristatin E (AE) and monomethyl auristatin (MMAE) are conjugated to chimeric monoclonal antibodies cBR96 (specific for Louis Y on cancer cells) and cAC10 (specific for CD30 on hematological malignancies) (Doronina et al., (2003) Nature Biotechnology 21 (7): 778-784), and are in the therapeutic development stage.

Chemotherapeutic agents useful for the formation of immune complexes (immunoconjugates) are described herein (*e.g.*, above). Enzymatically-active toxins and fragments thereof that can be used include diphtheria A chain, nonbinding active fragments of diphtheria toxin, exotoxin A chain (from *Pseudomonas aeruginosa*), ricin A chain, abrin A chain, modeccin A chain, alpha-sarcin, *Aleurites fordii* protein, dianthin protein, *Phytolacca americana* protein (PAPI, PAPII, and PAP-S), *Momordica charantia* inhibitor, curcin, crotin, *Saponaria officinalis* inhibitor, gelonin, mitogellin, restrictocin, phenomycin, enomycin, and trichothecenes. See WO 93/21232 published on October 28, 1993, for example. A variety of radionucleotides are available for the production of radioconjugated antibodies. Examples include 212Bi, 1311, 131In, 90Y and 186Re. Conjugates of antibody and cytotoxic drug are made using various bifunctional protein coupling agents, such as N-succinimidyl-3-(2-pyridyldithiol) propionate (SPDP), and iminothiolane (IT), bifunctional derivatives of imidoesters (dimethyl adipimidate HCL, and such), active esters (disuccinimidyl suberate, and such), aldehydes (glutaraldehyde, and such), bis-azido compounds (bis(p-azidobenzoyl)hexanediamine, etc.), bis-diazonium derivatives (bis-(p-diazonium benzoyl)-ethylenediamine, and such), diisocyanates (triene 2,6-diisocyanate, and such), and bis-active fluorine compounds (1,5-difluoro-2,4-dinitrobenzene, and such). For example, the ricin immunotoxin can be prepared as described in Vitetta et al., Science 238: 1098 (1987). Carbon-14-labeled 1-isothiocyanatobenzyl-3-methyldiethylenetriaminepentaacetic acid (MX-DTPA) is an example of a chelating agent for conjugation of a radionucleotide to an antibody. See WO 94/11026.

Antibody conjugates and one or more small molecule toxins such as calicheamicins, maytansinoids, dolastatins, auristatins, trichothenes and CC1065, and their derivatives with toxic activity are discussed herein.

i. Maytansine and maytansinoids

In some embodiments, the immunoconjugate comprises an antibody (full length or fragment) of the present disclosure that is bound to one or more maytansinoid molecules. Maytansinoids are mitotic inhibitors that act to inhibit tubulin polymerization. Maytansine was first isolated from East African shrub *Maytenus serrata* (U.S. Pat. No. 3,896111). It was subsequently discovered that certain microorganisms produce maytansinoids, such as maytansinol and C-3 maytansinol esters (U.S. Pat. No. 4,151,042). Synthetic maytansinol and its derivatives and analogs are described, for example, in U.S. Pat. Nos. 4,137,230; 4,248,870; 4,256,746; 4,260,608; 4,265,814; 4,294,757; 4,307,016; 4,308,268; 4,308,269; 4,309,428; 4,313, 946; 4,315,929; 4,317,821; 4,322,348; 4,331,598; 4,361,650; 4,364,866, No. 4,424,219; No. 4,450,254; No. 4,362,663; and No. 4,371,533.

Maytansinoid drug components are attractive drug components of antibody-drug conjugates since they (i) are relatively available for preparation by fermentation or chemical modification, derivatization of fermented products, (ii) comply with derivatization with functional groups suitable for conjugation through a non-disulfide linker to antibodies, (iii) are stable in plasma, and (iv) are effective against various tumor cell lines.

Immunoconjugates containing maytansinoids, their preparation methods and their therapeutic uses are disclosed, for example, in U.S. Pat. Nos. 5208020 and 5416064, and European Patent No. 0425235 B1, the disclosures of which are incorporated herein by specifying the source. Liu et al., Proc. Natl. Acad. Sci., USA 93: 8618-8623 (1996) describes immunoconjugates containing a maytansinoid named DM1, which is linked to the monoclonal antibody C242 against human colorectal cancer. The conjugate has been found to be highly cytotoxic to cultured colon cancer cells and exhibits antitumor activity in *in vivo* tumor growth assays. Chari et al., Cancer Research, 52: 127-131 (1992) describes an immunoconjugate where a maytansinoid is conjugated via a disulfide bond to the murine antibody A7 that binds to an antigen of human colon cancer lineage cells, or to another murine monoclonal antibody TA.1 that binds to HER-2/neu oncogene. The cytotoxicity of the TA.1-maytansinoid conjugate was tested *in vitro* in human breast cancer cell line SK-BR-3, which expresses $3\times10^5$ HER-2 surface antigens per cell. The drug conjugate achieves a degree of cytotoxicity similar to that of a free maytansinoid agent, which is increased by increasing the number of maytansinoid molecules per antibody molecule. The A7-maytansinoid conjugate showed low systemic cytotoxicity in mice.

Antibody-maytansinoid conjugates are prepared by chemically linking an antibody to a maytansinoid molecule with little reduction in the biological activity of either the antibody or the maytansinoid molecule. See, for example, U.S. Pat. No. 5,208,020 (specially incorporated herein by specifying the source). One molecule of toxin/antibody is expected to increase cytotoxicity than when using naked antibodies, but those to which an average of 3-4 maytansinoid molecules are bound per antibody molecule have the effect of improving cytotoxicity to target cells without adversely affecting antibody function or solubility. Maytansinoids are well known in the art and can be synthesized by known techniques or isolated from natural sources. Suitable Maytansinoids are disclosed, for example, in U.S. Pat. No. 5,208,020, and other patents, as well as non-patent publications mentioned above. Preferred maytansinoids are maytansinol and maytansinol analogs such as various maytansinol esters, in which the aromatic ring or other position of the maytansinol molecule is modified.

There are many linking groups known in the art for making antibody-maytansinoid conjugates, including those disclosed in, for example, U.S. Pat. No. 5208020 or European Patent No. 0425235 B1, Chari et al., Cancer Research, 52: 127-131 (1992), and U.S. Patent Application No. 10/960602, filed October 8, 2004 (these disclosures are specially incorporated herein by specifying the source). An antibody-maytansinoid conjugate comprising the linker component SMCC can be prepared as disclosed in U.S. Patent Application No. 10/960602 filed October 8, 2004. Linking groups include disulfide groups, thioether groups, acid-labile groups, light-labile groups, peptidase-labile groups, or esterase-labile groups as disclosed in the patents described above, but disulfide and thioether groups are preferable. Further linking groups are described and exemplified herein.

Antibody and cytotoxic drug conjugates can be formed using various bifunctional protein coupling agents, for example, N-succinimidyl-3-(2-pyridyldithio)propionate (SPDP), succinimidyl-4-(N-maleimidomethyl)cyclohexane-1-carboxylate (SMCC), iminothiolane (IT), bifunctional derivatives of imidoesters *(e.g.,* dimethyl adipimidate HCL), active esters (*e.g*., disuccinimidyl suberate), aldehydes *(e.g.,* glutaraldehyde), bis-azide compounds *(e.g.,* bis (p-azidobenzoyl)hexanediamine), bis-diazonium derivatives (e.g., bis-(p-diazoniumbenzoyl) ethylenediamine), diisocyanates (e.g., toluene-2,6-diisocyanate), and bis-active fluorine compounds (e.g., 1,5-difluoro-2,4-dinitrobenzene). Particularly preferred coupling agents include N-succinimidyl-4-(2-pyridylthio)pentanoate (SPP) and N-succinimidyl-3-(2-pyridyldithio)propionate (SPDP) (Carlsson et al. Biochem. J. 173: 723-737 [1978]) to provide disulfide linkage.

The linker can be attached to the maytansinoid molecule at various positions depending on the type of link. For example, an ester linkage can be formed by reacting with a hydroxyl group using a conventional coupling technique. The reaction occurs at the C-3 position with a hydroxyl group, the C-14 position modified with hydroxymethyl, the C-15 position modified with a hydroxyl group, and the C-20 position with a hydroxyl group. In a preferred embodiment, the linkage is formed at the C-3 position of maytansinol or an analog of maytansinol.

ii. Auristatins and dolastatins

In some embodiments, the immunoconjugate comprises an antibody of the present disclosure conjugated to dolastatin or the dolastatin peptidyl analog and derivatives, or auristatins (U.S. Pat. No. 5,635,483; 5,780,588). Dolastatins and auristatins have been shown to interfere with microtubule kinetics, GTP hydrolysis, and nuclei and cell division (Woyke et al. (2001) Antimicrob. Agents and Chemother. 45 (12): 3580-3584), and shown to have anticancer activity (U.S. Pat. No.5663149) and antifungal activity (Pettit et al. (1998) Antimicrob. Agents Chemother. 42: 2961-2965). The dolastatin or auristatin drug component can attach to the antibody by the N (amino)-terminus or C (carboxyl) terminus of the peptidyl drug moiety (WO 02/088772).

An exemplary auristatin embodiment comprises the N-terminus linked monomethyl auristatin drug moieties DE and DF, and is disclosed in US Continuation Patent No. 10/983340, filed November 5, 2004, "Monomethylvaline Compounds Capable of Conjugation to Ligands". The entire content of this disclosure is specially incorporated herein by specifying the source.

In general, peptide-based drug components can be prepared by forming peptide bonds between two or more amino acids and/or peptide fragments. Such peptide bonds can be prepared, for example, according to a liquid phase synthesis method well known in the field of peptide chemistry (See E. Schroder and K. Lubke, "The Peptides", volume 1, pp 76-136, 1965, Academic Press). The auristatin/dolastatin drug component can be prepared according to a method described in U.S. Pat. No. 5635843; No. 5780588; Pettit et al. (1989) J. Am. Chem. Soc. 111: 5463-5465; Pettit et al. (1998) Anti-Cancer Drug Design 13: 243-277; Pettit, G. R., et al. Synthesis, 1996, 719-725; and Pettit et al. (1996) J. Chem. Soc. Perkin Trans. 1 5: 859-863. Also see Doronina (2003) Nat Biotechnol 21 (7): 778-784; "Monomethylvaline Compounds Capable of Conjugation to Ligands", US Continuation Patent Application 10/983340, filed on November 5, 2004. These are incorporated herein in their entirety by specifying the source *(e.g.,* they disclose how to prepare linkers and monomethylvaline compounds such as MMAE and MMAF conjugated to a linker).

iii. Calicheamicins

In another embodiment, the immunoconjugate comprises an antibody of the present disclosure conjugated to one or more calicheamicin molecules. The calicheamicin family of antibiotics can cause double-stranded DNA breaks at sub-picomolar concentrations. Regarding the preparation of conjugates of the calicheamicin family, see U.S. Pat. Nos. 5712374, 5714586, 5739116, 5767285, 5770701, 5770710, 5773001, and 5877296 (all American Cyanamid Company). Structural analogs of calicheamicins that can be used include, but are not limited to, $\gamma$1I, $\alpha$2I, $\alpha$3I, N-acetyl-$\gamma$1I, PSAG and $\theta$I1 (Hinman et al., Cancer Research, 53: 3336-3342 (1993), Lode et al., Cancer Research, 58: 2925-2928 (1998) and the above-mentioned U.S. patents of American Cyanamid Company). Another anti-tumor agent to which the antibody can be conjugated is the antifolate QFA. Both calicheamicins and QFA have intracellular sites of action and do not easily cross the plasma membrane. Therefore, the uptake of these drugs into cells by antibody-mediated internalization greatly improves their cytotoxic effects.

iv. Other cytotoxic agents

Other anti-tumor agents that can be conjugated to the antibodies of the present disclosure include BCNU, streptozocin, vincristine and 5-fluorouracil, the family of agents collectively known as the LL-E33288 complex described in U.S. Pat. Nos. 5053394, 5770710, as well as esperamicine (U.S. Pat. No. 5877296).

Enzymatically-active toxins and fragments thereof that can be used include diphtheria A chain, nonbinding active fragments of diphtheria toxin, exotoxin A chain (from *Pseudomonas aeruginosa),* ricin A chain, abrin A chain, modeccin A chain, alpha-sarcin, *Aleurites fordii* protein, dianthin protein, *Phytolacca americana* protein (PAPI, PAPII, and PAP-S), *Momordica charantia* inhibitor, curcin, crotin, *Saponaria officinalis* inhibitor, gelonin, mitogellin, restrictocin, phenomycin, enomycin, and trichothecenes. See, for example, WO 93/21232, published October 28, 1993.

The present disclosure further discusses immunoconjugates formed between antibodies and compounds with nucleolytic activity (e.g., ribonucleases or DNA endonucleases, such as deoxyribonuclease; DNase).

Antibodies may contain highly radioactive atoms in order to selectively destroy the tumor. Various radioisotopes are utilized to produce radioconjugated antibodies. Examples include At211, 1131, 1125, Y90, Re186, Re188, Sm153, Bi212, P32, Pb212 and radioactive isotopes of Lu. When the conjugate is used for detection, it may contain a radioactive atom for scintigraphic studies, such as technesium-99m or iodine-123, or a spin label for nuclear magnetic resonance (NMR) imaging (also known as magnetic resonance imaging, MRI), for example, iodine-123, iodine-131, indium-111, fluorine-19, carbon-13, nitrogen-15, oxygen-17, gadolinium, manganese or iron.

Radio- or another label is introduced into the conjugate by a known method. For example, peptides are either biosynthesized or synthesized by chemical amino acid synthesis using suitable amino acid precursors containing fluorine-19 instead of hydrogen. Labels such as technetium-99m or iodine-123, rhenium-186, rhenium-188 and indium-111 can be attached via the cysteine residue of the peptide. Yttrium-90 can bind via a lysine residue. The IODOGEN method (Fraker et al. (1978) Biochem. Biophys. Res. Commun. 80: 49-57) can be used to incorporate iodine-123. Details of other methods are described in "Monoclonal Antibodies in Immunoscintigraphy" (Chatal, CRC Press 1989).

Conjugates of antibodies and cytotoxic agents maybe made using various bifunctional protein coupling agents such as N-succinimidyl-3-(2-pyridyldithio) propionate (SPDP), succinimidyl-4-(N-maleimidomethyl)cyclohexane-1-carboxylate (SMCC), iminothiolane (IT), bifunctional derivatives of imidoesters (dimethyl adipimidate HCL, and such), active esters (disuccinimidyl suberate, and such), aldehydes (glutaraldehyde, and such), bis-azido compounds (bis(p-azidobenzoyl)hexanediamine, etc.), bis-diazonium derivatives (bis-(p-diazonium benzoyl)ethylenediamine, and such), diisocyanates (triene-2,6-diisocyanate, and such), and bis-active fluorine compounds (1,5-difluoro-2,4-dinitrobenzene, and such). For example, the ricin immunotoxin can be prepared as described in Vitetta et al., Science 238: 1098 (1987). Carbon-14-labeled 1-isothiocyanatobenzyl-3-methyldiethylene-triaminepentaacetic acid (MX-DTPA) is an example of a chelating agent for conjugating radionucleotides to an antibody. See WO 94/11026. The linker may be a "cleavable linker" to facilitate the release of the cytotoxic agent in the cell. For example, acid-labile linkers, peptidase-labile linkers, photolabile linkers, dimethyl linkers or disulfide-containing linkers can be used (Chari et al., Cancer Research, 52: 127-131 (1992); U.S. Pat. No. 5208020).

Particularly conceivable compounds of the present disclosure are, without restriction, ADCs prepared with cross-linking agents BMPS, EMCS, GMBS, HBVS, LC-SMCC, MBS, MPBH, SBAP, SIA, SIAB, SMCC, SMPB, SMPH, Sulfo-EMCS, Sulfo-GMBS, Sulfo-KMUS, Sulfo-MBS, Sulfo-SIAB, Sulfo-SMCC, and Sulfo-SMPB, and SVSB (succinimidyl-(4-vinylsulfone)benzoate) that are commercially available (e.g., from Pierce Biotechnology, Inc., Rockford, IL., USA). See pages 467-498 of the 2003-2004 Applications Handbook and Catalog.

v. Preparation of antibody-drug conjugates

In the antibody-drug conjugates (ADCs) of the present disclosure, an antibody (Ab) is conjugated to one or more drug moieties (D), e.g., about 1 to about 20 drug moieties per antibody, via a linker (L). The ADC of formula I can be prepared using several means using organic chemical reactions, conditions and reagents known to those skilled in the art, including:
(1) reaction of a nucleophilic group of an antibody with a bivalent linker reagent to form Ab-L via a covalent bond, followed by reaction with a drug moiety D; and (2) reaction of a nucleophilic group of a drug moiety with a bivalent linker reagent to form D-L via a covalent bond, followed by reaction with the nucleophilic group of an antibody. Further methods for preparing ADCs are described herein.

Ab-(L-D)p I

The linker may consist of one or more linker components. Exemplary linker components are 6-maleimide caproyl ("MC"), maleimidopropanoyl ("MP"), valine-citrulline ("val-cit"), alanine-phenylalanine ("ala-phe"), p-amino benzyloxy carbonyl ("PAB"), N-succinimidyl 4-(2-pyridylthio)-pentanoate ("SPP"), N-succinimidyl 4-(N-maleimidomethyl) cyclohexane-1-carboxylate ("SMCC"), and N-succinimidyl (4-iodo-acetyl)aminobenzoate ("SIAB"). Additional linker components are known in the art, some of which are described herein. See also "Monomethylvaline Compounds Capable of Conjugation to Ligands", US Application No. 10/983340 filed on November 5, 2004. The contents of which are incorporated herein by specifying the source.

In some embodiments, the linker may contain amino acid residues. Exemplary amino acid linker components include dipeptides, tripeptides, tetrapeptides or pentapeptides. Exemplary dipeptides include valine-citrulline (vc or val-cit), alanine-phenylalanine (af or ala-phe). Exemplary tripeptides include glycine-valine-citrulline (gly-val-cit) and glycine-glycine-glycine (gly-gly-gly). Amino acid residues which comprise an amino acid linker component include those occurring naturally, as well as minor amino acids and non-naturally occurring amino acid analogs, such as citrulline. The amino acid linker component can be designed and can specifically optimized for the selectivity of enzymatic cleavage by enzymes such as tumor-associated proteases, cathepsin B, C and D or plasmin proteases.

The nucleophilic groups on the antibody include, but are not limited to: (i) N-terminal amine groups, (ii) side chain amine groups such as lysine, (iii) side chain thiol groups such as cysteine, and (iv) sugar hydroxyl groups or amino groups by which the antibody is glycosylated. Amines, thiols and hydroxyl groups are nucleophilic and capable of reacting to form covalent bonds with electrophilic groups on linker moieties and linker reagents including: (i) active esters such as NHS esters, HOBt esters, haloformates and acid halides; (ii) alkyl and benzyl halides, such as haloacetamides; and (iii) aldehydes, ketones, carboxyls and maleimides. Certain antibodies have reducible interchain disulfides, *i.e.,* cysteine crosslinks. Antibodies may be conjugated with a linker reagent by treatment with a reducing agent such as DTT (dithiothreitol). Therefore, each cysteine crosslink theoretically forms two reactive thiol nucleophilic groups. Additional nucleophilic groups can be introduced into the antibody by reaction of lysines with 2-iminothiolane (Traut's reagent) that converts amines to thiols. Reactive thiol groups may be introduced into the antibody by introducing 1, 2, 3, 4 or more cysteine residues (*e.g.*, to prepare mutant antibodies comprising one or more non-natural cysteine amino acid residues).

Further, the antibody-drug conjugate of the present disclosure may be produced by modifying the antibody to introduce an electrophilic moiety (which can react with nucleophilic substituents on the linker reagent or drug). The glycosylated antibody sugars may be oxidized, for example, with a periodate oxidizing agent to form an aldehyde or ketone group that reacts with the amine group of the linker reagent or drug moiety. The resulting imine Schiff base may form a stable link, or may be reduced, for example, with a borohydride reagent to form a stable amine link. In one embodiment, the reaction of the carbohydrate portion of the glycosylated antibody with either galactose oxidase or sodium metaperiodate can result in a protein carbonyl (aldehyde and ketone) group that can react with a suitable group on the drug (Hermanson, Bioconjugate Techniques). In another embodiment, a protein containing the N-terminal serine or threonine residue reacts with sodium metaperiodate to produce an aldehyde instead of the first amino acid (Geoghegan & Stroh, (1992) Bioconjugate Chem. 3:138-146; U.S. Pat. No. 5362852). Such aldehydes can react with drug moieties or linker nucleophilic groups.

Similarly, nucleophilic groups on a drug moiety include, but are not limited to, the following: amines, thiols, hydroxyls, hydrazides, oximes, hydrazines, thiosemicarbazones, hydrazine carboxylate and arylhydrazine groups that can react and covalently bind to electrophilic groups on linker moieties and linker reagents including: (i) active esters (e.g. NHS esters, HOBt esters, haloformates and acid halides); (ii) alkyl and benzyl halides, such as haloacetamides; and (iii) aldehydes, ketones, carboxyl and maleimide groups.

Alternatively, a fusion protein containing an antibody and a cytotoxic agent is produced, for example, by recombinant techniques or peptide synthesis. The length of the DNA comprises each of the regions encoding the two parts of the conjugate that are either adjacent to each other, or are separated by a region encoding a linker peptide that does not destroy the desired properties of the conjugate.

In another embodiment, the antibody is conjugated to a "receptor" (*e.g.*, streptavidin) for use in tumor pretargeting, where the antibody-receptor conjugate is administered to the individual, followed by the use of a clearing agent to remove unbound conjugates from the circulation, and administration of a "ligand" (*e.g.*, avidin) that conjugates to a cytotoxic agent *(e.g.,* a radionucleotide).

Method of using an anti-FGFR3 S249C mutant antibody

The present disclosure features the use of an anti-FGFR3 S249C mutant antibody as part of a particular therapeutic regimen intended to provide a beneficial effect from the activity of a therapeutic agent. The present disclosure is particularly useful in the treatment of different types of cancer at different stages.

The tumor can be a solid tumor, a non-solid tumor, or a soft tissue tumor. Examples of soft tissue tumors include leukemias (*e.g.*, chronic myeloid leukemia, acute myeloid leukemia, adult acute lymphocytic leukemia, acute myeloid leukemia, mature B-cell acute lymphocytic leukemia, chronic lymphocytic leukemia, pre-lymphocytic leukemia, or hairy cell leukemia), or lymphoma (e.g., non-Hodgkin's lymphoma, cutaneous T cell lymphoma, or Hodgkin's disease). Solid tumors include any cancer of a body tissue other than blood, bone marrow, or lymphatic system. Solid tumors are further divided into those originating from epithelial cells and those originating from non-epithelial cells. Examples of epithelial cell solid tumors include tumors of the gastrointestinal tract, large intestine, breast, prostate, lung, kidney, liver, pancreas, ovary, head and neck, oral cavity, gastric, duodenum, small intestine, large intestine, anus, gallbladder, labia majora, the nasopharynx, skin, uterus, male reproductive organs, prostate, urinary tract, bladder, and skin. Solid tumors of non-epithelial origin include sarcomas, brain tumors, and bone tumors. Other examples of tumors are described in the definition section.

In another embodiment of the present disclosure, provided is a method of treating cancer, which comprises the step of administering a multispecific antigen-binding molecule of the present disclosure. In the therapeutic method of the present disclosure, it is desirable to confirm in advance that the cancer to be treated expresses an FGFR3 S249C mutant before administering the multispecific antigen-binding molecule. That is, the present disclosure relates to a method of treating cancer, which comprises the step of selecting (or identifying) a subject having a cancer expressing an FGFR3 S249C mutant, and administering a multispecific antigen binding molecule of the present disclosure to the selected subject (or the identified subject). Further, in another embodiment, the present disclosure relates to a multispecific antigen-binding molecule of the present disclosure for administration to a subject having a cancer expressing an FGFR3 S249C mutant.

Alternatively, the present disclosure relates to the use of a multispecific antigen-binding molecule of the present disclosure in the treatment of cancer expressing an FGFR3 S249C mutant. Furthermore, the present disclosure relates to the use of an multispecific antigen-binding molecule of the present disclosure in the manufacture of a therapeutic pharmaceutical composition for a cancer expressing an FGFR3 S249C mutant. The present disclosure also relates to a method for producing a therapeutic pharmaceutical composition for a cancer expressing an FGFR3 S249C mutant, which comprises the step of formulating the multispecific antigen-binding molecule of the present disclosure with a pharmaceutically-acceptable carrier.

In one embodiment of the present disclosure, the following amino acid sequence can be exemplified as CDRs for constituting the variable region domains of the FGFR3 S249C mutant-binding arms constituting the multispecific antigen-binding molecules:

| | | | |
|---|---|---|---|
| FGA0002 | | SEQ ID NO: 5 | SEQ ID NO: 6 |
| | CDR1 | SEQ ID NO: 17 | SEQ ID NO: 20 |
| | CDR2 | SEQ ID NO: 18 | SEQ ID NO: 21 |
| | CDR3 | SEQ ID NO: 19 | SEQ ID NO: 22 |
| FGA0005 | | SEQ ID NO: 7 | SEQ ID NO: 8 and |
| | CDR1 | SEQ ID NO: 23 | SEQ ID NO: 26 |
| | CDR2 | SEQ ID NO: 24 | SEQ ID NO: 27 |
| | CDR3 | SEQ ID NO: 25 | SEQ ID NO: 28 |
| FGA0007 | | SEQ ID NO: 9 | SEQ ID NO: 10 |
| | CDR1 | SEQ ID NO: 29 | SEQ ID NO: 32 |
| | CDR2 | SEQ ID NO: 30 | SEQ ID NO: 33 |
| | CDR3 | SEQ ID NO: 31 | SEQ ID NO: 34 |

On the other hand, in one embodiment of the present disclosure, the following amino acid sequences can be exemplified as CDR amino acids for constituting the antigen-binding domain of the CD3 binding arm that constitutes the multispecific antigen-binding molecule:

| | VH | VL |
|---|---|---|
| CDR1 | SEQ ID NO: 59 | SEQ ID NO: 62 |
| CDR2 | SEQ ID NO: 60 | SEQ ID NO: 63 |
| CDR3 | SEQ ID NO: 61 | SEQ ID NO: 64 |

Diagnostic tests before and/or during and after treatment

In some embodiments, diagnostic tests are performed on the patients of the present disclosure, *e.g.*, before and/or during and after treatment. That is, a diagnostic test of the subject of the present disclosure can be performed at any of, or a combination of, the timings selected from before, during, and after treatment. Generally, when a diagnostic test is performed, a sample can be taken from a patient in need of treatment. If the subject has cancer, the sample for diagnosing it can usually be a tumor sample, or other biological sample. Specifically, it is, for example, a biological fluid containing, but not limited to, blood, urine, saliva, ascites, or derivatives such as serum and plasma.

In the present specification, the biological sample is a fixed sample, for example, a formalin-fixed, paraffin-embedded (FFPE) sample, or a frozen sample.

Various methods for determining mRNA or protein expression include, but are not limited to, gene expression analysis by gene expression profiling, polymerase chain reaction (PCR) including quantitative real-time PCR (qRT-PC), microarray analysis, SAGE method, MassARRAY, massively parallel signature sequencing (MPSS), and proteomics and immunohistochemistry (IHC), etc. Preferably, the mRNA is quantified. Such mRNA analyses are preferably performed using the polymerase chain reaction (PCR) technique or by microarray analysis. When PCR is used, the preferred form of PCR is quantitative real-time PCR (qRT-PCR). In one embodiment, expression of one or more of the genes is considered a positive expression if it is greater than or equal to the median, for example, compared to other samples of the same tumor type. The median expression level can basically be determined at the same time as the measurement of gene expression or can be determined in advance.

The steps of a typical protocol for gene expression profiling using fixed paraffin-embedded tissue as the RNA source include mRNA isolation, purification, primer extension, and amplification, and are described in articles in various publications (for example, Godfrey et al. J. Molec. Diagnostics 2: 84-91 (2000); Specht et al., Am. J. Pathol. 158: 419-29 (2001)). Briefly, the typical process begins with cutting a sample of paraffin-embedded tumor tissue at a thickness of about 10 micrograms. RNA is then extracted, and protein and DNA are removed. After analysis of the RNA concentration, RNA repair and/or amplification steps may be included, if necessary, and RNA is reverse transcribed using gene specific promoters followed by PCR. Finally, the data is analyzed to identify the best treatment options available to the patient, based on the characteristic gene expression identified in the tumor sample tested.

Detection of gene or protein expression can be performed directly or indirectly.

The expression or translocation or amplification of the FGFR3 S249C mutant in cancer can be determined (directly or indirectly). Various diagnostic/prognostic assays are available for this purpose. In one embodiment, FGFR3 S249C mutant overexpression can be analyzed by IHC. An IHC assay may be performed on paraffin-embedded tissue sections from a tumor biopsy to meet the following FGFR3 S249C mutant protein staining intensity criteria:

Score 0: No staining is observed, or membrane staining is observed in less than 10% of tumor cells.
Score 1+: a faint/barely perceptible membrane staining is detected in more than 10% of tumor cells. Only part of the cell membrane is stained.
Score 2+: a weak to moderate complete membrane staining is observed in more than 10% of tumor cells.
Score 3+: a moderate to strong complete membrane staining is observed in more than 10% of tumor cells.

In some embodiments, tumors that exhibit a 0 or 1+ score for overexpression of the FGFR3 S249C mutant may be characterized as not overexpressing the FGFR3 S249C mutant, whereas tumors exhibiting a 2+ or 3+ score may be characterized as overexpressing the FGFR3 S249C mutant.

In some embodiments, tumors that overexpress the FGFR3 S249C mutant may be assessed by an immunohistochemical score corresponding to the number of copies of the FGFR3 S249C mutant molecule expressed per cell, which can be biochemically quantified. That is:

0 = 0-90 copies/cell
1+ = at least about 100 copies/cell
2+ = at least about 1,000 copies/cell
3+ = at least about 10,000 copies/cell

Alternatively, or in addition, a FISH assay can be performed on formalin-fixed, paraffin-embedded tumor tissue to determine (if any) the presence and/or extent of FGFR3 S249C mutant amplification or translocation in the tumor.

FGFR3 S249C mutant activity

FGFR3 S249C mutant activity can be quantified directly (*e.g.*, by the phospho-ELISA test, or other methods of quantifying phosphorylated receptors) or indirectly (*e.g.*, detection of activated downstream signaling pathway components, receptor dimers (*e.g.*, homodimers, heterodimers), gene expression profile, etc.).

Similarly, constitutive FGFR3 S249C mutant activation and/or ligand-independent or ligand-dependent FGFR3 S249C mutant can be quantified directly or indirectly (*e.g.*, by detecting receptor mutations that correlate with constitutive activity, or by detecting receptor amplification that correlates with constitutive activity).

Methods for detecting nucleic acid mutations are known in the art. Often, but not necessarily, the target nucleic acid in the sample is amplified to obtain the desired amount of material for determining if a mutation is present. Amplification techniques are well known. For example, the amplified product may or may not include all nucleic acid sequences encoding the protein of interest, as long as it contains a particular amino acid sequence position/nucleic acid sequence position where mutation is predicted to be.

In one example, the presence of a mutation can be determined by contacting a nucleic acid from a sample with a nucleic acid probe capable of specifically hybridizing to a nucleic acid encoding a mutated nucleic acid, and detecting the hybridization. In one embodiment, the probe is detectably labeled with, for example, a radioisotope (3H, 32P, 33P, etc.), a fluorescent agent (rhodamine, fluorescein, etc.) or a chromogenic agent. In some embodiments, the probe is an antisense oligomer, such as PNA, morpholino-phosphoroamidates, LNA, or 2'-alkoxyalkoxy. The probe can be from about 8 nucleotides to about 100 nucleotides, or from about 10 to about 75, or from about 15 to about 50, or from about 20 to about 30. In another embodiment, the nucleic acid probe of the present disclosure is provided as a kit for identifying FGFR3 mutations in a sample, and the kit contains an oligonucleotide that specifically hybridizes to or adjacent to a site of mutation in the nucleic acid encoding FGFR3. The kit may further include instructions for treating patients with tumors containing an FGFR3 mutation with FGFR3 antagonists based on the results of hybridization tests using the kit.

Mutations can also be detected by comparing the electrophoretic mobility of the corresponding nucleic acid encoding wild-type FGFR3 with the electrophoretic mobility of the amplified nucleic acid. Differences in mobility indicate the presence of mutations in the amplified nucleic acid sequence. Electrophoretic mobility can be measured by any suitable molecular separation technique, for example on a polyacrylamide gel.

In addition, nucleic acids can be analyzed for mutation detection using Enzymatic Mutation Detection method (EMD) (Del Tito et al., Clinical Chemistry 44: 731-739, 1998). EMD uses the bacteriophage resolvase T4 endonuclease VII, which scans along double-stranded DNA until it detects and cleaves structural distortions caused by base pair mismatches resulting from nucleic acid mutations such as point mutations, insertions, and deletions. For example, detection by gel electrophoresis of two short fragments formed by resolvase cleavage indicates the presence of a mutation. The advantage of the EMD method is that it is a single protocol that identifies point mutations, deletions, and insertions assayed directly from the amplification reaction, eliminating the need to purify the sample, reducing hybridization time, and increasing the signal-to-noise ratio. Mixed samples containing up to 20-fold excess normal nucleic acid and fragments up to 4 kb in size can be assayed. However, EMD scans do not identify specific base changes that occur in mutation-positive samples and require additional sequencing procedures to identify specific mutations, if necessary. The CELI enzyme can be used in the same manner as the resolvase T4 endonuclease VII as shown in U.S. Pat. No. 5869245.

Another convenient kit for detecting mutations is the reverse hybridization test strip similar to the Hemochromatosis StripAssay™ (ViennaLabs http://www.bamburgmarrsh.com/pdf/4220.pdf) for the detection of multiple mutations in the HFE, TFR2 and FPN1 genes that cause hemochromatosis. Such assays are based on sequence-specific hybridization following amplification by PCR. For single mutation assays, a microplate-based detection system can be applied, while for multiple mutation assays, the strip can be used as a "macro-array". The kit may include ready-to-use reagents for sample preparation, amplification, and mutation detection. The multiple amplification protocol provides convenience and allows testing of samples in very limited volumes. Using a direct StripAssay embodiment, testing for 20 and more mutations can be completed in less than 5 hours without the use of expensive equipment. The DNA is isolated from the sample, and the target nucleic acid is amplified *in vitro* (*e.g.*, PCR), and generally biotin-labeled in a single ("multiplex") amplification reaction. The amplification products are selectively hybridized to oligonucleotide probes (wild-type and variant-specific) immobilized on a solid support such as a strip in which the probes are immobilized as parallel lines or bands. Bound biotin-labeled amplicons are detected using streptavidin-alkaline phosphatase and color substrates. Such an assay can detect all or any subset of the mutations disclosed in the present disclosure.

One of three signaling patterns is possible for a particular mutant probe band: (i) a band for only a wild-type probe showing a normal nucleic acid sequence, (ii) bands for both wild-type and mutant probes showing a heterozygous genotype, and (iii) a band for only the mutant probe showing a homozygous mutant genotype.

Accordingly, in a certain embodiment, the present disclosure provides a method of detecting mutations of the present disclosure by isolating and/or amplifying a target FGFR3 nucleic acid sequence from a sample such that the amplification product contains a ligand, contacting the amplification product with a probe containing a detectable binding partner for the ligand which can specifically hybridize with a mutant of the present disclosure, followed by detecting the hybridization of the probe to the amplification product. In one embodiment, the ligand is biotin and the binding partner comprises avidin or streptavidin. In one embodiment, the binding partner is streptavidin-alkaline, detectable with a color substrate. In one embodiment, the probes are anchored, *e.g.*, on a strip, where probes that are complementary to different mutations are separated from each other. Alternatively, the amplified nucleic acid is labeled with a radioisotope, in which case the probe does not need to contain a detectable label.

Alteration of wild-type genes includes all forms of mutations such as insertions, inversions, deletions, and/or point mutations. In one embodiment, the mutations are somatic. Somatic mutations occur only in specific tissues, such as tumor tissues, and are not inherited by germlines. Germline mutations are found in all body tissues.

Samples containing the target nucleic acid can be obtained by methods well known in the art that are suitable for the particular type and location of the tumor. Tissue biopsy is often used to obtain a representative portion of tumor tissue. Tumor cells, on the other hand, are indirectly obtainable in the form of tissue/fluid that is known to or may contain the tumor cells of interest. For example, samples of lung cancer lesions may be obtained by excision, bronchoscopy, fine needle aspiration, bronchial brushings, or from sputum, pleural fluid or blood. Mutant genes or gene products can be detected in tumors or in other body samples such as urine, sputum or serum. The same techniques described above for the detection of mutant target genes or gene products in tumor samples can be applied to other body samples. Cancer cells detach from the tumor and appear in such body samples. By screening such a body sample, a disease such as cancer can be diagnosed easily and quickly. In addition, the course of treatment can be more easily monitored by testing such body samples for mutation target genes or gene products.

Means for concentrating a tissue preparation of tumor cells are known. For example, the tissue may be isolated from a paraffin or cryostat section. In addition, cancer cells may be separated from normal cells by flow cytometry or laser capture microdissection. These as well as other techniques for isolating tumors from normal cells are known. Mutations are more difficult to detect when the tumor tissue is highly mixed with normal cells, but techniques are known to minimize contamination and/or false positive/negative results. Some of them are described below. For example, a sample can be evaluated for the presence of biomarkers (including mutations) that are known to bind to tumor cells of interest but not to the corresponding normal cells, and *vice versa.*

Detection of point mutations in a target nucleic acid can be achieved by molecular cloning of the target nucleic acid and sequencing the nucleic acid using techniques well known in the art. Alternatively, the polymerase chain reaction (PCR) can be used to amplify the target nucleic acid sequence directly from a genomic DNA preparation from tumor tissue. The nucleic acid sequence of the amplified sequence can then be determined and the mutation can be identified therefrom. Amplification techniques are well known in the art and are described in Saiki et al., Science 239: 487, 1988; U.S. Pat. No. 4683203; and U.S. Pat. No. 4683195.

Note that the design and selection of appropriate primers have been established in prior art.

In addition, a ligase chain reaction known in the art can be used to amplify the target nucleic acid sequence. For example, see Wu et al., Genomics, Vol. 4, pp. See 560-569 (1989). In addition, a technique known as allele-specific PCR can be used. For example, see Ruano and Kidd, Nucleic Acids Research, Vol. 17, p. See 8392, 1989. According to this technique, primers that hybridize at their 3' ends to a particular target nucleic acid mutation are used. In the absence of a particular mutation, no amplification product is observed. The Amplification Refractory Mutation System (ARMS) disclosed in European Patent Application Publication No. 0332435 and Newton et al., Nucleic Acids Research, Vol. 17, p. 7, 1989 can also be used. Gene insertions and deletions can also be detected by cloning, sequencing, and amplification. In addition, restriction fragment length polymorphism (RFLP) probes for the gene or surrounding marker genes can be used to score an allelic alteration or insertion in a polymorphic fragment. Single-strand DNA conformation polymorphism (SSCP) analysis can also be used to detect base change variants of an allele. See for example, Orita et al., Proc. Natl. Acad. Sci., USA Vol. 86, pp. 2766-2770, 1989, and Genomics, Vol. 5, pp. See 874-879, 1989. Alternatively, other methods of detecting insertions and deletions known in the art can be used.

Alteration of the wild-type gene can also be detected based on the alteration of the wild-type gene expression product. Such expression products include both mRNA and protein products. Point mutations can be detected by molecular cloning of cDNAs made from mRNA or by amplifying and sequencing the mRNA. The sequence of the cloned cDNA can be determined using DNA sequencing methods well known in the art. The cDNA can also be sequenced by a polymerase chain reaction (PCR).

A mismatch is a hybridized nucleic acid double strand that is not 100% complementary. Lack of complete complementarity can be due to a deletion, insertion, inversion, substitution or frameshift mutation. Mismatch detection can be used to detect point mutations in a target nucleic acid. Although these techniques are less sensitive than sequencing, they can be more conveniently performed on large numbers of tumor samples. An example of a mismatch cleavage technique is the ribonuclease protection method, which is described in detail in Winter et al., Proc. Natl. Acad. Sci., USA, Vol. 82, p. 7575, 1985 and Meyers et al., Science, Vol. 230, p. 1242 and 1985. For example, a method of the present disclosure may involve the use of a labeled riboprobe complementary to the human wild-type target nucleic acid. The riboprobe and the target nucleic acid from the tumor sample are annealed (hybridized) together, and subsequently digested with the enzyme ribonuclease A, which can detect some mismatches in the double-stranded RNA structure. If a mismatch is detected by ribonuclease A, it is cleaved at the mismatch site. Thus, when the annealed RNA preparation is separated on an electrophoretic gel matrix and when the mismatch is detected and cleaved by ribonuclease A, an RNA product that is smaller than the full-length double-stranded RNA for the riboprobe and mRNA or DNA can be seen. The riboprobe does not have to be the full length of the target nucleic acid mRNA or gene, but is a part of the target nucleic acid and contains sites that are suspected to be mutated. If the riboprobe contains only a segment of the target nucleic acid mRNA or gene, it is desirable to use many of these probes to screen the entire target nucleic acid sequence for mismatches.

In a similar manner, a DNA probe can be used to detect a mismatch via enzymatic or chemical cleavage. For example, see Cotton et al., Proc. Natl. Acad. Sci., USA, Vol. 85, 4397, 1988; and Shenk et al., Proc. Natl. Acad. Sci., USA, Vol. 72, p. 989, 1975. Alternatively, mismatches can be detected by a change in the electrophoretic mobility of the mismatched duplex with respect to the matched duplex. For example, see Cariello, Human Genetics, Vol. 42, p. 726, 1988. Using either a riboprobe or a DNA probe, the target nucleic acid mRNA or DNA that may contain the mutation can be amplified prior to hybridization. DNA changes in the target nucleic acid can also be detected using Southern hybridization, especially if the changes are gross rearrangements, such as deletions and insertions.

It is also possible to screen the amplified DNA sequence of the target nucleic acid using an allele-specific probe. These probes are nucleic acid oligomers, each containing a region of the target nucleic acid gene with a known mutation. For example, one oligomer can be about 30 nucleotides in length, corresponding to a portion of the target gene sequence. The use of such a series of allele-specific probes makes it possible to screen target nucleic acid amplification products and identify the presence of previously identified mutations in the target gene. Hybridization of the allele-specific probe with the amplified target nucleic acid sequence can be performed, for example, with a nylon filter. Hybridization of specific probes under stringent hybridization conditions indicates that the same mutations in allele-specific probes are present in the tumor tissue.

Alterations of wild-type target genes can also be detected by screening for alterations of the corresponding wild-type protein. For example, tissues can be screened using monoclonal antibodies that are immunoreactive with the target gene product, such as antibodies that are known to bind to specific mutated positions in the gene product (protein). For example, the antibody used can be an antibody that binds to a deleted exon or an antibody that binds to a conformational epitope comprising a deleted site of a target protein. Lack of a cognate antigen will indicate a mutation. In addition, antibodies specific for products of mutant alleles can be used to detect the mutant gene products. Antibodies can be identified from a phage display library. Such immunological assays can be performed in any convenient format known in the art. These include Western blots, immunohistochemical assays, and ELISA assays. Any means for detecting an altered protein can be used to detect an alteration of the wild-type target gene.

Primer pairs are useful for determining the nucleotide sequence of a target nucleic acid using nucleic acid amplification techniques such as the polymerase chain reaction. A pair of single-stranded DNA primers can be annealed to or around the target nucleic acid sequence to prime amplification of the target sequence. Allele-specific primers can also be used. Such primers anneal only to specific mutation target sequences and thus amplify the product only in the presence of the mutation target sequence as a template. To facilitate subsequent cloning of amplified sequences, primers may have restriction enzyme site sequences attached to their ends. Such enzymes and sites are well known in the art. The primers themselves can be synthesized using techniques well known in the art. In general, primers can be made using a commercially available oligonucleotide synthesizer. The design of a particular primer is well within the skill of one of ordinary skill in the art.

Nucleic acid probes are useful for many purposes. They can be used in Southern hybridization to genomic DNA and in ribonuclease protection methods for detecting point mutations already discussed above. The probes can be used to detect target nucleic acid amplification products. They can also be used to detect mismatches with wild-type genes or mRNAs using other techniques. A mismatch can be detected using either an enzyme (e.g., S1nuclease), a chemical (e.g., hydroxylamine or osmium tetroxide and piperidine), or a change in the electrophoretic mobility of the mismatch hybrid when compared to a perfectly matched hybrid. These techniques are known in the art. See Novack et al., Proc. Natl. Acad. Sci., USA, Vol. 83, p. 586, 1986. In general, probes are complementary to sequences outside the kinase domain. A series of nucleic acid probes can be used to construct a kit for detecting mutations in a target nucleic acid. The kit allows hybridization of the target sequence of interest to a large region. The probes may overlap with or be in close proximity to each other.

When a riboprobe is used to detect a mismatch with mRNA, it is complementary to the mRNA of the target gene. Thus, the riboprobe is an antisense probe in that it does not code for the corresponding gene product because it is complementary to the sense strand. Riboprobes are generally labeled with radioactive, colorimetric, or fluorometric substance, which can be achieved by any means known in the art. When a riboprobe is used to detect a mismatch with DNA, it can be of either polarity, sense or antisense. Similarly, DNA probes can also be used to detect mismatches.

Chemotherapeutic agents

The combination therapies of the present disclosure may further include one or more chemotherapeutic agents. Concomitant administration includes simultaneous or concurrent administration using separate or single pharmaceutical formulations, and continuous administration in any order which provides a period of time during which the biological activities of preferably both (or all) of the active agents are simultaneously exerted.

When administered, chemotherapeutic agents are usually given at doses known for such agents, or reduced doses in some cases due to adverse side effects resulting from the combined effect of the agents or the administration of antimetabolite chemotherapeutic agents. Preparation and dosing regimens of such chemotherapeutic agents can be done according to the manufacturer’s instructions or by empirical determination of those skilled in the art.

Various chemotherapeutic agents that can be used in combination are disclosed in the present specification.

In some embodiments, the preferred chemotherapeutic agents to combine are selected from the group consisting of lenalidomide (REVLIMID), proteasome inhibitors (e.g., bortezomib (VELCADE) and PS342), plant-derived (vincristine, vinblastine, etoposide, irinotecan, nogitecan, paclitaxel, docetaxel), bora taxoids (including docetaxel and paclitaxel), vinca (e.g., vinorelbine or vinblastine), platinum compounds (e.g., carboplatin, cisplatin or nedaplatin), aromatase inhibitors *(e.g.,* letrozole, anastrozole, or exemestane), anti-estrogens *(e.g.,* fulvestrant or tamoxifen), etoposide, thiotepa, alkylating agents (cycphosphamide, ifosfamide), methotrexate, liposomal doxorubicin, pegylated liposomal doxorubicin, capecitabine, gemcitabine, vinorelbine, melthalin, anticancer antibiotics (bleomycin, peblomycin, mitomycin C, doxorubicin, epirubicin, pirarubicin), vincristine, irinotecan, COX-2 inhibitors (*e.g.*, celecoxib) or steroids (*e.g.*, dexamethasone and prednisone), metabolic antagonists (5-fluorouracil, UFT (tegafur uracil), doxifluridine, and TS-1 (also abbreviated as S -1) (tegafur, gimeracil, and oteracil potassium), cytarabine). In some embodiments (*e.g.*, embodiments associated with the treatment of t(4;14) + multiple myeloma), dexamethasone and lenalidomide, or dexamethasone, or bortezomib, or vincristine, doxorubicin and dexamethasone, or thalidomide and dexamethasone, or liposome doxorubicin, vincristine and dexamethasone, or lenalidomide and dexamethasone, or bortezomib and dexamethasone, or bortezomib, doxorubicin, and dexamethasone are combined. In some embodiments (*e.g.*, embodiments involving bladder cancer), taxanes (*e.g.*, paclitaxel, docetaxel), or pemetrexed, or methotrexate, vinblastine, doxorubicin and cisplatin, or carboplatin, or mitomycin C in combination with 5-fluorouracil, or cisplatin, or cisplatin and 5-fluorouracil are combined.

Formulations, doses, and administrations

The formulations, doses, and administration of therapeutic agents used in the present disclosure are determined in accordance with good medical practice. Factors to consider in this context include the particular disorder to be treated, the particular subject to be treated, the clinical condition of the individual patient, the cause of the disorder, the site of delivery of the drug, the method of administration, the dosing regimen, and interactions between drugs used in combination, and other factors known to healthcare professionals.

Therapeutic formulations are prepared using standard methods known in the art, by mixing the active ingredient of the desired purity with optional physiologically acceptable carriers, excipients or stabilizers (Remington's Pharmaceutical Sciences (20th edition), A. Gennaro ed., 2000, Lippincott, Williams & Wilkins, Philadelphia, PA).

The formulations herein can also include two or more active compounds required for a particular symptom to be treated, preferably compounds with complementary activities that do not reverse the effect of each other. Such molecules are appropriately combined in an amount effective for the intended purpose.

The therapeutic agent of the present disclosure can be administered to a human patient by intravenous administration as a bolus, or continuous infusion over a certain period of time, or according to known methods such as intramuscular, intraoral, intracerebrospinal, subcutaneous, intraarticular, intrameningeal, intrathecal, oral, or topical administration, or through inhalation. Appropriate administration methods can be selected according to the patient's age and symptoms. The dosage can be selected, for example, from 0.0001 mg to 1,000 mg/kg body weight for each dose. Alternatively, the dose can be selected, for example, from 0.001 mg/body to 100,000 mg/body per patient. However, the doses of the pharmaceutical compositions of the present invention are not limited thereto. *Ex vivo* strategies can also be used for therapeutic applications. In *ex vivo* strategies, cells harvested from a subject are transfected or transduced with a polynucleotide encoding an FGFR3 antagonist. The transfected or transduced cells are then returned to the subject. Cells can be any of a wide variety including, without limitation, hematopoietic cells (e.g., bone marrow cells, macrophages, monocytes, dendritic cells, T cells, or B cells), fibroblasts, epithelial cells, endothelial cells, keratinocytes, or muscle cells.

Gene therapy to generate intracellular antibodies

In this application, administration of anti-antibodies by gene therapy is considered. See, for example, WO 96/07321 published March 14, 1996, on the use of gene therapy to generate intracellular antibodies.

Products

The following is an example of the method and composition of the present disclosure. It should be understood that various other embodiments are feasible based on the above overview.

[Examples]

[Example 1] Preparation of antibodies that selectively bind to the human FGFR3 (hFGFR3) S249C mutant.

<u>Preparation of hFGFR3-expressing CHO cells (hFGFR3/CHO DXB11s) and hFGFR3 S249C mutant-expressing CHO cells (hFGFR3/S249C/CHO DXB11s)</u>

The cDNA sequence (SEQ ID NO: 2) encoding the hFGFR3 protein (SEQ ID NO: 1) and the cDNA sequence (SEQ ID NO: 4) encoding the hFGFR3 S249C mutant protein (SEQ ID NO: 3) were inserted into pCXND3 (WO2008/156083) to prepare expression vectors. The PvuI-digested products of these vectors were introduced into CHO cells (DXB11s, distributed by the University of Tokyo) by the electroporation method (LONZA, Nucleofector). Gene-introduced cell lines were selected with 500 μg/mL Geneticin. After drug selection, the selected cell lines were seeded into 96-well plates so as to be 1 cell/well using a cell sorter (FACS Aria III, BD) in order to obtain single cell-derived clones, and expansion cultures were performed to establish monoclonal cell lines (hFGFR3/CHO DXB11s cells and hFGFR3 S249C/CHO DXB11s cells).

Measurement of hFGFR3 expression level in CHO cells forcibly expressing wild-type hFGFR3 (hFGFR3/CHO DXB11s) and CHO cells forcibly expressing the hFGFR3 S249C mutant (hFGFR3 S249C/CHO DXB11s)

The antibody binding capacity (ABC) of wild-type hFGFR3 or the hFGFR3 S249C mutant in hFGFR3/CHO DXB11s and hFGFR3 S249C/CHO DXB11s cells was measured using QIFI Kit (DAKO). Anti-FGFR3 antibody (R&D systems) that binds to both wild-type and S249C mutant was added to each of $5.0 \times 10^4$ cells to a final concentration of 20 μg/mL and incubated at 4°C, for 30 minutes. After washing, a secondary antibody (attached to the kit) was added at 1/50 volume and incubated at 4°C, for 30 minutes. The washed cells were analyzed using FACS Lyric and the ABC was calculated. ABC was $2.69 \times 10^4$/cell for FGFR3/CHO DXB11s and $2.58 \times 10^4$/cell for hFGFR3 S249C/CHO DXB11s, showing almost the same expression levels.

[Table 2]

| Cell line | Antibody binding capacity (ABC) (antibodies/cell) |
|---|---|
| hFGFR3/CHO DXB11s | $2.69 \times 10^4$ |
| hFGFR3 S249C/CHO DXB11s | $2.58 \times 10^4$ |

[Example 2] Preparation of anti-hFGFR3 S249C mutant antibodies

Antibodies in this specification are named according to the following rule:
(Heavy chain variable region)-(Heavy chain constant region)/(Light chain variable region)-(Light chain constant region)
For example, if the antibody name is A0000-B0000/C0000-D0000, it means that the heavy chain variable region of this antibody is A0000, the heavy chain constant region is B0000, the light chain variable region is C0000, and the light chain constant region is D0000.
Similarly, the variable region of the antibody may be indicated according to the following rule:
(Heavy chain variable region)/(Light chain variable region).
For example, if the antibody name is A0000/C0000, it means that the heavy chain variable region of this antibody is A0000 and the light chain variable region is C0000.
Furthermore, bispecific antibodies are named according to the following rule:
AA (first antibody heavy chain)/XX (first antibody light chain)//BB (second antibody heavy chain)/YY (second antibody light chain).
In the preparation of anti-hFGFR3 S249C mutant antibodies, DNA immunization (SEQ ID NO: 4) was performed using a Helios Gene Gun and an *in vivo* gene transfer apparatus (ECM830) for immunization of rabbits, and monoclonal antibody screening was performed by flow cytometry using hFGFR3/CHO DXB11s and hFGFR3 S249C/CHO DXB11s described in Example 1.
For the immunization, 12-week-old rabbits (New Zealand white rabbits, purchased from Kitayama Labes Co., Ltd.) were used. For gene transfer by gene gun, plasmid DNA (SEQ ID NO: 4) encoding the hFGFR3 S249C mutant were coated on gold particles and immunized to the abdomen of rabbits according to the Helios Gene Gun simple operations manual of BIO-RAD. At the same time as gene transfer by the gene gun, the same expression plasmid was introduced into the thigh muscles of both legs of rabbits and the skin of both shoulders and legs using an *in vivo* gene transfer apparatus (ECM830). The immunization schedule was once a week, and a total of 6 to 10 immunizations were performed. Blood and spleens were collected after confirming an increase in antibody titer due to DNA immunization.
Peripheral blood mononuclear cells and spleen cells prepared from blood and spleens were stained with anti-rabbit IgG-PE, and B cells expressing B cell receptors on the surface were concentrated with a cell sorter. The acquired B cells were seeded on a 96-well plate and cultured in BT medium for 6-12 days in the presence of 25,000 cells/well EL4 cells (cell growth suppressed by prior X-ray irradiation treatment). As the BT medium, RPMI 1640 with L-Gln (nacalai tesque) was used as the basic medium, to which Fetal Bovine Serum, ultra-low IgG (Life technologies) and 1/20-fold diluted rabbit T cell culture medium were added. Rabbit T cell culture medium was prepared by culturing rabbit T cells in RPMI-1640 medium supplemented with Phytohemagglutinin-M (Roche, Cat No. 1 1082132-001), phorbol 12-myristate 13-acetate (Sigma, Cat No. P1585) and 2% FBS.

The screening was carried out as follows. After reacting the rabbit antibody secreted into the B cell culture medium with the FGFR3-expressing cell lines (CHO DXB11s cells, hFGFR3/CHO DXB11s cells, and hFGFR3 S249C/CHO DXB11s cells), the cells were stained with a secondary antibody against the rabbit antibody bound to the cell surface (Goat Anti-Rabbit IgG (H+L)-Alexa647, Thermo Fisher Scientific, Cat No. A-21244), and then analyzed with a flow cytometer (CyAn™ ADP, Beckman Coulter). As a result of the screening, wells containing an antibody that binds more strongly to hFGFR3 S249C/CHO DXB11s cells as compared to the binding to CHO DXB11s cells and hFGFR3/CHO DXB11s cells were selected as positive wells containing B cells expressing an antibody that selectively binds to the FGFR3 S249C mutant.

The antibody genes of the B cells in the selected positive wells were cloned as follows. B cells were lysed by treating with CellsDirect/Resuspension and Lysis Buffer (Life technologies, 11739-010), and then DNase I (E1009-A, Zymo Research) was added and reacted to disrupt the genome DNAs. The heavy and light chain cDNAs of the antibody genes were synthesized using SuperScript III CellsDirect cDNA Synthesis System (Life Technologies, 18080-300). Using the obtained cDNA as a template, the variable regions (FGA0002VH (SEQ ID NO: 5), FGA0002VL (SEQ ID NO: 6), FGA0005VH (SEQ ID NO: 7), FGA0005VL (SEQ ID NO: 8), FGA0007VH (SEQ ID NO: 9) and FGA0007VL (SEQ ID NO: 10)) were amplified by PCR (Platinum Taq DNA Polymerase, High Fidelity, Life Technologies, 11304-029), and the PCR amplification products were integrated into plasmid DNA encoding the human heavy chain constant region (F760mnN17, SEQ ID NO: 11) and the human light chain constant region (k0MTC, SEQ ID NO: 12) using In-fusion according to the manual.

[Reference Example 1] Expression and purification of antibodies

The prepared plasmids were transiently introduced into human embryonic kidney cancer cell-derived HEK293H strain (Invitrogen), FreeStyle293 cells (Invitrogen), or Expi293 cells (Invitrogen) to express the antibodies. The resultant culture supernatant was recovered, and filtered through a 0.22 μm filter (MILLEX (R)-GV (Millipore)) or a 0.45 μm filter (MILLEX (R)-GV (Millipore)) to obtain a culture supernatant. From this obtained culture supernatant, antibodies were purified by a method known to those skilled in the art using rProtein A Sepharose Fast Flow (GE Healthcare Japan) or Protein G Sepharose 4 Fast Flow (GE Healthcare Japan). The purified antibody concentration was calculated by measuring the absorbance at 280 nm using a spectrophotometer, and the antibody concentration was calculated from the obtained value using the absorption coefficient calculated by a method such as PACE (Protein Science 1995; 4: 2411-2423).

[Example 3] Preparation of bispecific antibodies

Normally, an antibody has two arms having the same antigen-binding activity, and thus, it is a bivalent antibody in which the two arms can simultaneously bind to a target antigen on the cell surface (referred to as "monospecific antibody" in the present specification). In contrast, antibodies composed of two arms with different antigen-binding activities are called bispecific antibodies (referred to as "bispecific antibody" in the present specification) and only one arm binds to cells expressing only one of the antigens (monovalent binding). Since the binding activity of a bivalent antibody and a bispecific antibody is different, bispecific antibodies were prepared in addition to the bivalent antibodies in order to evaluate monovalent binding and bivalent binding.

The preparation of bivalent antibodies selected by screening was carried out as follows. The antibody genes selected by B cell screening were introduced into Expi293 cells (Invitrogen) to express the antibodies. 200 mL of medium was added to the culture flask and transfection was performed according to the manufacturer's manual. After culturing for 4 days, cell supernatants were prepared, and the purified antibodies were obtained by column purification using rProtein A Sepharose (GE Healthcare) by a method known to those skilled in the art.

[Table 3]

| Bivalent antibody (monospecific antibody) | FGA0002 bi valent Ab: FGA0002VH-F760mnN17/ FGA0002VL-k0MTC | FGA0005 bi valent Ab: FGA0005VH-F760mnN17/ FGA0005VL-k0MTC | FGA0007 bi valent Ab: FGA0007VH-F760mnN17/ FGA0007VL-k0MTC |
|---|---|---|---|
| Heavy chain variable region | FGA0002VH (SEQ ID NO: 5) | FGA0005VH (SEQ ID NO: 7) | FGA0007VH (SEQ ID NO: 9) |
| Light chain variable region | FGA0002VL (SEQ ID NO: 6) | FGA0005VL (SEQ ID NO: 8) | FGA0007VL (SEQ ID NO: 10) |
| Heavy chain constant region | F760mnN17 (SEQ ID NO: 11) | F760mnN17 (SEQ ID NO: 11) | F760mnN17 (SEQ ID NO: 11) |

(continued)

| Bivalent antibody (monospecific antibody) | FGA0002 bi valent Ab: FGA0002VH-F760mnN17/ FGA0002VL-k0MTC | FGA0005 bi valent Ab: FGA0005VH-F760mnN17/ FGA0005VL-k0MTC | FGA0007 bi valent Ab: FGA0007VH-F760mnN17/ FGA0007VL-k0MTC |
|---|---|---|---|
| Light chain constant region | k0MTC (SEQ ID NO: 12) | k0MTC (SEQ ID NO: 1 2) | k0MTC (SEQ ID NO: 12) |

Next, the preparation of bispecific antibodies was carried out as follows. Two kinds of antibodies were mixed at 500 μg each, and 10 μL of 2-mercaptoethylamine· HCl (2-MEA, Sigma-Aldrich) prepared to 250 mM with D-PBS (-) (Wako Pure Chemical Industries, Ltd.) was added. The total volume was increased to 500 μL with D-PBS (-). After reacting this solution at 37 °C, for 17 hours, 2-MEA was removed by dialysis in D-PBS (-) using Toru-kun (Nippon Genetics). FGA0005 and FGA0007 and anti-CD3 antibody (heavy chain variable region: TR01H113 (SEQ ID NO: 13), heavy chain constant region: F760mnP17 (SEQ ID NO: 14), light chain variable region: L0011 (SEQ ID NO: 15), light chain constant region: k0 (SEQ ID NO: 16)) were mixed in the combination shown below under the reaction conditions to produce bispecific antibodies, and the reaction products were evaluated according to the method in Reference Example 2.

(1) FGA0002VH-F760mnN17/FGA0002VL-k0MTC and TR01H113-F760mnP17/L0011-k0
(2) FGA0005VH-F760mnN17/FGA0005VL-k0MTC and TR01H113-F760mnP17/L0011-k0
(3) FGA0007VH-F760mnN17/FGA0007VL-k0MTC and TR01H113-F760mnP17/L0011-k0 Reaction conditions: In PBS (pH 7.4), [each mAb] = 1.0 mg/ml, [2-MEA (Sigma)] = 25 mM, 37°C, overnight reaction.

[Reference Example 2] Evaluation of bispecific antibody production rate by ion exchange chromatography

Separation of each sample was evaluated using the ion exchange chromatography purification method using Prominence UFLC (Shimadzu Corporation). A tris buffer including 9.6 mM Tris, 6.0 mM piperazine, and 11.0 mM imidazole (pH 5.0) as well as a tris buffer including 150 mM sodium chloride-containing 9.6 mM Tris, 6.0 mM piperazine, and 11.0 mM imidazole (pH: 10.1) was used for the mobile phase, and ProPac WCX-10 (Thermo Scientific) was used as the column to separate the bispecific antibodies by the two-solution mixed gradient method. The data was acquired at a wavelength of 280 nm, and the elution result was evaluated using Empower2 (Waters).

The bispecific antibody production rate (%) was obtained by dividing the bispecific antibody peak area value by the peak area value of all antibodies present in the system and multiplying by 100. When the recovery rate of one of the homodimers was poor, the value obtained by combining the double value of the area of the other homodimer with the bispecific antibody peak area value was calculated as the peak area value of all antibodies.

[Table 4]

| Bispecific antibody | Structure |
|---|---|
| FGA0002/ CD3-TRAB | FGA0002VH-F760mnN17/FGA0002VL-k0MTC //TR01H113-F760mnP17/L0011-k0 |
| FGA0005/ CD3-TRAB | FGA0005VH-F760mnN17/FGA0005VL-k0MTC //TR01H113-F760mnP17/L0011-k0 |
| FGA0007/ CD3-TRAB | FGA0007VH-F760mnN17/FGA0007VL-k0MTC //TR01H113-F760mnP17/L0011-k0 |

As a result, monospecific anti-hFGFR3 S249C mutant antibodies (FGA0002 bivalent Ab, FGA0005 bivalent Ab and FGA0007 bivalent Ab) and bispecific anti-hFGFR3 S249C mutant/CD3 antibodies (FGA0002/CD3-TRAB, FGA0005/CD3-TRAB and FGA0007/CD3-TRAB), which are antibodies capable of specifically binding to the hFGFR3 S249C mutant were obtained. The amino acid sequences of the variable regions of the arm of each antibody that binds to the hFGFR3 S249C mutant and the CDRs (HVRs) that compose them are as follows:

[Table 5]

| | FGA0002 biv alent Ab FGA0002/CD3 -TRAB | FGA0005 biv alent Ab FGA0005/CD3 -TRAB | FGA0007 biv alent Ab FGA0007/CD3 -TRAB |
|---|---|---|---|
| VH | SEQ ID NO: 5 | SEQ ID NO: 7 | SEQ ID NO: 9 |
| CDR1 | SEQ ID NO: 1 7 | SEQ ID NO: 2 3 | SEQ ID NO: 2 9 |

(continued)

| | FGA0002 biv alent Ab FGA0002/CD3 -TRAB | FGA0005 biv alent Ab FGA0005/CD3 -TRAB | FGA0007 biv alent Ab FGA0007/CD3 -TRAB |
|---|---|---|---|
| CDR2 | SEQ ID NO: 1 8 | SEQ ID NO: 2 4 | SEQ ID NO: 3 0 |
| CDR3 | SEQ ID NO: 1 9 | SEQ ID NO: 2 5 | SEQ ID NO: 3 1 |
| FR1 | SEQ ID NO: 3 5 | SEQ ID NO: 3 9 | SEQ ID NO: 4 3 |
| F R 2 | SEQ ID NO: 3 6 | SEQ ID NO: 4 0 | SEQ ID NO: 4 4 |
| FR3 | SEQ ID NO: 3 7 | SEQ ID NO: 4 1 | SEQ ID NO: 4 5 |
| FR4 | SEQ ID NO: 3 8 | SEQ ID NO: 4 2 | SEQ ID NO: 4 6 |
| VL | SEQ ID NO: 6 | SEQ ID NO: 8 | SEQ ID NO: 9 |
| CDR1 | SEQ ID NO: 2 0 | SEQ ID NO: 2 6 | SEQ ID NO: 3 2 |
| CDR2 | SEQ ID NO: 2 1 | SEQ ID NO: 2 7 | SEQ ID NO: 3 3 |
| CDR3 | SEQ ID NO: 2 2 | SEQ ID NO: 2 8 | SEQ ID NO: 3 4 |
| FR1 | SEQ ID NO: 4 7 | SEQ ID NO: 5 1 | SEQ ID NO: 5 5 |
| FR2 | SEQ ID NO: 4 8 | SEQ ID NO: 5 2 | SEQ ID NO: 5 6 |
| FR3 | SEQ ID NO: 4 9 | SEQ ID NO: 5 3 | SEQ ID NO: 5 7 |
| FR4 | SEQ ID NO: 5 0 | SEQ ID NO: 5 4 | SEQ ID NO: 5 8 |

[Example 4] Measurement of the binding activity of (a) monospecific anti-hFGFR3 S249C mutant antibodies (FGA0005 bivalent Ab and FGA0007 bivalent Ab) and (b) bispecific anti-hFGFR3 S249C mutant/CD3 antibodies (FGA0005/CD3-TRAB and FGA0007/CD3-TRAB) in hFGFR3/CHO DXB11s and hFGFR3 S249C/CHO DXB11s cells

The binding activity of monospecific antibodies FGA0005 and FGA0007 and bispecific antibodies FGA0005/CD3-TRAB and FGA0007/CD3-TRAB against wild-type hFGFR3 or hFGFR3 S249C mutant on the cell surface of hFGFR3/CHO DXB11s cells and hFGFR3 S249C/CHO DXB11s cells prepared by the method described in Example 1 and parent strain CHO DXB11s cells was measured using flow cytometry.

After $9 \times 10^6$ cells of each cell line were washed with FACS/PBS supplemented with 0.5% BSA, 450 μL of Fixable Viability Dye 780 (eBioscience) diluted 5000 times was added to each of the cells. 25 μL each of the prepared cell suspensions was dispensed into v-bottom 96 well plates (Greiner Bio-one). Monospecific antibodies FGA0005 and FGA0007 with twice the final concentration of 0.01, 0.1, 1 μg/mL were prepared. In addition, bispecific antibodies FGA0005/CD3-TRAB and FGA0007/CD3-TRAB with twice the final concentration of 5, 10, and 20 μg/mL were prepared. 25 μL solutions containing the monospecific antibodies FGA0005 bivalent Ab and FGA0007 bivalent Ab, and the bispecific antibodies FGA0005/CD3-TRAB and FGA0007/CD3-TRAB prepared at a double concentration were added to the v-bottom 96 well plates, and allowed to stand at 4°C for 30 minutes. After the cells were washed with FACS/PBS, Goat anti-Human IgG Fc Cross-Adsorbed Secondary Antibody, Dylight 650 Conjugate (Invitrogen) diluted 100-fold with FACS/PBS was added at 50 μL each and left to stand at 4°C for 30 minutes. Cells were washed with FACS/PBS and then analyzed by flow cytometry.

As a result, the monospecific antibodies FGA0005 bivalent Ab and FGA0007 bivalent Ab, and the bispecific antibodies FGA0005/CD3-TRAB and FGA0007/CD3-TRAB showed volume-dependent binding activity to hFGFR3 S249C/CHO DXB11s cells, but they did not show binding activity to hFGFR3/CHO DXB11s cells and the parent strain CHO DXB11s. From the above, it was confirmed that the monospecific antibodies FGA0005 bivalent Ab and FGA0007 bivalent Ab, and the bispecific antibodies FGA0005/CD3-TRAB and FGA0007/CD3-TRAB show selective binding activity to the hFGFR3 S249C mutant compared to wild-type hFGFR3.

[Example 5] Measurement of hFGFR3 expression level on cell surface of human lung cancer cell PC10 cell line (PC10), PC10 cells forcibly expressing wild-type hFGFR3 (PC10/FGFR3 WT), and PC10 cells forcibly expressing the hFGFR3 S249C mutant (PC10/FGFR3 S249C)

The antibody binding capacity to wild-type hFGFR3 or hFGFR3 S249C mutant on the cell surface of PC10-derived cell lines was calculated by flow cytometry using QIFI Kit (DAKO).

The antibody binding capacity of FGFR3 or FGFR3 S249C in PC10, PC10/FGFR3 WT and PC10/FGFR3 S249C cells was measured using QIFI Kit (DAKO). An anti-FGFR3 antibody (R&D systems) that binds to both wild-type and S249C mutant was added to each $5 \times 10^5$ cells to a final concentration of 20 μg/mL and incubated at 4°C, for 30 minutes. After washing, a secondary antibody (attached to the kit) was added at 1/50 volume and incubated at 4°C, for 30 minutes. The washed cells were analyzed using flow cytometry and ABC was calculated.

ABC of FGFR3 WT or S249C on the cell surface in each cell line was calculated and shown in Table 6. ABC was $4.56 \times 10^4$ antibodies/cell for PC10/FGFR3 WT and $4.31 \times 10^4$ antibodies/cell for PC10/FGFR3 S249C, which were almost the same expression levels. In addition, in PC10, it was $1.94 \times 10^3$ antibodies/cell, showing almost no expression.

[Table 6]

| Cell line | Antibody binding capacity (ABC) (antibodies/cell) |
|---|---|
| PC10 | $1.94 \times 10^3$ |
| PC10/FGFR3 WT | $4.56 \times 10^4$ |
| PC10/FGFR3 S249C | $4.31 \times 10^4$ |

[Example 6] Measurement of binding activity of a bispecific antibody where one arm binds to the hFGFR3 S249C mutant and the other arm binds to CD3(FGA0002/CD3-TRAB), and a bispecific antibody where one arm does not bind to the hFGFR3 S249C mutant and the other arm binds to CD3 (KLH/CD3-TRAB) in PC10, PC10/FGFR3 WT, and PC10/FGFR3 S249C

For each cell line, the binding activity of FGA0002/CD3-TRAB to FGFR3 WT or FGFR3 S249C on the cell surface was calculated using flow cytometry.

$5.2 \times 10^6$ cells of each cell line were washed with FACS/PBS supplemented with 0.5% BSA, and then 325 μL of Fixable Viability Dye 780 (eBioscience) diluted 5000 times was added to each of the cells. 25 μL each of the prepared cell suspension was dispensed. A 25 μL solution containing FGA0002/CD3-TRAB or KLH/CD3-TRAB prepared at 2-fold concentration to obtain a final concentration of 3, 0.3, or 0.03 μg/mL, was added to the cell preparation solutions, and allowed to stand at 4°C for 30 minutes. After the cells were washed with FACS/PBS, 100-fold diluted Goat anti-Human IgG Fc Cross-Adsorbed Secondary Antibody, Dylight 650 Conjugate (Invitrogen) was added at 50 μL each, and left to stand at 4°C for 30 minutes. Cells were washed with FACS/PBS and then analyzed by flow cytometry.

As a result, FGA0002/CD3-TRAB showed strong binding activity to PC10/FGFR3 S249C, but showed almost no binding activity to PC 10 parent strain or PC10/FGFR3 WT. From the above, it was confirmed that FGA0002/CD3-TRAB exhibits selective binding activity to hFGFR3 S249C mutant compared to wild-type hFGFR3.

On the other hand, it was confirmed that KLH/CD3-TRAB does not show selective binding activity to the hFGFR3 S249C mutant.

[Example 7] T cell-dependent cellular cytotoxicity (TDCC) activity of FGA0002/CD3-TRAB in PC10, PC10/FGFR3 WT, PC10/FGFR3 S249C, using human peripheral blood mononuclear cells as effector cells

The TDCC activity of FGA0002/CD3-TRAB was measured according to the following method. The TDCC activity of each test antibody was measured as follows, using human peripheral blood mononuclear cells (hereinafter referred to as hPBMC) as effector cells.

(1) Preparation of target cells

The target cells were detached with trypsin (nacalai) and the cell number was measured. Cells were diluted with a culture solution of each cell (RPMI-1640 (SIGMA) containing 10% FBS) (hereinafter referred to as 10% FBS/RPMI-1640) so as to have $2 \times 10^5$ cells/ml. 50 μL each of medium was added to E-Plate 96 (ACEA Bioscience Inc), and the background cell index was measured. 50 μL of the prepared target cells was added into the E-plate, and successive measurement of cell index was started.

(2) Antibody preparation

An antibody solution was prepared the day following (1). The antibody solution diluted with the culture solution of the target cells to concentrations 4 times the final concentrations (0.12, 0.4, 1.2, 4, 12, and 40 μg/ml) was added to E-plate 96 at 50 μl per well. For antibodies, FGA0002/CD3-TRAB and KLH/CD3-TRAB as a control were used.

(3) Preparation of a human PBMC solution

The day following (1), human PBMC (Cellular Technology Limited (CTL)) that had been cryopreserved was thawed while adding 20-fold diluted Anti-Aggregate Wash (CTL). After centrifugation, the supernatant was removed, and the cells were suspended in 10% FBS/RPMI-1640 and counted. The cells were suspended in 10% FBS/RPMI-1640 so that the cell density was $2 \times 10^6$ cells/ml. 50 μl of the cell suspension was added to each well of E-plate 96 as effector cells.

[Example 8] Measurement of TDCC activity by xCELLigence

E-Plate to which target cells, antibody, and effector cells were added was placed in xCELLigence, and the cell index was measured every 10 minutes. Cell growth inhibition (CGI) % was calculated from cell index at 72 hours after the addition of the antibody and effector cells, and cell index immediately before the cell addition (all set to 1) by the following method. It was measured at N = 3 and the mean value was used in the following calculations.

$$CCI\% = 100 \text{ x } (A - B)/(A - 1)$$

A: Cell Index of wells (target cells and effector cells only) to which no antibody has been added. B: Cell Index of wells to which target cells, antibody, and effector cells have been added. The calculation was performed setting all cell indexes immediately before cell addition to 1.

As a result, FGA0002/CD3-TRAB showed strong TDCC activity against PC10/FGFR3 S249C, but showed only weak TDCC activity at a high concentration against PC10/FGFR3 WT. Moreover, it showed no activity against the PC10 parent strain. From the above, it was confirmed that FGA0002/CD3-TRAB exhibited TDCC activity selective for the hFGFR3 S249C mutant compared to wild-type hFGFR3.

[Industrial applicability]

As already mentioned, it is well known that FGFR3 plays an important role in cell proliferation. However, since wild-type FGFR3 is expressed not only in cancer tissues but also in normal tissues, an antibody that binds to it also binds to normal tissues. On the other hand, the FGFR3 S249C mutant is expressed specifically in cancer. Therefore, an antibody that distinguishes between wild-type FGFR3 and an FGFR3 S249C mutant can be a specific antibody drug against cancer cells. Alternatively, an antibody specific for an FGFR3 S249C mutant is also useful as a diagnostic tool in the imaging of cancer tissues, pathological examination of cancer cells, and the like.

In a preferred embodiment, the present disclosure provides novel multispecific antigen-binding molecules with a long blood half-life while maintaining strong antitumor activity and excellent safety properties such as not inducing cytokine storms in a cancer antigen-independent manner. A cytotoxic agent comprising an antigen-binding molecule of the present disclosure as an active ingredient can induce cytotoxicity by targeting FGFR3 S249C mutant-expressing cells and tumor tissues containing the cells. When a multispecific antigen-binding molecule of the present disclosure is administered to a patient, not only is it highly safe, but it also enables a desirable treatment that is less physically burdensome and more convenient.

## Claims

1. A multispecific antigen-binding molecule comprising: a first antigen-binding domain having selective binding activity to an FGFR3 S249C mutant; and a second antigen-binding domain having T cell receptor complex-binding activity.

2. The multispecific antigen-binding molecule according to claim 1, wherein the selective binding activity is such that the binding of the antigen-binding molecule to an FGFR3 S249C mutant-expressing cell line is similar to or greater than the binding of the antigen-binding molecule to a wild-type FGFR3-expressing cell line when the antigen-binding molecule is added to the wild-type FGFR3-expressing cell line at a concentration at least 10 times higher than that added to the FGFR3 S249C mutant-expressing cell line, and wherein the expressing cell lines are cell lines each expressing wild-type FGFR3 or FGFR3 S249C mutant to the same extent.

3. The multispecific antigen-binding molecule according to claim 1 or 2, wherein the multispecific antigen-binding molecule has cytotoxic activity.

4. The multispecific antigen-binding molecule according to any one of claims 1 to 3, wherein the T cell receptor complex-binding activity is the binding activity to a CD3 epsilon chain.

5. The multispecific antigen-binding molecule according to any one of claims 1 to 4, wherein the FGFR3 S249C mutant-binding activity is the binding activity to the FGFR3 S249C mutant on the surface of eukaryotic cells.

6. The multispecific antigen-binding molecule according to any one of claims 1 to 5, wherein either or both of the first antigen-binding domain and the second antigen-binding domain are antibody variable fragments.

7. An isolated multispecific antigen-binding molecule having a first antigen-binding domain and a second antigen-binding domain, wherein:

(a)

the heavy chain variable region of the first antigen-binding domain comprises:

HVR-H1 comprising the amino acid sequence of SEQ ID NO: 17,
HVR-H2 comprising the amino acid sequence of SEQ ID NO: 18, and
HVR-H3 comprising the amino acid sequence of SEQ ID NO: 19; and

the light chain variable region of the first antigen-binding domain comprises:

HVR-L1 comprising the amino acid sequence of SEQ ID NO: 20,
HVR-L2 comprising the amino acid sequence of SEQ ID NO: 21, and
HVR-L3 comprising the amino acid sequence of SEQ ID NO: 22;

(b)

the heavy chain variable region of the first antigen-binding domain comprises:

HVR-H1 comprising the amino acid sequence of SEQ ID NO: 23,
HVR-H2 comprising the amino acid sequence of SEQ ID NO: 24, and
HVR-H3 comprising the amino acid sequence of SEQ ID NO: 25; and

the light chain variable region of the first antigen-binding domain comprises:

HVR-L1 comprising the amino acid sequence of SEQ ID NO: 26,
HVR-L2 comprising the amino acid sequence of SEQ ID NO: 27, and
HVR-L3 comprising the amino acid sequence of SEQ ID NO: 28; or

(c)

the heavy chain variable region of the first antigen-binding domain comprises:

HVR-H1 comprising the amino acid sequence of SEQ ID NO: 29,
HVR-H2 comprising the amino acid sequence of SEQ ID NO: 30, and
HVR-H3 comprising the amino acid sequence of SEQ ID NO: 31; and

the light chain variable region of the first antigen-binding domain comprises:

HVR-L1 comprising the amino acid sequence of SEQ ID NO: 32,
HVR-L2 comprising the amino acid sequence of SEQ ID NO: 33, and
HVR-L3 comprising the amino acid sequence of SEQ ID NO: 34;

and wherein:

the heavy chain variable region of the second antigen-binding domain comprises:

HVR-H1 comprising the amino acid sequence of SEQ ID NO: 59,
HVR-H2 comprising the amino acid sequence of SEQ ID NO: 60, and
HVR-H3 comprising the amino acid sequence of SEQ ID NO: 61; and

the light chain variable region of the second antigen-binding domain comprises:

HVR-L1 comprising the amino acid sequence of SEQ ID NO: 62,
HVR-L2 comprising the amino acid sequence of SEQ ID NO: 63, and
HVR-L3 comprising the amino acid sequence of SEQ ID NO: 64.

**8.** The multispecific antigen-binding molecule according to any one of claims 1 to 7, further comprising an Fc region having reduced binding activity to Fc$\gamma$ receptor.

**9.** The multispecific antigen-binding molecule according to any one of claims 1 to 8, which is a bispecific antibody consisting of a first antibody variable fragment having FGFR3 S249C mutant-binding activity, a second antibody variable fragment having CD3 epsilon chain-binding activity, and an Fc region having reduced binding activity to FcyR.

**10.** A nucleic acid encoding the multispecific antigen-binding molecule according to any one of claims 1 to 9.

**11.** A vector into which the nucleic acid according to claim 10 has been introduced.

**12.** A cell comprising the nucleic acid according to claim 10 or the vector according to claim 11.

**13.** A pharmaceutical composition comprising the multispecific antigen-binding molecule according to any one of claims 1 to 9.

**14.** An isolated antigen-binding molecule that binds to an FGFR3 S249C mutant, which has higher binding activity to the FGFR3 S249C mutant than to wild-type FGFR3.

**15.** The antigen-binding molecule according to claims 1 to 10, wherein the binding to the FGFR3 S249C mutant competes with an antibody comprising the pair of SEQ ID NO: 5 and SEQ ID NO: 6, SEQ ID NO: 7 and SEQ ID NO: 8, or SEQ ID NO: 9 and SEQ ID NO: 10, or binds to the same epitope as an antibody comprising the pair of SEQ ID NO: 5 and SEQ ID NO: 6, SEQ ID NO: 7 and SEQ ID NO: 8, or SEQ ID NO: 9 and SEQ ID NO: 10.

**16.** The antigen-binding molecule according to claim 14 or 15, which is (a) a monoclonal antibody; (b) a human, humanized, or chimeric antibody; (c) an IgG antibody; or (d) an antibody fragment.

**17.** An isolated antigen-binding molecule which comprises any one of the following sets of six HVRs:

(a)

HVR-H1 comprising the amino acid sequence of SEQ ID NO: 17,
HVR-H2 comprising the amino acid sequence of SEQ ID NO: 18,
HVR-H3 comprising the amino acid sequence of SEQ ID NO: 19,
HVR-L1 comprising the amino acid sequence of SEQ ID NO: 20,
HVR-L2 comprising the amino acid sequence of SEQ ID NO: 21, and
HVR-L3 comprising the amino acid sequence of SEQ ID NO: 22;

(b)

HVR-H1 comprising the amino acid sequence of SEQ ID NO: 23,
HVR-H2 comprising the amino acid sequence of SEQ ID NO: 24,
HVR-H3 comprising the amino acid sequence of SEQ ID NO: 25,
HVR-L1 comprising the amino acid sequence of SEQ ID NO: 26,
HVR-L2 comprising the amino acid sequence of SEQ ID NO: 27, and
HVR-L3 comprising the amino acid sequence of SEQ ID NO: 28; or

(c)

HVR-H1 comprising the amino acid sequence of SEQ ID NO: 29,
HVR-H2 comprising the amino acid sequence of SEQ ID NO: 30,
HVR-H3 comprising the amino acid sequence of SEQ ID NO: 31,
HVR-L1 comprising the amino acid sequence of SEQ ID NO: 32,
HVR-L2 comprising the amino acid sequence of SEQ ID NO: 33, and
HVR-L3 comprising the amino acid sequence of SEQ ID NO: 34.

**18.** The antigen-binding molecule according to claim 7, further comprising: a heavy chain variable domain framework FR1 comprising any one of the amino acid sequences of SEQ ID NO: 39, 43 and 47; a heavy chain variable domain framework FR2 comprising any one of the amino acid sequences of SEQ ID NO: 40, 44 and 48; a heavy chain variable domain framework FR3 comprising any one of the amino acid sequences of SEQ ID NOs: 41, 45 and 49; and a heavy chain variable domain framework FR4 comprising the amino acid sequences of SEQ ID NOs: 42, 46 and 50.

**19.** The antigen-binding molecule according to any one of claims 14 to 17, which comprises

(a) a VH sequence having at least 95% sequence identity to any one of the amino acid sequences of SEQ ID NO: 5, 7, and 9;
(b) a VL sequence having at least 95% sequence identity to any one of the amino acid sequences of SEQ ID NO: 6, 8 and 10; or
(c) a VH sequence of any one of SEQ ID NOs: 5, 7, and 9 and a VL sequence of any one of SEQ ID NOs: 6, 8, and 10.

**20.** The antigen-binding molecule according to any one of claims 14 to 19, wherein the antigen-binding molecule is (a) a monoclonal antibody; (b) a humanized or chimeric antibody; (c) a human antibody; (d) a full-length IgG antibody; or (e) an antibody fragment.

**21.** A nucleic acid encoding the antigen-binding molecule according to claim 20.

**22.** A vector comprising the nucleic acid according to claim 21.

**23.** A cell comprising the nucleic acid according to claim 21 or the vector according to claim 22.

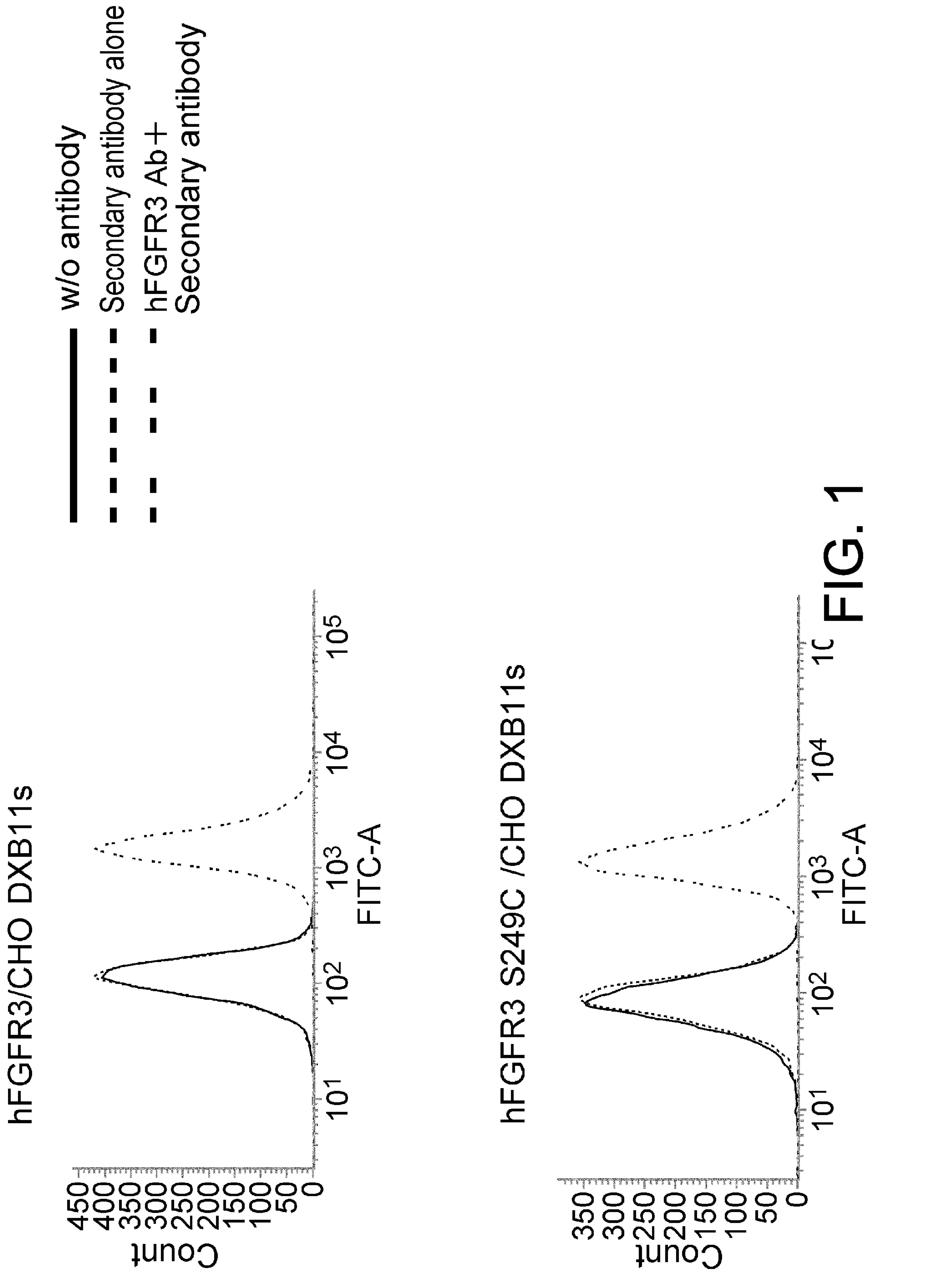
hFGFR3/CHO DXB11s
Count
450
400
350
300
250
200
150
100
50
0
10^1
10^2
10^3
10^4
10^5
FITC-A
hFGFR3 S249C /CHO DXB11s
Count
350
300
250
200
150
100
50
0
10^1
10^2
10^3
10^4
FITC-A
w/o antibody
Secondary antibody alone
hFGFR3 Ab +
Secondary antibody

FIG. 1

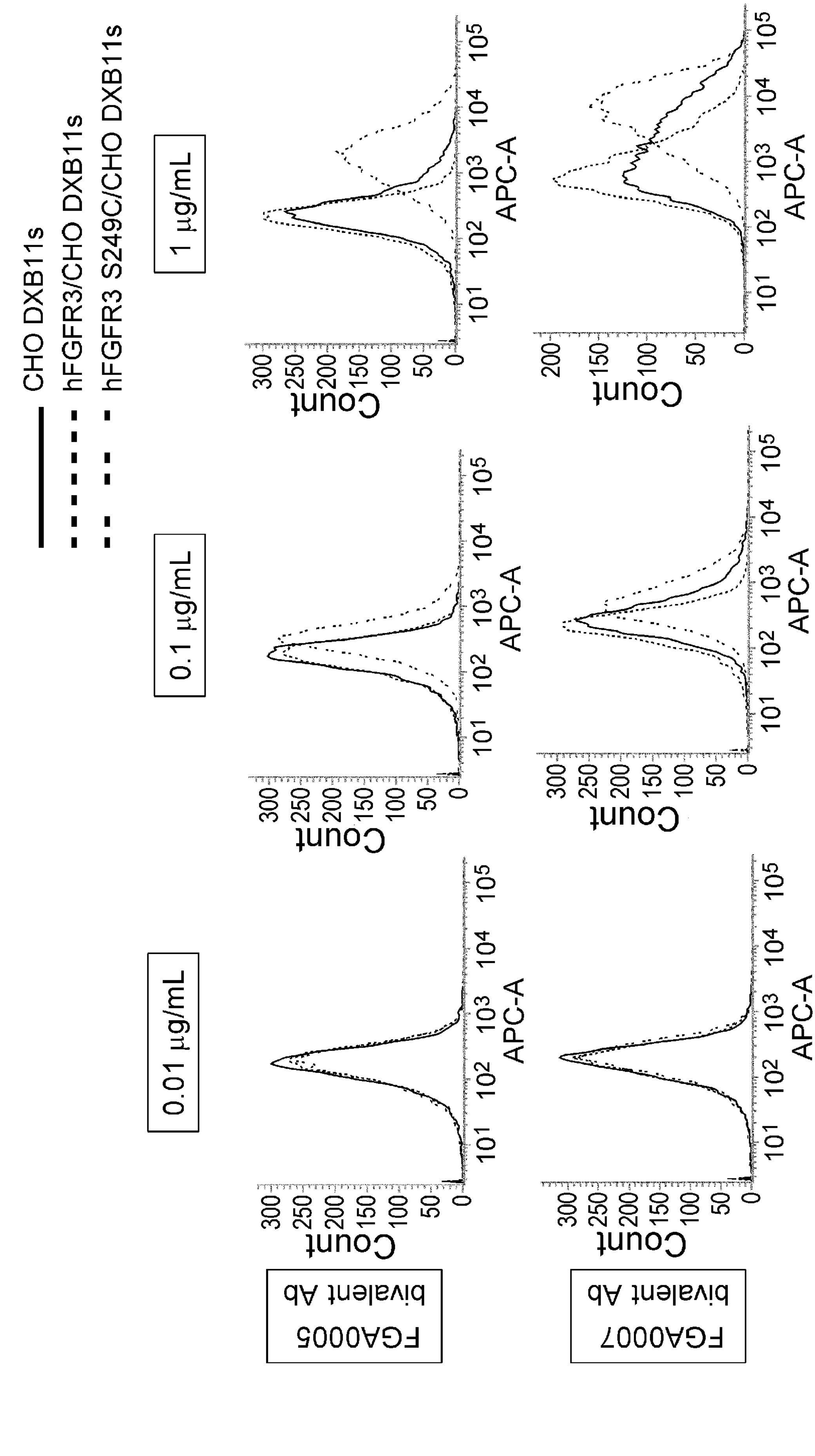
CHO DXB11s
hFGFR3/CHO DXB11s
hFGFR3 S249C/CHO DXB11s
0.01 μg/mL
0.1 μg/mL
1 μg/mL
FGA0005 bivalent Ab
FGA0007 bivalent Ab
Count
APC-A
0
50
100
150
200
250
300
$10^1$
$10^2$
$10^3$
$10^4$
$10^5$

FIG. 2a

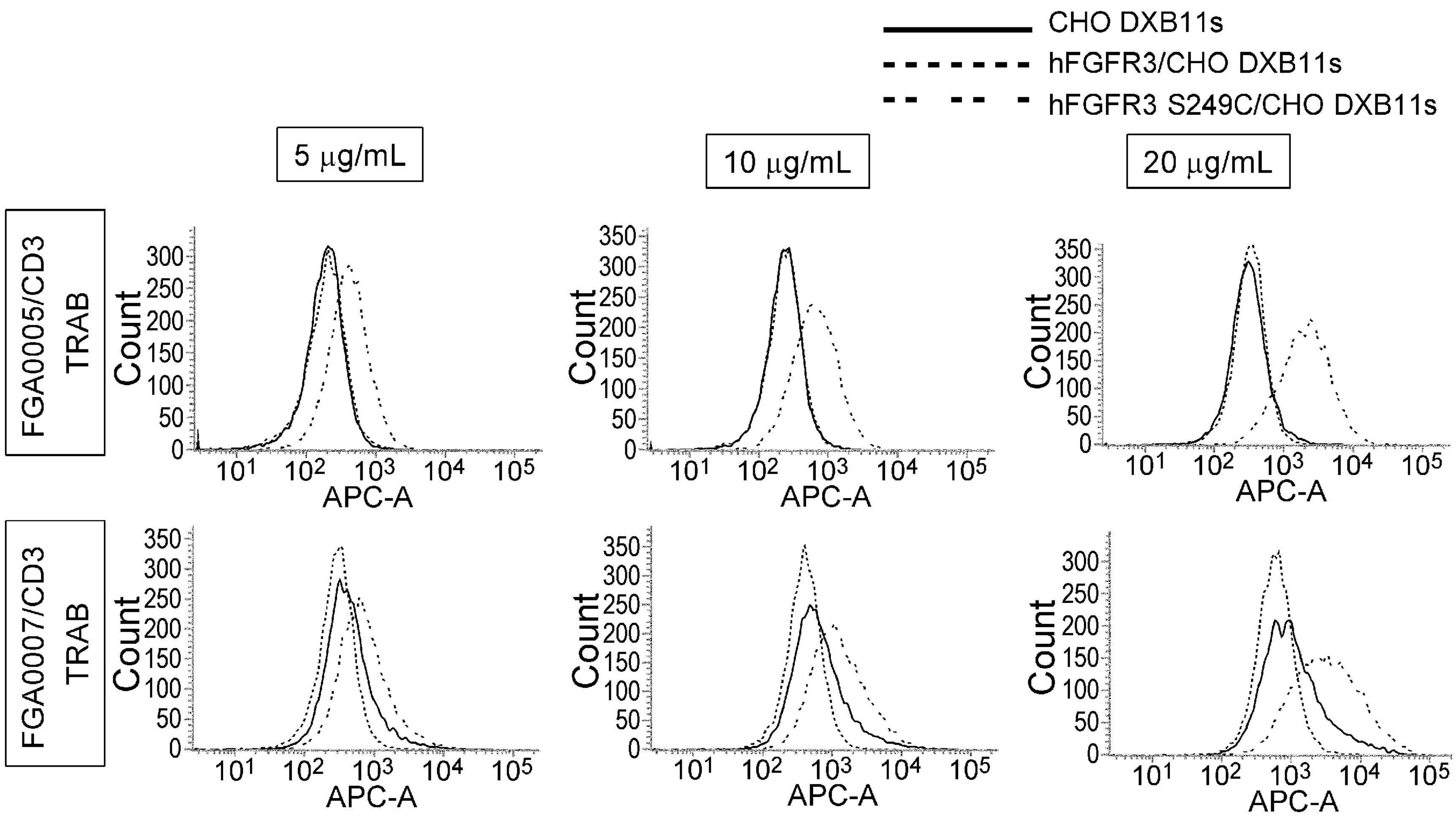
CHO DXB11s
hFGFR3/CHO DXB11s
hFGFR3 S249C/CHO DXB11s
5 μg/mL
10 μg/mL
20 μg/mL
FGA0005/CD3 TRAB
FGA0007/CD3 TRAB
Count
APC-A
0
50
100
150
200
250
300
350
$10^1$
$10^2$
$10^3$
$10^4$
$10^5$

FIG. 2b

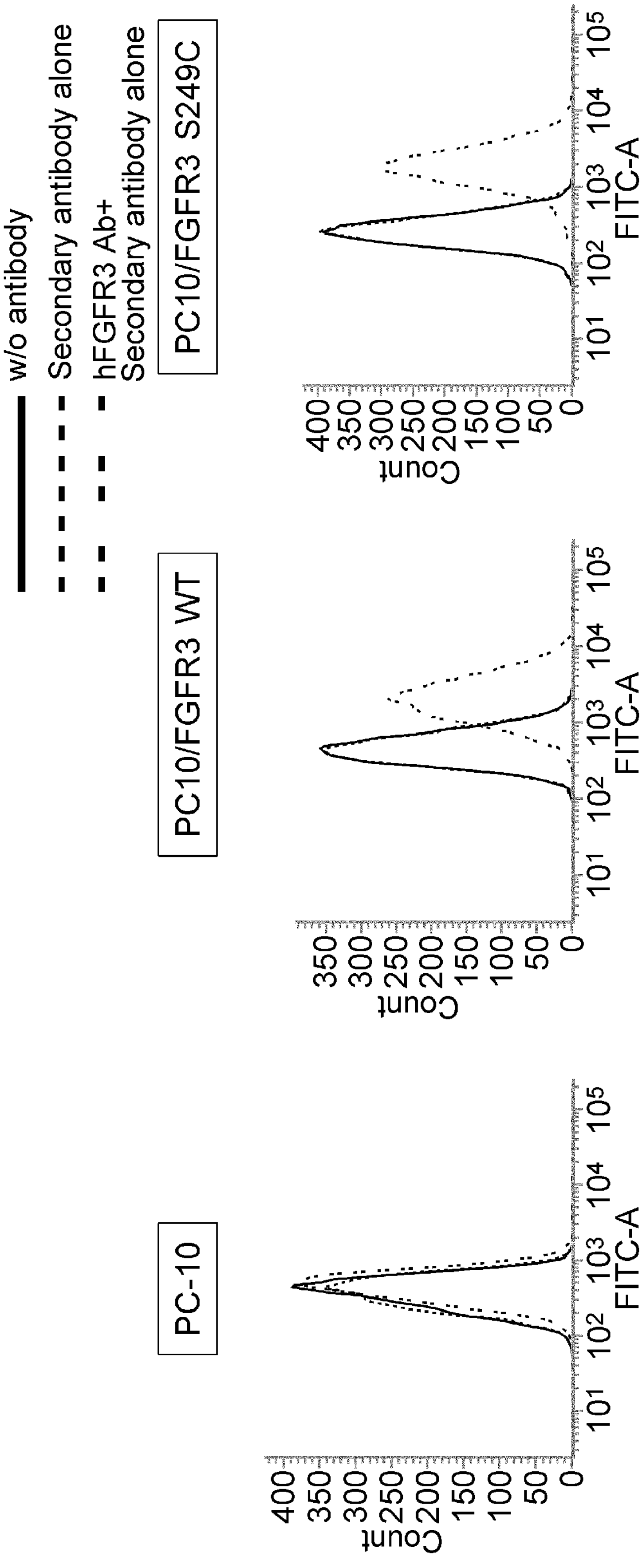
w/o antibody
Secondary antibody alone
hFGFR3 Ab+
Secondary antibody alone
PC-10
PC10/FGFR3 WT
PC10/FGFR3 S249C
Count
FITC-A

FIG. 3

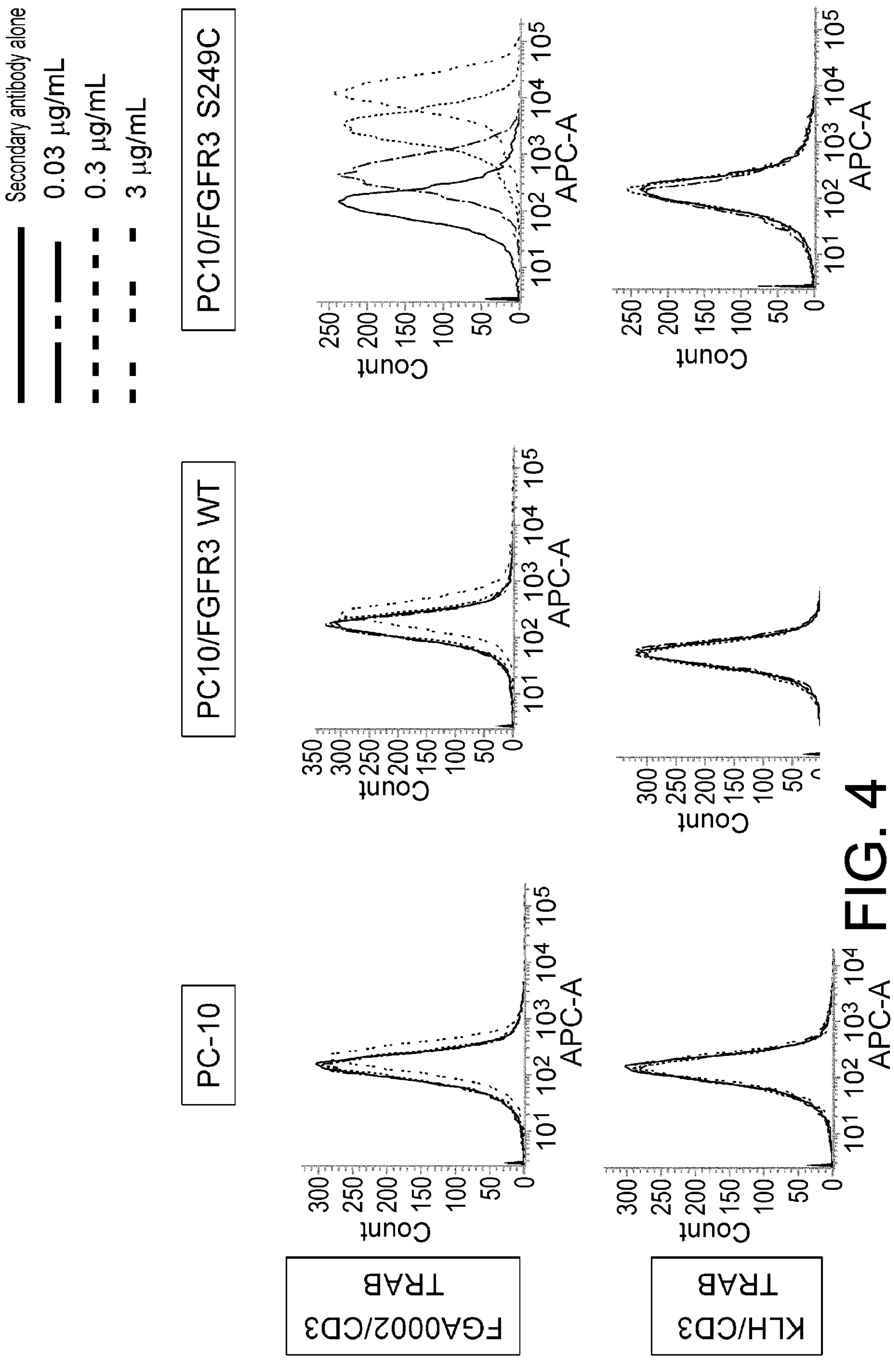

Secondary antibody alone
0.03 μg/mL
0.3 μg/mL
3 μg/mL
PC-10
PC10/FGFR3 WT
PC10/FGFR3 S249C
FGA0002/CD3 TRAB
KLH/CD3 TRAB
Count
APC-A


FIG. 4

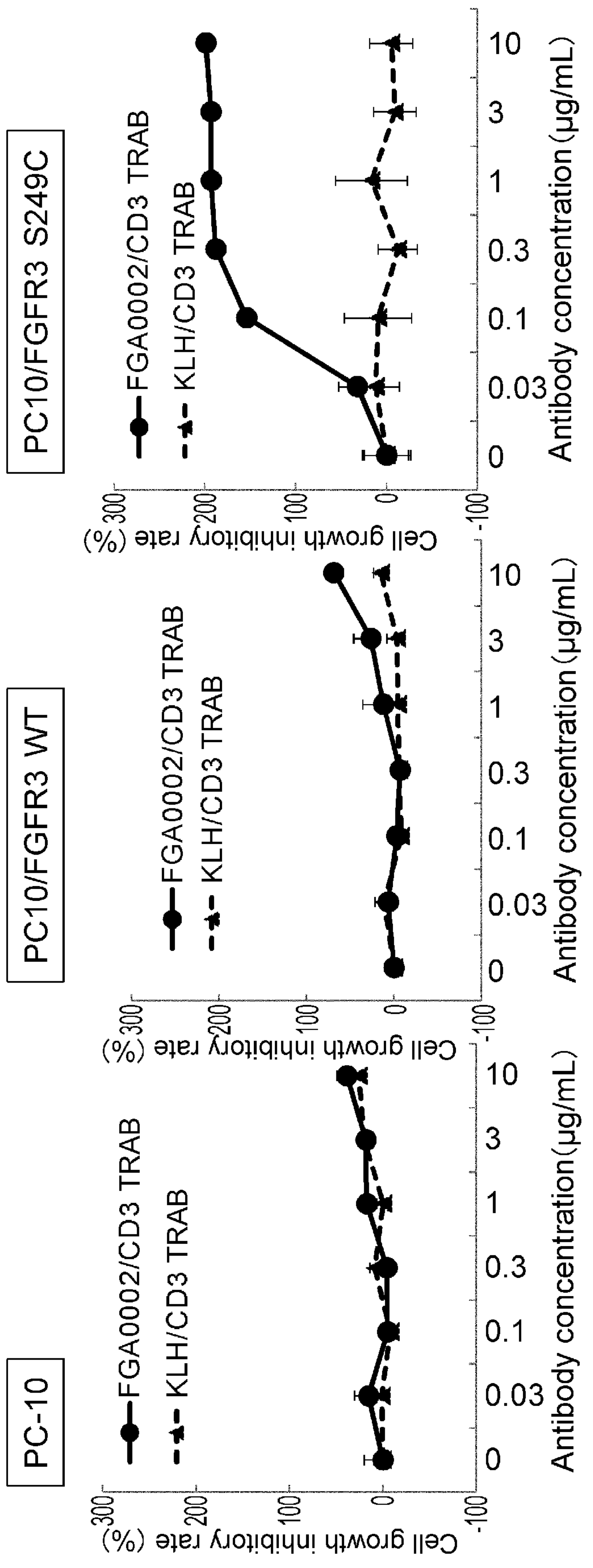
PC-10
FGA0002/CD3 TRAB
KLH/CD3 TRAB
Cell growth inhibitory rate (%)
300
200
100
0
-100
0
0.03
0.1
0.3
1
3
10
Antibody concentration (μg/mL)
PC10/FGFR3 WT
FGA0002/CD3 TRAB
KLH/CD3 TRAB
Cell growth inhibitory rate (%)
Antibody concentration (μg/mL)
PC10/FGFR3 S249C
FGA0002/CD3 TRAB
KLH/CD3 TRAB
Cell growth inhibitory rate (%)
Antibody concentration (μg/mL)

FIG. 5

## INTERNATIONAL SEARCH REPORT

International application No.
PCT/JP2020/027043

A. CLASSIFICATION OF SUBJECT MATTER
A61K 39/395(2006.01)i; A61P 35/00(2006.01)i; C07K 16/28(2006.01)i; C07K 16/46(2006.01)i; C12N 5/10(2006.01)i; C12N 1/15(2006.01)i; C12N 1/19(2006.01)i; C12N 1/21 (2006.01)i; C12N 15/13(2006.01)i; C12N 15/63(2006.01)i
FI: C12N15/13 ZNA; C12N5/10; C12N1/15; C12N1/19; C12N1/21; C07K16/28; A61K39/395 P; A61K39/395 N; A61P35/00; C12N15/63 Z; C07K16/46
According to International Patent Classification (IPC) or to both national classification and IPC

B. FIELDS SEARCHED
Minimum documentation searched (classification system followed by classification symbols)
A61K39/395; A61P35/00; C07K16/28; C07K16/46; C12N5/10; C12N1/15; C12N1/19; C12N1/21; C12N15/13; C12N15/63

Documentation searched other than minimum documentation to the extent that such documents are included in the fields searched

| | |
|---|---|
| Published examined utility model applications of Japan | 1922-1996 |
| Published unexamined utility model applications of Japan | 1971-2020 |
| Registered utility model specifications of Japan | 1996-2020 |
| Published registered utility model applications of Japan | 1994-2020 |

Electronic data base consulted during the international search (name of data base and, where practicable, search terms used)
JSTPlus/JMEDPlus/JST7580 (JDreamIII); CAplus/MEDLINE/EMBASE/BIOSIS/WPIDS (STN); UniProt/GeneSeq

C. DOCUMENTS CONSIDERED TO BE RELEVANT

| Category* | Citation of document, with indication, where appropriate, of the relevant passages | Relevant to claim No. |
|---|---|---|
| Y | WO 2018/191438 A1 (INHIBRX. INC.) 18.10.2018 (2018-10-18) paragraphs [0009], [0020], [0044] | 1-6, 8-13 |
| A | paragraphs [0009], [0020], [0044] | 7, 14-23 |
| Y | JP 2012-521759 A (GENENTECH, INC.) 20.09.2012 (2012-09-20) examples | 1-6, 8-13 |
| A | examples | 7, 14-23 |
| A | GORBENKO, Olena, et al., "Generation of Monoclonal Antibody Targeting fibroblast Growth factor Receptor 3", Hybridoma, vol. 28, no. 4, 10 August 2009, [retrieved on 27 August 2020], Retrieved from the Internet:<URL:https:www.liebertpub.com/doi/pdf/10..1089/hyb.2009.0018> abstract | 1-23 |

☐ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>27 August 2020 (27.08.2020) | Date of mailing of the international search report<br>08 September 2020 (08.09.2020) |
| Name and mailing address of the ISA/<br>Japan Patent Office<br>3-4-3, Kasumigaseki, Chiyoda-ku,<br>Tokyo 100-8915, Japan | Authorized officer<br><br>Telephone No. |

Form PCT/ISA/210 (second sheet) (January 2015)

**INTERNATIONAL SEARCH REPORT**
Information on patent family members

International application No.
PCT/JP2020/027043

| Patent Documents referred in the Report | Publication Date | Patent Family | Publication Date |
|---|---|---|---|
| WO 2018/191438 A1 | 18 Oct. 2018 | US 2019/0010242 A1<br>EP 3619235 A1<br>CA 3058477 A1<br>AU 2018250641 A1<br>SG 11201909160W A<br>KR 10-2020-0005732 A<br>CN 110770255 A<br>TW 201843177 A<br>IL 269826 D<br>JP 2020-516287 A | |
| JP 2012-521759 A | 20 Sep. 2012 | US 2010/0247531 A1<br>examples<br>WO 2010/111367 A1<br>EP 2411414 A1<br>AR 75939 A<br>CA 2754163 A1<br>TW 201038283 A<br>AU 2010229994 A1<br>SG 174904 A<br>KR 10-2011-0129976 A<br>IL 214433 D<br>CN 102378767 A<br>MX 2011009961 A<br>PE 5532012 A<br>MA 33208 B<br>CO 6450665 A<br>NZ 594339 A<br>RU 2011142905 A<br>CN 104 788564 A<br>DK 2411414 T<br>ES 2549769 T<br>HR P20150965 T<br>HK 1167666 A<br>SI 2411414 T<br>ZA 201105574 B<br>KR 10-2016-0135854 A<br>HU E025726 T<br>IL 250624 A<br>MX 345909 B<br>PE 20120553 A<br>AU 2013205318 A1 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2010111367 A
- WO 2016105503 A
- US 8410250 B
- WO 2009011941 A
- WO 5840837 A
- WO 6293194 A
- US 4816567 A
- WO 9607754 A
- WO 9209690 A
- US 5869046 A
- WO 9316185 A
- US 5571894 A
- US 5587458 A
- US 5641870 A
- WO 9306213 A
- WO 9308829 A
- WO 9404690 A
- US 4676980 A
- WO 9100360 A
- WO 9200373 A
- EP 03089 A
- US 6352694 B
- US 6534055 B
- US 6905680 B
- US 6692964 B
- US 5858358 A
- US 6887466 B
- US 6905681 B
- US 7144575 B
- US 7067318 B
- US 7172869 B
- US 7232566 B
- US 7175843 B
- US 5883223 A
- US 6905874 B
- US 6797514 B
- US 6867041 B
- US 20060121005
- US 20030157108 A, Presta, L.
- US 2004093621 A
- WO 03011878 A, Jean-Mairet
- US 6602684 B, Umana
- WO 9730087 A, Patel
- WO 9858964 A, Raju, S.
- WO 9922764 A, Raju, S.
- US 20050123546 A, Umana
- WO 200061739 A
- WO 200129246 A
- US 20030115614 A
- US 20020164328 A
- US 20040093621 A
- US 20040132140 A
- US 20040110704 A
- US 20040110282 A
- US 20040109865 A
- WO 2003085119 A
- WO 2003084570 A
- WO 2005035586 A
- WO 2005035778 A
- WO 2005053742 A
- WO 2004056312 A, Adams
- WO 9951642 A
- US 5648260 A
- US 5624821 A
- WO 9429351 A
- WO 0042072 A, Presta
- US 20050014934 A1, Hinton
- US 6194551 B1
- US 5500362 A
- US 5821337 A
- US 5648237 A, Carter
- US 5639635 A
- US 6083715 A, Georgiou
- US 6027888 A, Georgiou
- US 5264365 A, Georgio
- US 5508192 A, Georgiou
- US 4965199 A
- US 4419446 A
- US 46601978 B
- WO 9411026 A
- US 4767704 A
- US 4657866 A
- US 4927762 A
- US 4560655 A
- US 5122469 A
- WO 9003430 A
- WO 8700195 A
- US 30985 A
- US 4975278 A
- EP 1391213 A
- US 4970198 A
- US 5079233 A
- US 5585089 A
- US 5606040 A
- US 5693762 A
- US 5739116 A
- US 5767285 A
- US 5773001 A

- WO 9321232 A
- US 3896111 A
- US 4151042 A
- US 4137230 A
- US 4248870 A
- US 4256746 A
- US 4260608 A
- US 4265814 A
- US 4294757 A
- US 4307016 A
- US 4308268 A
- US 4308269 A
- US 4309428 A
- US 4313 A
- US 946 A
- US 4315929 A
- US 4317821 A
- US 4322348 A
- US 4331598 A
- US 4361650 A
- US 4364866 A
- US 4424219 A
- US 4450254 A
- US 4362663 A
- US 4371533 A
- US 5208020 A
- US 5416064 A
- EP 0425235 B1
- US 96060204
- US 5635483 A
- US 5780588 A
- US 5663149 A
- WO 02088772 A
- US 98334004
- US 5635843 A
- US 5712374 A
- US 5714586 A
- US 5770701 A
- US 5770710 A
- US 5877296 A
- US 5053394 A
- US 10983340 B
- US 5362852 A
- US 5869245 A
- US 4683203 A
- US 4683195 A
- EP 0332435 A
- WO 9607321 A
- WO 2008156083 A

**Non-patent literature cited in the description**


- **SAICHAEMCHAN et al.** *Current molecular medicine,* 2016, vol. 16, 40-62
- **KELLEHER et al.** *Carcinogenesis,* 2013, vol. 34, 2198-2205
- **HART et al.** *Molecular biology of the cell,* 2001, vol. 12, 931-942
- **D'AVIS et al.** *the molecular biology journal of the American Association for Cancer Research,* 1998, vol. 9, 71-78
- **DI MARTINO et al.** *Oncogene,* 03 December 2009, vol. 28 (48), 4306-4316
- **WANG et al.** *Oncotarget,* 2015, vol. 6, 34300-34308
- **SEIWERT et al.** *an official journal of the American Association for Cancer Research,* 2015, vol. 21, 632-641
- **ROSTY et al.** *Molecular cancer,* 2005, vol. 4, 15
- **HERNANDEZ et al.** *Mod Pathol,* 17 April 2009, vol. 22 (6), 848-56
- **QING et al.** *The Journal of clinical investigation,* 2009, vol. 119, 1216-1229
- *Oncotarget,* 2017, vol. 8 (9), 16052-74
- **GORBENKO.** *Hybridoma (Larchmt),* August 2009, vol. 28 (4), 295-300
- **KONTERMANN.** *mAbs,* 2012, vol. 4, 182-197
- **MEZZANZANICA et al.** *International journal of cancer,* 1988, vol. 41, 609-615
- **STAERZ ; BEVAN.** *Proceedings of the National Academy of Sciences of the United States of America,* 1986, vol. 83, 1453-1457
- **STAERZ et al.** *Nature,* 1985, vol. 314, 628-631
- **SINGLETON et al.** Dictionary of Microbiology and Molecular Biology. J. Wiley & Sons, 1994
- **MARCH.** Advanced Organic Chemistry Reactions, Mechanisms and Structure. John Wiley & Sons, 1992
- Epitope Mapping Protocols in Methods in Molecular Biology. 1996, vol. 66
- **JUNGHANS et al.** *Cancer Res,* 1990, vol. 50, 1495-1502
- **FLATMAN et al.** *J. Chromatogr. See B,* 2007, vol. 848, 79-87
- **KABAT et al.** Sequences of Proteins of Immunological Interest. 1991, vol. 1-3
- **KINDT et al.** Kuby Immunology. W.H. Freeman and Co, 2007, 91
- **PORTOLANO et al.** *J. Immunol.,* 1993, vol. 150, 880-887
- **CLARKSON et al.** *Nature,* 1991, vol. 352, 624-628
- **CHOTHIA ; LESK.** *J. Mol. Biol.,* 1987, vol. 196, 901-917
- **KABAT et al.** Sequences of Proteins of Immunological Interest. Public Health Service, National Institutes of Health, 1991

- **MACCALLUM et al.** *J. Mol. Biol.,* 1996, vol. 262, 732-745
- *Proc. Natl. Acad. Sci. USA,* 2006, vol. 103 (11), 4005-4010
- **DAVIS et al.** *Immunological Reviews,* 2002, vol. 190, 123-136
- *J. Rheumatol,* 2007, vol. 34, 11
- *Hum. Antibod. Hybridomas,* 1990, vol. 1 (1), 47-54
- *Blood,* 2007, vol. 109, 1185-1192
- **BRENTJENS et al.** *Molecular Therapy,* 2010, vol. 18 (4), 666-668
- **MORGAN et al.** *Molecular Therapy,* 23 February 2010, 1-9
- **TILL et al.** *Blood,* 2008, vol. 112, 2261-2271
- **GODING.** Monoclonal Antibodies: Principles and Practice. Academic Press, 1986, 59-103
- **KOZBOR.** *J. Immunol.,* 1984, vol. 133, 3001
- **BRODEUR et al.** Monoclonal Antibody Production Techniques and Applications. Marcel Dekker, Inc, 1987, 51-63
- **MUNSON et al.** *Anal. Biochem.,* 1980, vol. 107, 220
- **WINTER et al.** *Ann. Rev. Immunol.,* 1994, vol. 12, 433-455
- **GRIFFITHS et al.** *EMBO J,* 1993, vol. 12, 725-734
- **HOOGENBOOM ; WINTER, J. et al.** *Mol. Biol.,* 1992, vol. 227, 381-388
- **MARKS et al.** *J. Mol. Biol.,* 1991, vol. 222, 581-597
- **HOOGENBOOM et al.** *Nucl. Acids. Res.,* 1991, vol. 19, 4133-4137
- **ORLANDI et al.** *Proc. Natl. Acad. Sci. (USA),* 1989, vol. 86, 3833-3837
- **WARD et al.** *Nature,* 1979, vol. 341, 544-546
- **JONES et al.** *Biotechnol.,* 1991, vol. 9, 88-89
- **SASTRY et al.** *Proc. Natl. Acad. Sci. (USA),* 1989, vol. 86, 5728-5732
- **ORUM et al.** *Nucleic Acids Res.,* 1993, vol. 21, 4491-4498
- **CLACKSON et al.** *Nature,* 1991, vol. 352, 624-628
- **TOMLINSON et al.** *J. Mol. Biol.,* 1992, vol. 227, 776-798
- **MATSUDA et al.** *Nature Genet.,* 1993, vol. 3, 88-94
- **HOOGENBOOM ; WINTER.** *J. Mol. Biol.,* 1992, vol. 227, 381-388
- **BARBAS et al.** *Proc. Natl. Acad. Sci. USA,* 1992, vol. 89, 4457-4461
- **WILLIAMS ; WINTER.** *Eur. J. Immunol.,* 1993, vol. 23, 1456-1461
- **HOGREFE et al.** *Gene,* 1993, vol. 128, 119-126
- **WATERHOUSE.** *Nucl. Acids Res.,* 1993, vol. 21, 2265-2266
- **BARBAS et al.** *Proc. Natl. Acad. Sci. USA,* 1991, vol. 88, 7978-7982
- **EMBLETON et al.** *Nucl. Acids Res.,* 1992, vol. 20, 3831-3837
- **HAWKINS et al.** *J. Mol. Biol.,* 1992, vol. 226, 889-896
- **GRAM et al.** *Proc. Natl. Acad. Sci USA,* 1992, vol. 89, 3576-3580
- **LEUNG et al.** *Technique,* 1989, vol. 1, 11-15
- **MARKS et al.** *Biotechnol,* 1992, vol. 10, 779-783
- **ENGELS et al.** *Agnew. Chem. Int. Ed. Engl.,* 1989, vol. 28, 716-734
- **ABRAHMSEN et al.** *EMBO J.,* 1985, vol. 4, 3901
- *Methods in Enzymology,* 1976, vol. 44
- **BASS et al.** *Proteins,* 1990, vol. 8, 309-314
- **MARKS et al.** *Biotechnol.,* 1992, vol. 10, 779-783
- **SKERRA et al.** *Curr. Opinion in Immunol.,* 1993, vol. 5, 256-262
- **PLUCKTHUN.** *Immunol. Revs.,* 1992, vol. 130, 151-188
- **MORRISON et al.** *Proc. Nat. Acad. Sci. USA,* 1984, vol. 81, 6851
- **MORIMOTO et al.** *Journal of Biochemical and Biophysical Methods,* 1992, vol. 24, 107-117
- **BRENNAN et al.** *Science,* 1985, vol. 229, 81
- **CARTER et al.** *Bio/Technology,* 1992, vol. 10, 163-167
- **JONES et al.** *Nature,* 1986, vol. 321, 522-525
- **RIECHMANN et al.** *Nature,* 1988, vol. 332, 323-327
- **VERHOEYEN et al.** *Science,* 1988, vol. 239, 1534-1536
- **SIMS et al.** *J. Immunol.,* 1993, vol. 151, 2296
- **CHOTHIA et al.** *J. Mol. Biol.,* 1987, vol. 196, 901
- **CARTER et al.** *Proc. Natl. Acad. Sci., USA,* 1992, vol. 89, 4285
- **PRESTA et al.** *J. Immunol.,* 1993, vol. 151, 2623
- **KOZBOR.** *J., Immunol.,* 1984, vol. 133, 3001
- **BOERNER et al.** *J. Immunol.,* 1991, vol. 147, 86
- **JAKOBOVITS et al.** *Proc. Natl. Acad. Sci., USA,* 1993, vol. 90, 2551-255
- **JAKOBOVITS et al.** *Nature,* 1993, vol. 362, 255-258
- *Proc. Natl. Acad. Sci., USA,* 1980, vol. 77, 4914-4917
- **MILSTEIN et al.** *Nature,* 1983, vol. 305, 537-539
- **TRAUNECKER et al.** *EMBO J,* 1991, vol. 10, 3655-3459

- **SURESH et al.** *Methods in Enzymology,* 1986, vol. 121, 210
- **BERG et al.** *Transplant Proc,* 1998, vol. 30 (8), 3975-3977
- **HAANEN et al.** *J. Exp. Med.,* 1999, vol. 190 (9), 13191328
- **GARLAND et al.** *J. Immunol Meth.,* 1999, vol. 227 (1-2), 53-63
- **SHALABY et al.** *J.M. Exp. Med.,* 1992, vol. 175, 217-225
- **KOSTELNY et al.** *J. Immunol.,* 1992, vol. 148 (5), 1547-1553
- **HOLLINGER et al.** *Proc. Natl. Acad. Sci. USA,* 1993, vol. 90, 6444-6448
- **GRUBER et al.** *J. Immunol.,* 1994, vol. 152, 5368
- **TUTT et al.** *J. Immunol.,* 1991, vol. 147, 60
- *Science,* 1989, vol. 244, 1081-1085
- **OKAZAKI et al.** *Mol. Biol.,* 2004, vol. 336, 1239-1249
- **RIPKA et al.** *Arch. Biochem. Biophys.,* 1986, vol. 249, 533-545
- **YAMANE-OHNUKI et al.** *Biotech. Bioeng.,* 2004, vol. 87, 614
- **DUNCAN ; WINTER.** *Nature,* 1988, vol. 322, 738-40
- **SHIELDS et al.** *J. Biol. Chem.,* 2001, vol. 9 (2), 6591-6604
- **GUYER et al.** *J. Immunol.,* 1976, vol. 117, 587
- **KIM et al.** *J. Immunol.,* 1994, vol. 24, 249
- **IDUSOGIE et al.** *J. Immunol.,* 2000, vol. 164, 4178-4184
- **RAVETCH ; KINET.** *Annu. Rev. Immunol,* 1991, vol. 9, 457-92
- **CLYNES et al.** *PNAS (USA),* 1998, vol. 95, 652-656
- **GAZZANO-SANTORO et al.** *J. Immunol. Methods,* 1996, vol. 202, 163
- **SIEBENLIST et al.** *Cell,* 1980, vol. 20, 269
- **PROBA ; PLUCKTHUN.** *Gene,* 1995, vol. 159, 203
- **BACHMANN.** Cellular and Molecular Biology. American Society for Microbiology, 1987, vol. 2, 1190-1219
- **SIMMONS et al.** *J. Immunol Methods,* 2002, vol. 263, 133-147
- **CHEN et al.** *J Bio Chem,* 1999, vol. 274, 19601-1605
- **BOTHMANN ; PLUCKTHUN et al.** *Biol. Chem.,* 2000, vol. 275, 17100-17105
- **RAMM ; PLUCKTHUN et al.** *Biol. Chem.,* 2000, vol. 275, 17106-17113
- **ARIE et al.** *Mol. Microbiol.,* 2001, vol. 39, 199-210
- **HARA et al.** *Microbial Drug Resistance,* 1996, vol. 2, 63-72
- **LINDMARK et al.** *J. Immunol., Meth.,* 1983, vol. 62, 1-13
- **YANIV.** *Nature,* 1982, vol. 297, 17-18
- **GRAHAM et al.** *J. Gen Virol.,* 1977, vol. 36, 59
- **URLAUB et al.** *Proc. Natl. Acad. Sci., USA,* 1980, vol. 77, 4216
- **MATHER.** *Biol. Reprod.,* 1980, vol. 23, 243-251
- **MATHER et al.** *Annals N.Y. Acad. Sci.,* 1982, vol. 383, 44-68
- **HAM et al.** *Meth. Enz.,* 1979, vol. 58, 44
- **BARNES et al.** *Anal. Biochem.,* 1980, vol. 102, 255
- **LINDMARK et al.** *J. Immunol Meth,* 1983, vol. 62, 1-13
- **GUSS et al.** *EMBO J,* 1986, vol. 5
- **SYRIGOS ; EPENETHOS.** *Anticancer Research,* 1999, vol. 19, 605-614
- **NICULESCU-DUVAZ ; SPRINGER.** *Adv. Drg Del. Rev.,* 1997, vol. 26, 151-172
- **BALDWIN et al.** *Lancet,* 15 March 1986, 603-05
- Antibody Carriers Of Cytotoxic Agents In Cancer Therapy: A Review. **THORPE et al.** Monoclonal Antibodies ’84: Biological And Clinical Applications. 1985, 475-506
- **ROWLAND et al.** *Cancer Immunol. Immunother.,* 1986, vol. 21, 183-87
- **MANDLER et al.** *Jour. of the Nat. Cancer Inst.,* 2000, vol. 92 (19), 1573-1581
- **MANDLER et al.** *Bioorganic & Med. Chem. Letters,* 2000, vol. 10, 1025-1028
- **MANDLER et al.** *Bioconjugate Chem,* 2002, vol. 13, 786-791
- **LIU et al.** *Proc. Natl. Acad. Sci. USA,* 1996, vol. 93, 8618-8623
- **LODE et al.** *Cancer Res,* 1998, vol. 58, 2928
- **HINMAN et al.** *Cancer Res,* 1993, vol. 53, 3336-3342
- **WISEMAN et al.** *Eur. Jour. Nucl. Med.,* 2000, vol. 27 (7), 766-77
- **WISEMAN et al.** *Blood,* 2002, vol. 99 (12), 4336-42
- **WITZIG et al.** *J. Clin. Oncol.,* 2002, vol. 20 (10), 2453-63
- **WITZIG et al.** *J. Clin. Oncol.,* 2002, vol. 20 (15), 3262-69
- *Drugs of the Future,* 2000, vol. 25 (7), 686
- **DORONINA et al.** *Nature Biotechnology,* 2003, vol. 21 (7), 778-784
- **VITETTA et al.** *Science,* 1987, vol. 238, 1098
- **LIU et al.** *Proc. Natl. Acad. Sci.,* 1996, vol. 93, 8618-8623
- **CHARI et al.** *Cancer Research,* 1992, vol. 52, 127-131
- **CARLSSON et al.** *Biochem. J.,* 1978, vol. 173, 723-737
- **WOYKE et al.** *Antimicrob. Agents and Chemother.,* 2001, vol. 45 (12), 3580-3584

- **PETTIT et al.** *Antimicrob. Agents Chemother.,* 1998, vol. 42, 2961-2965
- **E. SCHRODER ; K. LUBKE.** The Peptides. Academic Press, 1965, vol. 1, 76-136
- **PETTIT et al.** *J. Am. Chem. Soc.,* 1989, vol. 111, 5463-5465
- **PETTIT et al.** *Anti-Cancer Drug Design,* 1998, vol. 13, 243-277
- **PETTIT, G. R. et al.** *Synthesis,* 1996, 719-725
- **PETTIT et al.** *J. Chem. Soc. Perkin Trans.,* 1996, vol. 1 (5), 859-863
- **DORONINA.** *Nat Biotechnol,* 2003, vol. 21 (7), 778-784
- **HINMAN et al.** *Cancer Research,* 1993, vol. 53, 3336-3342
- **LODE et al.** *Cancer Research,* 1998, vol. 58, 2925-2928
- **FRAKER et al.** *Biochem. Biophys. Res. Commun.,* 1978, vol. 80, 49-57
- **CHATAL.** Monoclonal Antibodies in Immunoscintigraphy. CRC Press, 1989
- Applications Handbook and Catalog. 2003, 467-498
- **GEOGHEGAN ; STROH.** *Bioconjugate Chem,* 1992, vol. 3, 138-146
- **GODFREY et al.** *J. Molec. Diagnostics,* 2000, vol. 2, 84-91
- **SPECHT et al.** *Am. J. Pathol.,* 2001, vol. 158, 419-29
- **DEL TITO et al.** *Clinical Chemistry,* 1998, vol. 44, 731-739
- **SAIKI et al.** *Science,* 1988, vol. 239, 487
- **WU et al.** *Genomics,* 1989, vol. 4, 560-569
- **RUANO ; KIDD.** *Nucleic Acids Research,* 1989, vol. 17, 8392
- **NEWTON et al.** *Nucleic Acids Research,* 1989, vol. 17, 7
- **ORITA et al.** *Proc. Natl. Acad. Sci., USA,* 1989, vol. 86, 2766-2770
- *Genomics,* 1989, vol. 5, 874-879
- **WINTER et al.** *Proc. Natl. Acad. Sci., USA,* 1985, vol. 82, 7575
- **MEYERS et al.** *Science,* 1985, vol. 230, 1242
- **COTTON et al.** *Proc. Natl. Acad. Sci., USA,* 1988, vol. 85, 4397
- **SHENK et al.** *Proc. Natl. Acad. Sci., USA,* 1975, vol. 72, 989
- **CARIELLO.** *Human Genetics,* 1988, vol. 42, 726
- **NOVACK et al.** *Proc. Natl. Acad. Sci., USA,* 1986, vol. 83, 586
- Remington's Pharmaceutical Sciences. Lippincott, Williams & Wilkins, 2000
- *Protein Science,* 1995, vol. 4, 2411-2423

SEQUENCE LISTING

<110> CHUGAI SEIYAKU KABUSHIKI KAISHA

<120> Anti mutated-FGFR3 antibodies and uses thereof

<130> C1-A1811P

<150> JP 2019-130236
<151> 2019-07-12

<160> 69

<170> PatentIn version 3.5

<210> 1
<211> 808
<212> PRT
<213> Homo Sapiens

<400> 1

```
Met Gly Ala Pro Ala Cys Ala Leu Ala Leu Cys Val Ala Val Ala Ile
1               5                   10                  15

Val Ala Gly Ala Ser Ser Glu Ser Leu Gly Thr Glu Gln Arg Val Val
            20                  25                  30

Gly Arg Ala Ala Glu Val Pro Gly Pro Glu Pro Gly Gln Gln Glu Gln
        35                  40                  45

Leu Val Phe Gly Ser Gly Asp Ala Val Glu Leu Ser Cys Pro Pro Pro
    50                  55                  60

Gly Gly Gly Pro Met Gly Pro Thr Val Trp Val Lys Asp Gly Thr Gly
65                  70                  75                  80

Leu Val Pro Ser Glu Arg Val Leu Val Gly Pro Gln Arg Leu Gln Val
                85                  90                  95

Leu Asn Ala Ser His Glu Asp Ser Gly Ala Tyr Ser Cys Arg Gln Arg
            100                 105                 110

Leu Thr Gln Arg Val Leu Cys His Phe Ser Val Arg Val Thr Asp Ala
        115                 120                 125

Pro Ser Ser Gly Asp Asp Glu Asp Gly Glu Asp Glu Ala Glu Asp Thr
    130                 135                 140

Gly Val Asp Thr Gly Ala Pro Tyr Trp Thr Arg Pro Glu Arg Met Asp
145                 150                 155                 160

Lys Lys Leu Leu Ala Val Pro Ala Ala Asn Thr Val Arg Phe Arg Cys
```

165 170 175

Pro Ala Ala Gly Asn Pro Thr Pro Ser Ile Ser Trp Leu Lys Asn Gly
180 185 190

Arg Glu Phe Arg Gly Glu His Arg Ile Gly Gly Ile Lys Leu Arg His
195 200 205

Gln Gln Trp Ser Leu Val Met Glu Ser Val Val Pro Ser Asp Arg Gly
210 215 220

Asn Tyr Thr Cys Val Val Glu Asn Lys Phe Gly Ser Ile Arg Gln Thr
225 230 235 240

Tyr Thr Leu Asp Val Leu Glu Arg Ser Pro His Arg Pro Ile Leu Gln
245 250 255

Ala Gly Leu Pro Ala Asn Gln Thr Ala Val Leu Gly Ser Asp Val Glu
260 265 270

Phe His Cys Lys Val Tyr Ser Asp Ala Gln Pro His Ile Gln Trp Leu
275 280 285

Lys His Val Glu Val Asn Gly Ser Lys Val Gly Pro Asp Gly Thr Pro
290 295 300

Tyr Val Thr Val Leu Lys Ser Trp Ile Ser Glu Ser Val Glu Ala Asp
305 310 315 320

Val Arg Leu Arg Leu Ala Asn Val Ser Glu Arg Asp Gly Gly Glu Tyr
325 330 335

Leu Cys Arg Ala Thr Asn Phe Ile Gly Val Ala Glu Lys Ala Phe Trp
340 345 350

Leu Ser Val His Gly Pro Arg Ala Ala Glu Glu Glu Leu Val Glu Ala
355 360 365

Asp Glu Ala Gly Ser Val Tyr Ala Gly Ile Leu Ser Tyr Gly Val Gly
370 375 380

Phe Phe Leu Phe Ile Leu Val Val Ala Ala Val Thr Leu Cys Arg Leu
385 390 395 400

Arg Ser Pro Pro Lys Lys Gly Leu Gly Ser Pro Thr Val His Lys Ile
405 410 415

Ser Arg Phe Pro Leu Lys Arg Gln Val Ser Leu Glu Ser Asn Ala Ser
420 425 430

Met Ser Ser Asn Thr Pro Leu Val Arg Ile Ala Arg Leu Ser Ser Gly
435 440 445

Glu Gly Pro Thr Leu Ala Asn Val Ser Glu Leu Glu Leu Pro Ala Asp
450 455 460

Pro Lys Trp Glu Leu Ser Arg Ala Arg Leu Thr Leu Gly Lys Pro Leu
465 470 475 480

Gly Glu Gly Cys Phe Gly Gln Val Val Met Ala Glu Ala Ile Gly Ile
485 490 495

Asp Lys Asp Arg Ala Ala Lys Pro Val Thr Val Ala Val Lys Met Leu
500 505 510

Lys Asp Asp Ala Thr Asp Lys Asp Leu Ser Asp Leu Val Ser Glu Met
515 520 525

Glu Met Met Lys Met Ile Gly Lys His Lys Asn Ile Ile Asn Leu Leu
530 535 540

Gly Ala Cys Thr Gln Gly Gly Pro Leu Tyr Val Leu Val Glu Tyr Ala
545 550 555 560

Ala Lys Gly Asn Leu Arg Glu Phe Leu Arg Ala Arg Arg Pro Pro Gly
565 570 575

Leu Asp Tyr Ser Phe Asp Thr Cys Lys Pro Pro Glu Glu Gln Leu Thr
580 585 590

Phe Lys Asp Leu Val Ser Cys Ala Tyr Gln Val Ala Arg Gly Met Glu
595 600 605

Tyr Leu Ala Ser Gln Lys Cys Ile His Arg Asp Leu Ala Ala Arg Asn
610 615 620

Val Leu Val Thr Glu Asp Asn Val Met Lys Ile Ala Asp Phe Gly Leu
625 630 635 640

Ala Arg Asp Val His Asn Leu Asp Tyr Tyr Lys Lys Thr Thr Asn Gly
645 650 655

Arg Leu Pro Val Lys Trp Met Ala Pro Glu Ala Leu Phe Asp Arg Val
660 665 670

Tyr Thr His Gln Ser Asp Val Trp Ser Phe Gly Val Leu Leu Trp Glu
675 680 685

Ile Phe Thr Leu Gly Gly Ser Pro Tyr Pro Gly Ile Pro Val Glu Glu
690 695 700

Leu Phe Lys Leu Leu Lys Glu Gly His Arg Met Asp Lys Pro Ala Asn
705 710 715 720

Cys Thr His Asp Leu Tyr Met Ile Met Arg Glu Cys Trp His Ala Ala
725 730 735

Pro Ser Gln Arg Pro Thr Phe Lys Gln Leu Val Glu Asp Leu Asp Arg
740 745 750

Val Leu Thr Val Thr Ser Thr Asp Glu Tyr Leu Asp Leu Ser Ala Pro
755 760 765

Phe Glu Gln Tyr Ser Pro Gly Gly Gln Asp Thr Pro Ser Ser Ser Ser
770 775 780

Ser Gly Asp Asp Ser Val Phe Ala His Asp Leu Leu Pro Pro Ala Pro
785 790 795 800

Pro Ser Ser Gly Gly Ser Arg Thr
805

<210> 2
<211> 2424
<212> DNA
<213> Homo Sapiens

<400> 2
atgggggctc ctgcctgcgc tctggctctg tgcgtggccg tcgctatcgt cgccggggct 60

tcctccgaaa gtctggggac cgaacagagg gtggtgggaa gggcagcaga ggtgccagga 120

cctgagccag gccagcagga gcagctggtg ttcggaagcg gcgacgccgt ggagctgtcc 180

tgcccacctc caggaggagg acctatggga ccaacagtgt gggtgaagga tggaaccgga 240

ctggtgccat ccgagcgggt gctggtggga cctcagagac tgcaggtgct gaacgcaagc 300

cacgaggact ccggagcata ctcttgcagg cagcgcctga cacagcgggt gctgtgccac 360

tttagcgtga gagtgaccga tgcaccaagc tccggcgacg atgaggacgg agaggatgag 420

gcagaggaca caggagtgga taccggagca ccatattgga ccaggccaga gagaatggac 480

aagaagctgc tggccgtgcc tgccgccaat acagtgcggt tcagatgtcc agcagcagga 540

aacccaaccc cttctatcag ctggctgaag aatggcaggg agtttcgcgg cgagcacagg 600

```
atcggcggca tcaagctgcg ccaccagcag tggtccctgg tcatggagag cgtggtgcca      660
tccgaccggg gcaactacac atgcgtggtg gagaataagt tcggctccat cagacagacc      720
tatacactgg atgtgctgga gcggtctccc cacagaccta tcctgcaggc aggactgcct      780
gcaaaccaga ccgccgtgct gggatctgac gtggagtttc actgtaaggt gtacagcgat      840
gcccagccac acatccagtg gctgaagcac gtggaagtga atggctccaa agtgggccct      900
gacggcacac catatgtgac cgtgctgaag tcttggatct ccgagtctgt ggaggcagac      960
gtgaggctga ggctggcaaa cgtgtctgag agggatggag gagagtacct gtgcagggcc     1020
accaatttca tcggcgtggc cgagaaggcc ttttggctga gcgtgcacgg acctagggca     1080
gcagaggagg agctggtgga ggcagatgag gcaggaagcg tgtacgcagg catcctgagc     1140
tatggcgtgg gcttctttct gttcatcctg gtggtggcag cagtgaccct gtgcaggctg     1200
agaagccccc ctaagaaggg cctgggctcc ccaaccgtgc acaagatctc tcggtttccc     1260
ctgaagagac aggtgagcct ggagtccaac gcctctatgt ctagcaatac acccctggtg     1320
cggatcgcca gactgtcctc tggagaggga cctaccctgg caaacgtgag cgagctggag     1380
ctgccagcag acccaaagtg ggagctgtcc agggcaaggc tgacactggg caagccactg     1440
ggagagggat gcttcggaca ggtggtcatg gcagaggcca tcggcatcga caaggatagg     1500
gcagcaaagc ctgtgacagt ggcagtgaag atgctgaagg acgatgccac cgacaaggat     1560
ctgtctgatc tggtgagcga gatggagatg atgaagatga tcggcaagca caagaacatc     1620
atcaatctgc tgggagcatg tacccaggga ggaccactgt acgtgctggt ggagtatgcc     1680
gccaagggca atctgaggga gtttctgagg gcacggagac cacccggact ggactactcc     1740
ttcgatacat gcaagcctcc agaggagcag ctgaccttta aggacctggt gtcttgtgcc     1800
taccaggtgg ccaggggcat ggagtatctg gccagccaga agtgcatcca cagggatctg     1860
gccgcccgca acgtgctggt gacagaggac aatgtgatga agatcgcaga tttcggactg     1920
gcaagggacg tgcacaacct ggattactat aagaagacca caaatggcag gctgcctgtg     1980
aagtggatgg ccccagaggc cctgtttgac cgcgtgtaca cacaccagtc cgacgtgtgg     2040
tctttcggcg tgctgctgtg ggagatcttt accctgggag gaagcccata tccaggcatc     2100
ccagtggagg agctgttcaa gctgctgaag gagggccacc ggatggacaa gcccgccaac     2160
tgcacacacg atctgtatat gatcatgagg gagtgttggc acgcagcacc aagccagaga     2220
cctaccttca agcagctggt ggaggacctg gatagagtgc tgaccgtgac atccaccgac     2280
gagtacctgg atctgagcgc cccctttgag cagtattccc ctggaggaca ggacacccca     2340
agctcctcta gctccggcga cgattcagtg tttgctcacg acctgctgcc acctgctcct     2400
ccctcatctg gggctcacg gact                                            2424
```

<210> 3
<211> 808
<212> PRT
<213> Homo Sapiens

<400> 3

```
Met Gly Ala Pro Ala Cys Ala Leu Ala Leu Cys Val Ala Val Ala Ile
1               5                   10                  15

Val Ala Gly Ala Ser Ser Glu Ser Leu Gly Thr Glu Gln Arg Val Val
            20                  25                  30

Gly Arg Ala Ala Glu Val Pro Gly Pro Glu Pro Gly Gln Gln Glu Gln
        35                  40                  45

Leu Val Phe Gly Ser Gly Asp Ala Val Glu Leu Ser Cys Pro Pro Pro
    50                  55                  60

Gly Gly Gly Pro Met Gly Pro Thr Val Trp Val Lys Asp Gly Thr Gly
65                  70                  75                  80

Leu Val Pro Ser Glu Arg Val Leu Val Gly Pro Gln Arg Leu Gln Val
                85                  90                  95

Leu Asn Ala Ser His Glu Asp Ser Gly Ala Tyr Ser Cys Arg Gln Arg
            100                 105                 110

Leu Thr Gln Arg Val Leu Cys His Phe Ser Val Arg Val Thr Asp Ala
        115                 120                 125

Pro Ser Ser Gly Asp Asp Glu Asp Gly Glu Asp Glu Ala Glu Asp Thr
    130                 135                 140

Gly Val Asp Thr Gly Ala Pro Tyr Trp Thr Arg Pro Glu Arg Met Asp
145                 150                 155                 160

Lys Lys Leu Leu Ala Val Pro Ala Ala Asn Thr Val Arg Phe Arg Cys
                165                 170                 175

Pro Ala Ala Gly Asn Pro Thr Pro Ser Ile Ser Trp Leu Lys Asn Gly
            180                 185                 190

Arg Glu Phe Arg Gly Glu His Arg Ile Gly Gly Ile Lys Leu Arg His
        195                 200                 205

Gln Gln Trp Ser Leu Val Met Glu Ser Val Val Pro Ser Asp Arg Gly
    210                 215                 220
```

```
Asn Tyr Thr Cys Val Val Glu Asn Lys Phe Gly Ser Ile Arg Gln Thr
225                 230                 235                 240

Tyr Thr Leu Asp Val Leu Glu Arg Cys Pro His Arg Pro Ile Leu Gln
                245                 250                 255

Ala Gly Leu Pro Ala Asn Gln Thr Ala Val Leu Gly Ser Asp Val Glu
            260                 265                 270

Phe His Cys Lys Val Tyr Ser Asp Ala Gln Pro His Ile Gln Trp Leu
        275                 280                 285

Lys His Val Glu Val Asn Gly Ser Lys Val Gly Pro Asp Gly Thr Pro
    290                 295                 300

Tyr Val Thr Val Leu Lys Ser Trp Ile Ser Glu Ser Val Glu Ala Asp
305                 310                 315                 320

Val Arg Leu Arg Leu Ala Asn Val Ser Glu Arg Asp Gly Gly Glu Tyr
                325                 330                 335

Leu Cys Arg Ala Thr Asn Phe Ile Gly Val Ala Glu Lys Ala Phe Trp
            340                 345                 350

Leu Ser Val His Gly Pro Arg Ala Ala Glu Glu Glu Leu Val Glu Ala
        355                 360                 365

Asp Glu Ala Gly Ser Val Tyr Ala Gly Ile Leu Ser Tyr Gly Val Gly
    370                 375                 380

Phe Phe Leu Phe Ile Leu Val Val Ala Ala Val Thr Leu Cys Arg Leu
385                 390                 395                 400

Arg Ser Pro Pro Lys Lys Gly Leu Gly Ser Pro Thr Val His Lys Ile
                405                 410                 415

Ser Arg Phe Pro Leu Lys Arg Gln Val Ser Leu Glu Ser Asn Ala Ser
            420                 425                 430

Met Ser Ser Asn Thr Pro Leu Val Arg Ile Ala Arg Leu Ser Ser Gly
        435                 440                 445

Glu Gly Pro Thr Leu Ala Asn Val Ser Glu Leu Glu Leu Pro Ala Asp
    450                 455                 460

Pro Lys Trp Glu Leu Ser Arg Ala Arg Leu Thr Leu Gly Lys Pro Leu
465                 470                 475                 480
```

Gly Glu Gly Cys Phe Gly Gln Val Val Met Ala Glu Ala Ile Gly Ile
485 490 495

Asp Lys Asp Arg Ala Ala Lys Pro Val Thr Val Ala Val Lys Met Leu
500 505 510

Lys Asp Asp Ala Thr Asp Lys Asp Leu Ser Asp Leu Val Ser Glu Met
515 520 525

Glu Met Met Lys Met Ile Gly Lys His Lys Asn Ile Ile Asn Leu Leu
530 535 540

Gly Ala Cys Thr Gln Gly Gly Pro Leu Tyr Val Leu Val Glu Tyr Ala
545 550 555 560

Ala Lys Gly Asn Leu Arg Glu Phe Leu Arg Ala Arg Arg Pro Pro Gly
565 570 575

Leu Asp Tyr Ser Phe Asp Thr Cys Lys Pro Pro Glu Glu Gln Leu Thr
580 585 590

Phe Lys Asp Leu Val Ser Cys Ala Tyr Gln Val Ala Arg Gly Met Glu
595 600 605

Tyr Leu Ala Ser Gln Lys Cys Ile His Arg Asp Leu Ala Ala Arg Asn
610 615 620

Val Leu Val Thr Glu Asp Asn Val Met Lys Ile Ala Asp Phe Gly Leu
625 630 635 640

Ala Arg Asp Val His Asn Leu Asp Tyr Tyr Lys Lys Thr Thr Asn Gly
645 650 655

Arg Leu Pro Val Lys Trp Met Ala Pro Glu Ala Leu Phe Asp Arg Val
660 665 670

Tyr Thr His Gln Ser Asp Val Trp Ser Phe Gly Val Leu Leu Trp Glu
675 680 685

Ile Phe Thr Leu Gly Gly Ser Pro Tyr Pro Gly Ile Pro Val Glu Glu
690 695 700

Leu Phe Lys Leu Leu Lys Glu Gly His Arg Met Asp Lys Pro Ala Asn
705 710 715 720

Cys Thr His Asp Leu Tyr Met Ile Met Arg Glu Cys Trp His Ala Ala
725 730 735

Pro Ser Gln Arg Pro Thr Phe Lys Gln Leu Val Glu Asp Leu Asp Arg
740 745 750

Val Leu Thr Val Thr Ser Thr Asp Glu Tyr Leu Asp Leu Ser Ala Pro
755 760 765

Phe Glu Gln Tyr Ser Pro Gly Gly Gln Asp Thr Pro Ser Ser Ser Ser
770 775 780

Ser Gly Asp Asp Ser Val Phe Ala His Asp Leu Leu Pro Pro Ala Pro
785 790 795 800

Pro Ser Ser Gly Gly Ser Arg Thr
805

<210> 4
<211> 2424
<212> DNA
<213> Homo Sapiens

<400> 4
atgggggcac ccgcttgcgc tctggctctg tgcgtcgctg tcgctattgt cgctggggca 60

tcatccgagt cactggggac cgaacagagg gtggtgggaa gggcagcaga ggtgccagga 120

cctgagccag gccagcagga gcagctggtg ttcggaagcg gcgacgccgt ggagctgtcc 180

tgcccacctc caggaggagg acctatggga ccaacagtgt gggtgaagga tggaaccgga 240

ctggtgccat ccgagcgggt gctggtggga cctcagagac tgcaggtgct gaacgcaagc 300

cacgaggact ccggagcata ctcttgcagg cagcgcctga cacagcgggt gctgtgccac 360

tttagcgtga gagtgaccga tgcaccaagc tccggcgacg atgaggacgg agaggatgag 420

gcagaggaca caggagtgga taccggagca ccatattgga ccaggccaga gagaatggac 480

aagaagctgc tggccgtgcc tgccgccaat acagtgcggt tcagatgccc agcagcagga 540

aacccaaccc cttctatcag ctggctgaag aatggcaggg agtttcgcgg cgagcacagg 600

atcggcggca tcaagctgcg ccaccagcag tggagcctgg tcatggagag cgtggtgcca 660

tccgaccggg gcaactacac atgcgtggtg gagaataagt tcggctccat cagacagacc 720

tatacactgg atgtgctgga gcggtgtccc cacagaccta tcctgcaggc aggactgcct 780

gcaaaccaga ccgccgtgct gggatctgac gtggagtttc actgtaaggt gtacagcgat 840

gcccagccac acatccagtg gctgaagcac gtggaagtga atggctctaa agtgggccct 900

gacggcacac catatgtgac cgtgctgaag agctggatct ccgagtctgt ggaggcagac 960

gtgaggctga ggctggcaaa cgtgtctgag agggatggag gagagtacct gtgcagggcc 1020

accaatttca tcggcgtggc cgagaaggcc ttttggctga gcgtgcacgg acctagggca 1080

gcagaggagg agctggtgga ggcagatgag gcaggaagcg tgtacgcagg catcctgagc 1140

tatggcgtgg gcttctttct gttcatcctg gtggtggcag cagtgaccct gtgcaggctg 1200

agatcccccc ctaagaaggg cctgggctcc ccaaccgtgc acaagatctc tcggtttccc 1260

ctgaagagac aggtgagcct ggagtccaac gcctctatgt ctagcaatac accccctggtg 1320

cggatcgcca gactgtcctc tggagaggga cctaccctgg caaacgtgtc cgagctggag 1380

ctgccagcag accccaagtg ggagctgtct agggcaaggc tgacactggg caagccactg 1440

ggagagggat gcttcggaca ggtggtcatg gcagaggcca tcggcatcga caaggatagg 1500

gcagcaaagc ctgtgacagt ggcagtgaag atgctgaagg acgatgccac cgacaaggat 1560

ctgtctgatc tggtgagcga gatggagatg atgaagatga tcggcaagca caagaacatc 1620

atcaatctgc tgggagcatg tacccaggga ggaccactgt acgtgctggt ggagtatgcc 1680

gccaagggca atctgaggga gtttctgagg gcacggagac cacccggact ggactactcc 1740

ttcgatacat gcaagcctcc agaggagcag ctgaccttta aggacctggt gagctgtgcc 1800

taccaggtgg ccaggggcat ggagtatctg gcctcccaga agtgtatcca cagggatctg 1860

gccgcccgca acgtgctggt gacagaggac aatgtgatga agatcgcaga tttcggactg 1920

gcaagggacg tgcacaacct ggattactat aagaagacca caaatggcag gctgcctgtg 1980

aagtggatgg ccccagaggc cctgtttgac cgcgtgtaca cacaccagtc cgacgtgtgg 2040

tctttcggcg tgctgctgtg ggagatcttt accctgggag gaagcccata tccaggcatc 2100

ccagtggagg agctgttcaa gctgctgaag gagggccacc ggatggacaa gcccgccaac 2160

tgcacacacg atctgtatat gatcatgagg gagtgttggc acgcagcacc atcccagaga 2220

cctaccttca agcagctggt ggaggacctg gatagagtgc tgaccgtgac atctaccgac 2280

gagtacctgg atctgagcgc cccctttgag cagtattccc ctggaggaca ggacacccca 2340

agctcctcta gctccggcga cgattcagtg ttcgcccatg acctgctgcc ccctgccccc 2400

ccaagtagtg gaggctcaag gact 2424

<210> 5
<211> 124
<212> PRT
<213> Artificial sequence

<220>
<223> an artificially synthesized sequence

<400> 5

Gln Glu Leu Val Glu Ser Gly Gly Gly Leu Val Gln Pro Gly Gly Ser
1 5 10 15

Leu Lys Leu Ser Cys Lys Ala Ser Gly Ile Asp Phe Ser Ser Tyr Gly

20 25 30

Ile Ser Trp Val Arg Gln Ala Pro Gly Lys Gly Leu Glu Trp Ile Ala
35 40 45

Tyr Ile Val Pro Gly Phe Gly Ile Arg Asn Tyr Ala Asn Ser Val Lys
50 55 60

Gly Arg Phe Thr Ile Ser Ser Asp Asn Ala Gln Asn Thr Val Phe Leu
65 70 75 80

Gln Met Thr Ser Leu Thr Ala Ser Asp Thr Ala Thr Tyr Phe Cys Ala
85 90 95

Arg Val Pro Gln Tyr Ala Ser Ser Ser Gly Tyr Tyr Leu Pro Phe Asn
100 105 110

Leu Trp Gly Pro Gly Thr Leu Val Thr Val Ser Ser
115 120

<210> 6
<211> 111
<212> PRT
<213> Artificial sequence

<220>
<223> an artificially synthesized sequence

<400> 6

Ala Leu Val Met Thr Gln Thr Pro Ser Pro Val Ser Ala Ala Val Gly
1 5 10 15

Gly Thr Val Thr Ile Asn Cys Gln Ala Ser Glu Asp Ile Tyr Tyr Asn
20 25 30

Leu Ala Trp Phe Gln Gln Lys Pro Gly Gln Pro Pro Lys Leu Leu Ile
35 40 45

Tyr Arg Ala Ser Thr Leu Ala Ser Gly Val Pro Ser Arg Phe Ser Gly
50 55 60

Ser Gly Ser Gly Thr Glu Phe Thr Leu Thr Ile Ser Asp Val Gln Cys
65 70 75 80

Asp Asp Ala Ala Thr Tyr Tyr Cys Leu Gly Val Tyr Thr Tyr Ile Ser
85 90 95

Ala Asp Gly Asn Ala Phe Gly Gly Gly Thr Glu Val Val Val Lys
100 105 110

<210> 7
<211> 119
<212> PRT
<213> Artificial sequence

<220>
<223> an artificially synthesized sequence

<400> 7

Gln Ser Leu Glu Glu Ser Gly Gly Arg Leu Val Thr Pro Gly Thr Pro
1               5                   10                  15

Leu Thr Leu Thr Cys Thr Ala Ser Gly Phe Ser Leu Ser Thr Tyr Trp
            20                  25                  30

Ile Cys Trp Val Arg Gln Ala Pro Gly Glu Gly Leu Glu Trp Ile Gly
        35                  40                  45

Tyr Ile Ser Ser Ser Gly Asn Thr Tyr Tyr Ala Ser Trp Ala Lys Gly
    50                  55                  60

Arg Phe Thr Ile Ser Lys Thr Ser Ser Thr Thr Val Asp Leu Lys Met
65                  70                  75                  80

Thr Ser Leu Thr Thr Glu Asp Thr Ala Thr Tyr Phe Cys Gly Gly His
                85                  90                  95

Asn Ser Asp Tyr Ile Thr Gly Trp Ala Leu Asp Pro Trp Gly Pro Gly
            100                 105                 110

Thr Leu Val Thr Val Ser Ser
        115

<210> 8
<211> 111
<212> PRT
<213> Artificial sequence

<220>
<223> an artificially synthesized sequence

<400> 8

Ala Ala Val Leu Thr Gln Thr Ala Ser Pro Met Ser Ala Ala Val Gly
1               5                   10                  15

Gly Thr Val Thr Ile Asn Cys Gln Ser Ser Lys Ser Val Tyr Arg Asp
            20                  25                  30

Lys Trp Leu Ser Trp Phe Gln Gln Lys Pro Gly Gln Pro Pro Lys Leu

35 40 45

Leu Ile Tyr Gly Ala Ser Asn Leu Ala Ser Gly Val Ser Ser Arg Phe
50 55 60

Lys Gly Ser Gly Ser Gly Thr Gln Phe Thr Leu Thr Ile Ser Asp Val
65 70 75 80

Gln Cys Asp Asp Ala Ala Thr Tyr Tyr Cys Leu Gly Gly Tyr Ser Asp
85 90 95

Asn Ser Glu Asp Ala Phe Gly Gly Gly Thr Glu Val Val Val Lys
100 105 110

<210> 9
<211> 122
<212> PRT
<213> Artificial sequence

<220>
<223> an artificially synthesized sequence

<400> 9

Gln Ser Val Glu Glu Ser Gly Gly Arg Leu Val Thr Pro Gly Thr Pro
1 5 10 15

Leu Thr Leu Thr Cys Thr Val Ser Gly Phe Ser Leu Ser Ser Tyr Pro
20 25 30

Met Ser Trp Val Arg Gln Ala Pro Gly Lys Gly Leu Glu Trp Ile Gly
35 40 45

Ile Ile Ser Ser Ser Gly Arg Thr Tyr Tyr Ala Ser Trp Ala Lys Gly
50 55 60

Arg Phe Thr Ile Ser Lys Ala Ser Ser Thr Thr Ala Ala Leu Lys Met
65 70 75 80

Thr Ser Leu Thr Thr Glu Asp Thr Ala Thr Tyr Phe Cys Ala Arg Gly
85 90 95

Gly Val Tyr Lys Tyr Asp Asp Tyr Gly Asp Tyr Val Leu Asn Leu Trp
100 105 110

Gly Arg Gly Thr Leu Val Thr Val Ser Ser
115 120

<210> 10
<211> 110

<212> PRT
<213> Artificial sequence

<220>
<223> an artificially synthesized sequence

<400> 10

Ala Ile Glu Met Thr Gln Thr Pro Phe Ser Val Ser Ala Ala Val Gly
1 5 10 15

Gly Thr Val Thr Ile Asn Cys Gln Ala Ser Glu Asn Ile Tyr Ser Asn
20 25 30

Leu Ala Trp Tyr Gln Gln Lys Pro Gly Gln Pro Pro Lys Leu Leu Ile
35 40 45

Tyr Ala Ala Ser Tyr Leu Ala Ser Gly Val Pro Ser Arg Phe Lys Gly
50 55 60

Ser Gly Ser Gly Thr Asp Tyr Thr Leu Thr Ile Ser Gly Val Gln Cys
65 70 75 80

Ala Asp Ala Ala Thr Tyr Tyr Cys Gln Cys Thr Leu Tyr Ser Ser Thr
85 90 95

Tyr Gly Val Ala Phe Gly Gly Gly Thr Glu Val Val Val Lys
100 105 110

<210> 11
<211> 328
<212> PRT
<213> Artificial sequence

<220>
<223> an artificially synthesized sequence

<400> 11

Ala Ser Thr Lys Gly Pro Ser Val Phe Pro Leu Ala Pro Ser Ser Lys
1 5 10 15

Ser Thr Ser Gly Gly Thr Ala Ala Leu Gly Cys Leu Val Lys Asp Tyr
20 25 30

Phe Pro Glu Pro Val Thr Val Ser Trp Asn Ser Gly Ala Leu Thr Ser
35 40 45

Gly Val His Thr Phe Pro Ala Val Leu Gln Ser Ser Gly Leu Tyr Ser
50 55 60

Leu Ser Ser Val Val Thr Val Pro Ser Ser Ser Leu Gly Thr Gln Thr

65 70 75 80

Tyr Ile Cys Asn Val Asn His Lys Pro Ser Asn Thr Lys Val Asp Lys
85 90 95

Lys Val Glu Pro Lys Ser Cys Asp Lys Thr His Thr Cys Pro Pro Cys
100 105 110

Pro Ala Pro Glu Leu Arg Gly Gly Pro Lys Val Phe Leu Phe Pro Pro
115 120 125

Lys Pro Lys Asp Thr Leu Met Ile Ser Arg Thr Pro Glu Val Thr Cys
130 135 140

Val Val Val Asp Val Ser His Glu Asp Pro Glu Val Lys Phe Asn Trp
145 150 155 160

Tyr Val Asp Gly Val Glu Val His Asn Ala Lys Thr Lys Pro Arg Glu
165 170 175

Glu Gln Tyr Ala Ser Thr Tyr Arg Val Val Ser Val Leu Thr Val Leu
180 185 190

His Gln Asp Trp Leu Asn Gly Lys Glu Tyr Lys Cys Lys Val Ser Asn
195 200 205

Lys Ala Leu Pro Ala Pro Ile Glu Lys Thr Ile Ser Lys Ala Lys Gly
210 215 220

Gln Pro Arg Glu Pro Gln Val Tyr Thr Leu Pro Pro Ser Arg Glu Glu
225 230 235 240

Met Thr Lys Asn Gln Val Ser Leu Thr Cys Leu Val Lys Gly Phe Tyr
245 250 255

Pro Ser Asp Ile Ala Val Glu Trp Glu Ser Asn Gly Gln Pro Glu Asn
260 265 270

Asn Tyr Lys Thr Thr Pro Pro Tyr Leu Asp Ser Asp Gly Ser Phe Phe
275 280 285

Leu Tyr Ser Lys Leu Thr Val Asp Lys Ser Arg Trp Gln Gln Gly Asn
290 295 300

Val Phe Ser Cys Ser Val Met His Glu Ala Leu His Asn His Tyr Thr
305 310 315 320

```
Gln Glu Ser Leu Ser Leu Ser Pro
                325


<210>  12
<211>  107
<212>  PRT
<213>  Artificial sequence

<220>
<223>  an artificially synthesized sequence

<400>  12

Arg Thr Val Ala Ala Pro Ser Val Phe Ile Phe Pro Pro Ser Asp Glu
1               5                   10                  15


Gln Leu Lys Ser Gly Thr Ala Ser Val Val Cys Leu Leu Asn Asn Phe
            20                  25                  30


Tyr Pro Arg Glu Ala Lys Val Gln Trp Lys Val Asp Asn Ala Leu Gln
        35                  40                  45


Ser Gly Asn Ser Gln Glu Ser Val Thr Glu Gln Asp Ser Lys Asp Cys
    50                  55                  60


Thr Tyr Ser Leu Ser Ser Thr Leu Thr Leu Ser Lys Ala Asp Tyr Glu
65                  70                  75                  80


Lys His Lys Val Tyr Ala Cys Glu Val Thr His Gln Gly Leu Ser Ser
                85                  90                  95


Pro Val Thr Lys Ser Phe Asn Arg Gly Glu Cys
            100                 105


<210>  13
<211>  122
<212>  PRT
<213>  Artificial sequence

<220>
<223>  an artificially synthesized sequence

<400>  13

Gln Val Gln Leu Val Glu Ser Gly Gly Gly Val Val Gln Pro Gly Gly
1               5                   10                  15


Ser Leu Arg Leu Ser Cys Ala Ala Ser Gly Phe Thr Phe Ser Asn Ala
            20                  25                  30


Trp Met His Trp Val Arg Gln Ala Pro Gly Lys Gly Leu Glu Trp Val
        35                  40                  45
```

Ala Gln Ile Lys Asp Lys Ser Gln Asn Tyr Ala Thr Tyr Val Ala Glu
50 55 60

Ser Val Lys Gly Arg Phe Thr Ile Ser Arg Ala Asp Ser Lys Asn Ser
65 70 75 80

Ile Tyr Leu Gln Met Asn Ser Leu Lys Thr Glu Asp Thr Ala Val Tyr
85 90 95

Tyr Cys Arg Tyr Val His Tyr Ala Ala Gly Tyr Gly Val Asp Ile Trp
100 105 110

Gly Gln Gly Thr Thr Val Thr Val Ser Ser
115 120

<210> 14
<211> 328
<212> PRT
<213> Artificial sequence

<220>
<223> an artificially synthesized sequence

<400> 14

Ala Ser Thr Lys Gly Pro Ser Val Phe Pro Leu Ala Pro Ser Ser Lys
1 5 10 15

Ser Thr Ser Gly Gly Thr Ala Ala Leu Gly Cys Leu Val Lys Asp Tyr
20 25 30

Phe Pro Glu Pro Val Thr Val Ser Trp Asn Ser Gly Ala Leu Thr Ser
35 40 45

Gly Val His Thr Phe Pro Ala Val Leu Gln Ser Ser Gly Leu Tyr Ser
50 55 60

Leu Ser Ser Val Val Thr Val Pro Ser Ser Ser Leu Gly Thr Gln Thr
65 70 75 80

Tyr Ile Cys Asn Val Asn His Lys Pro Ser Asn Thr Lys Val Asp Lys
85 90 95

Lys Val Glu Pro Lys Ser Cys Asp Lys Thr His Thr Cys Pro Pro Cys
100 105 110

Pro Ala Pro Glu Leu Arg Gly Gly Pro Lys Val Phe Leu Phe Pro Pro
115 120 125

Lys Pro Lys Asp Thr Leu Met Ile Ser Arg Thr Pro Glu Val Thr Cys
    130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Ala Ser Thr Tyr Arg Val Val Ser Val Leu Thr Val Leu
            180                 185                 190

His Gln Asp Trp Leu Asn Gly Lys Glu Tyr Lys Cys Lys Val Ser Asn
        195                 200                 205

Lys Ala Leu Pro Ala Pro Ile Glu Lys Thr Ile Ser Lys Ala Lys Gly
    210                 215                 220

Gln Pro Arg Glu Pro Gln Val Tyr Thr Leu Pro Pro Ser Arg Lys Glu
225                 230                 235                 240

Met Thr Lys Asn Gln Val Ser Leu Thr Cys Leu Val Lys Gly Phe Tyr
                245                 250                 255

Pro Ser Asp Ile Ala Val Glu Trp Glu Ser Asn Gly Gln Pro Glu Asn
            260                 265                 270

Asn Tyr Lys Thr Thr Pro Pro Tyr Leu Asp Ser Asp Gly Ser Phe Phe
        275                 280                 285

Leu Tyr Ser Lys Leu Thr Val Asp Lys Ser Arg Trp Gln Gln Gly Asn
    290                 295                 300

Val Phe Ser Cys Ser Val Met His Glu Ala Leu His Asn His Tyr Thr
305                 310                 315                 320

Gln Lys Ser Leu Ser Leu Ser Pro
                325

<210> 15
<211> 112
<212> PRT
<213> Artificial sequence

<220>
<223> an artificially synthesized sequence

<400> 15

Asp Ile Val Met Thr Gln Ser Pro Leu Ser Leu Pro Val Thr Pro Gly

1 5 10 15

Glu Pro Ala Ser Ile Ser Cys Arg Ser Ser Gln Pro Leu Val His Ser
20 25 30

Asn Arg Asn Thr Tyr Leu His Trp Tyr Gln Gln Lys Pro Gly Gln Ala
35 40 45

Pro Arg Leu Leu Ile Tyr Lys Val Ser Asn Arg Phe Ser Gly Val Pro
50 55 60

Asp Arg Phe Ser Gly Ser Gly Ser Gly Thr Asp Phe Thr Leu Lys Ile
65 70 75 80

Ser Arg Val Glu Ala Glu Asp Val Gly Val Tyr Tyr Cys Gly Gln Gly
85 90 95

Thr Gln Val Pro Tyr Thr Phe Gly Gln Gly Thr Lys Leu Glu Ile Lys
100 105 110

<210> 16
<211> 107
<212> PRT
<213> Artificial sequence

<220>
<223> an artificially synthesized sequence

<400> 16

Arg Thr Val Ala Ala Pro Ser Val Phe Ile Phe Pro Pro Ser Asp Glu
1 5 10 15

Gln Leu Lys Ser Gly Thr Ala Ser Val Val Cys Leu Leu Asn Asn Phe
20 25 30

Tyr Pro Arg Glu Ala Lys Val Gln Trp Lys Val Asp Asn Ala Leu Gln
35 40 45

Ser Gly Asn Ser Gln Glu Ser Val Thr Glu Gln Asp Ser Lys Asp Ser
50 55 60

Thr Tyr Ser Leu Ser Ser Thr Leu Thr Leu Ser Lys Ala Asp Tyr Glu
65 70 75 80

Lys His Lys Val Tyr Ala Cys Glu Val Thr His Gln Gly Leu Ser Ser
85 90 95

Pro Val Thr Lys Ser Phe Asn Arg Gly Glu Cys
100 105

<210> 17
<211> 5
<212> PRT
<213> Artificial sequence

<220>
<223> an artificially synthesized sequence

<400> 17

Ser Tyr Gly Ile Ser
1 5

<210> 18
<211> 17
<212> PRT
<213> Artificial sequence

<220>
<223> an artificially synthesized sequence

<400> 18

Tyr Ile Val Pro Gly Phe Gly Ile Arg Asn Tyr Ala Asn Ser Val Lys
1 5 10 15

Gly

<210> 19
<211> 16
<212> PRT
<213> Artificial sequence

<220>
<223> an artificially synthesized sequence

<400> 19

Val Pro Gln Tyr Ala Ser Ser Ser Gly Tyr Tyr Leu Pro Phe Asn Leu
1 5 10 15

<210> 20
<211> 11
<212> PRT
<213> Artificial sequence

<220>
<223> an artificially synthesized sequence

<400> 20

Gln Ala Ser Glu Asp Ile Tyr Tyr Asn Leu Ala
1 5 10

<210> 21

<211> 7
<212> PRT
<213> Artificial sequence

<220>
<223> an artificially synthesized sequence

<400> 21

Arg Ala Ser Thr Leu Ala Ser
1 5

<210> 22
<211> 13
<212> PRT
<213> Artificial sequence

<220>
<223> an artificially synthesized sequence

<400> 22

Leu Gly Val Tyr Thr Tyr Ile Ser Ala Asp Gly Asn Ala
1 5 10

<210> 23
<211> 5
<212> PRT
<213> Artificial sequence

<220>
<223> an artificially synthesized sequence

<400> 23

Thr Tyr Trp Ile Cys
1 5

<210> 24
<211> 16
<212> PRT
<213> Artificial sequence

<220>
<223> an artificially synthesized sequence

<400> 24

Tyr Ile Ser Ser Ser Gly Asn Thr Tyr Tyr Ala Ser Trp Ala Lys Gly
1 5 10 15

<210> 25
<211> 13
<212> PRT
<213> Artificial sequence

<220>
<223> an artificially synthesized sequence

<400> 25

His Asn Ser Asp Tyr Ile Thr Gly Trp Ala Leu Asp Pro
1 5 10

<210> 26
<211> 10
<212> PRT
<213> Artificial sequence

<220>
<223> an artificially synthesized sequence

<400> 26

Gln Ser Ser Lys Ser Val Tyr Arg Asp Lys
1 5 10

<210> 27
<211> 7
<212> PRT
<213> Artificial sequence

<220>
<223> an artificially synthesized sequence

<400> 27

Gly Ala Ser Asn Leu Ala Ser
1 5

<210> 28
<211> 11
<212> PRT
<213> Artificial sequence

<220>
<223> an artificially synthesized sequence

<400> 28

Leu Gly Gly Tyr Ser Asp Asn Ser Glu Asp Ala
1 5 10

<210> 29
<211> 5
<212> PRT
<213> Artificial sequence

<220>
<223> an artificially synthesized sequence

<400> 29

Ser Tyr Pro Met Ser
1 5

<210> 30

<211> 16
<212> PRT
<213> Artificial sequence

<220>
<223> an artificially synthesized sequence

<400> 30

Ile Ile Ser Ser Ser Gly Arg Thr Tyr Tyr Ala Ser Trp Ala Lys Gly
1 5 10 15

<210> 31
<211> 16
<212> PRT
<213> Artificial sequence

<220>
<223> an artificially synthesized sequence

<400> 31

Gly Gly Val Tyr Lys Tyr Asp Asp Tyr Gly Asp Tyr Val Leu Asn Leu
1 5 10 15

<210> 32
<211> 11
<212> PRT
<213> Artificial sequence

<220>
<223> an artificially synthesized sequence

<400> 32

Gln Ala Ser Glu Asn Ile Tyr Ser Asn Leu Ala
1 5 10

<210> 33
<211> 7
<212> PRT
<213> Artificial sequence

<220>
<223> an artificially synthesized sequence

<400> 33

Ala Ala Ser Tyr Leu Ala Ser
1 5

<210> 34
<211> 12
<212> PRT
<213> Artificial sequence

<220>
<223> an artificially synthesized sequence

<400> 34

Gln Cys Thr Leu Tyr Ser Ser Thr Tyr Gly Val Ala
1 5 10

<210> 35
<211> 29
<212> PRT
<213> Artificial sequence

<220>
<223> an artificially synthesized sequence

<400> 35

Gln Glu Leu Val Glu Ser Gly Gly Gly Leu Val Gln Pro Gly Gly Ser
1 5 10 15

Leu Lys Leu Ser Cys Lys Ala Ser Gly Ile Asp Phe Ser
20 25

<210> 36
<211> 14
<212> PRT
<213> Artificial sequence

<220>
<223> an artificially synthesized sequence

<400> 36

Trp Val Arg Gln Ala Pro Gly Lys Gly Leu Glu Trp Ile Ala
1 5 10

<210> 37
<211> 32
<212> PRT
<213> Artificial sequence

<220>
<223> an artificially synthesized sequence

<400> 37

Arg Phe Thr Ile Ser Ser Asp Asn Ala Gln Asn Thr Val Phe Leu Gln
1 5 10 15

Met Thr Ser Leu Thr Ala Ser Asp Thr Ala Thr Tyr Phe Cys Ala Arg
20 25 30

<210> 38
<211> 11
<212> PRT
<213> Artificial sequence

<220>
<223> an artificially synthesized sequence

<400> 38

Trp Gly Pro Gly Thr Leu Val Thr Val Ser Ser
1 5 10

<210> 39
<211> 29
<212> PRT
<213> Artificial sequence

<220>
<223> an artificially synthesized sequence

<400> 39

Gln Ser Leu Glu Glu Ser Gly Gly Arg Leu Val Thr Pro Gly Thr Pro
1 5 10 15

Leu Thr Leu Thr Cys Thr Ala Ser Gly Phe Ser Leu Ser
20 25

<210> 40
<211> 14
<212> PRT
<213> Artificial sequence

<220>
<223> an artificially synthesized sequence

<400> 40

Trp Val Arg Gln Ala Pro Gly Glu Gly Leu Glu Trp Ile Gly
1 5 10

<210> 41
<211> 31
<212> PRT
<213> Artificial sequence

<220>
<223> an artificially synthesized sequence

<400> 41

Arg Phe Thr Ile Ser Lys Thr Ser Ser Thr Thr Val Asp Leu Lys Met
1 5 10 15

Thr Ser Leu Thr Thr Glu Asp Thr Ala Thr Tyr Phe Cys Gly Gly
20 25 30

<210> 42
<211> 11
<212> PRT
<213> Artificial sequence

<220>

<223> an artificially synthesized sequence

<400> 42

Trp Gly Pro Gly Thr Leu Val Thr Val Ser Ser
1 5 10

<210> 43
<211> 29
<212> PRT
<213> Artificial sequence

<220>
<223> an artificially synthesized sequence

<400> 43

Gln Ser Val Glu Glu Ser Gly Gly Arg Leu Val Thr Pro Gly Thr Pro
1 5 10 15

Leu Thr Leu Thr Cys Thr Val Ser Gly Phe Ser Leu Ser
20 25

<210> 44
<211> 14
<212> PRT
<213> Artificial sequence

<220>
<223> an artificially synthesized sequence

<400> 44

Trp Val Arg Gln Ala Pro Gly Lys Gly Leu Glu Trp Ile Gly
1 5 10

<210> 45
<211> 31
<212> PRT
<213> Artificial sequence

<220>
<223> an artificially synthesized sequence

<400> 45

Arg Phe Thr Ile Ser Lys Ala Ser Ser Thr Thr Ala Ala Leu Lys Met
1 5 10 15

Thr Ser Leu Thr Thr Glu Asp Thr Ala Thr Tyr Phe Cys Ala Arg
20 25 30

<210> 46
<211> 11
<212> PRT
<213> Artificial sequence

<220>
<223>  an artificially synthesized sequence

<400>  46

Trp Gly Arg Gly Thr Leu Val Thr Val Ser Ser
1               5                   10

<210>  47
<211>  23
<212>  PRT
<213>  Artificial sequence

<220>
<223>  an artificially synthesized sequence

<400>  47

Ala Leu Val Met Thr Gln Thr Pro Ser Pro Val Ser Ala Ala Val Gly
1               5                   10                  15

Gly Thr Val Thr Ile Asn Cys
            20

<210>  48
<211>  15
<212>  PRT
<213>  Artificial sequence

<220>
<223>  an artificially synthesized sequence

<400>  48

Trp Phe Gln Gln Lys Pro Gly Gln Pro Pro Lys Leu Leu Ile Tyr
1               5                   10                  15

<210>  49
<211>  32
<212>  PRT
<213>  Artificial sequence

<220>
<223>  an artificially synthesized sequence

<400>  49

Gly Val Pro Ser Arg Phe Ser Gly Ser Gly Ser Gly Thr Glu Phe Thr
1               5                   10                  15

Leu Thr Ile Ser Asp Val Gln Cys Asp Asp Ala Ala Thr Tyr Tyr Cys
            20                  25                  30

<210>  50
<211>  10
<212>  PRT
<213>  Artificial sequence

<220>
<223> an artificially synthesized sequence

<400> 50

Phe Gly Gly Gly Thr Glu Val Val Val Lys
1               5                   10

<210> 51
<211> 23
<212> PRT
<213> Artificial sequence

<220>
<223> an artificially synthesized sequence

<400> 51

Ala Ala Val Leu Thr Gln Thr Ala Ser Pro Met Ser Ala Ala Val Gly
1               5                   10                  15

Gly Thr Val Thr Ile Asn Cys
            20

<210> 52
<211> 18
<212> PRT
<213> Artificial sequence

<220>
<223> an artificially synthesized sequence

<400> 52

Trp Leu Ser Trp Phe Gln Gln Lys Pro Gly Gln Pro Pro Lys Leu Leu
1               5                   10                  15

Ile Tyr

<210> 53
<211> 32
<212> PRT
<213> Artificial sequence

<220>
<223> an artificially synthesized sequence

<400> 53

Gly Val Ser Ser Arg Phe Lys Gly Ser Gly Ser Gly Thr Gln Phe Thr
1               5                   10                  15

Leu Thr Ile Ser Asp Val Gln Cys Asp Asp Ala Ala Thr Tyr Tyr Cys
            20                  25                  30

<210> 54
<211> 10
<212> PRT
<213> Artificial sequence

<220>
<223> an artificially synthesized sequence

<400> 54

```
Phe Gly Gly Gly Thr Glu Val Val Val Lys
1               5                   10
```

<210> 55
<211> 23
<212> PRT
<213> Artificial sequence

<220>
<223> an artificially synthesized sequence

<400> 55

```
Ala Ile Glu Met Thr Gln Thr Pro Phe Ser Val Ser Ala Ala Val Gly
1               5                   10                  15

Gly Thr Val Thr Ile Asn Cys
            20
```

<210> 56
<211> 15
<212> PRT
<213> Artificial sequence

<220>
<223> an artificially synthesized sequence

<400> 56

```
Trp Tyr Gln Gln Lys Pro Gly Gln Pro Pro Lys Leu Leu Ile Tyr
1               5                   10                  15
```

<210> 57
<211> 32
<212> PRT
<213> Artificial sequence

<220>
<223> an artificially synthesized sequence

<400> 57

```
Gly Val Pro Ser Arg Phe Lys Gly Ser Gly Ser Gly Thr Asp Tyr Thr
1               5                   10                  15

Leu Thr Ile Ser Gly Val Gln Cys Ala Asp Ala Ala Thr Tyr Tyr Cys
            20                  25                  30
```

<210> 58
<211> 10
<212> PRT
<213> Artificial sequence

<220>
<223> an artificially synthesized sequence

<400> 58

Phe Gly Gly Gly Thr Glu Val Val Val Lys
1 5 10

<210> 59
<211> 5
<212> PRT
<213> Artificial sequence

<220>
<223> an artificially synthesized sequence

<400> 59

Asn Ala Trp Met His
1 5

<210> 60
<211> 19
<212> PRT
<213> Artificial sequence

<220>
<223> an artificially synthesized sequence

<400> 60

Gln Ile Lys Asp Lys Ser Gln Asn Tyr Ala Thr Tyr Val Ala Glu Ser
1 5 10 15

Val Lys Gly

<210> 61
<211> 11
<212> PRT
<213> Artificial sequence

<220>
<223> an artificially synthesized sequence

<400> 61

Val His Tyr Ala Ala Gly Tyr Gly Val Asp Ile
1 5 10

<210> 62

<211> 16
<212> PRT
<213> Artificial sequence

<220>
<223> an artificially synthesized sequence

<400> 62

Arg Ser Ser Gln Pro Leu Val His Ser Asn Arg Asn Thr Tyr Leu His
1 5 10 15

<210> 63
<211> 7
<212> PRT
<213> Artificial sequence

<220>
<223> an artificially synthesized sequence

<400> 63

Lys Val Ser Asn Arg Phe Ser
1 5

<210> 64
<211> 9
<212> PRT
<213> Artificial sequence

<220>
<223> an artificially synthesized sequence

<400> 64

Gly Gln Gly Thr Gln Val Pro Tyr Thr
1 5

<210> 65
<211> 806
<212> PRT
<213> Homo Sapiens

<400> 65

Met Gly Ala Pro Ala Cys Ala Leu Ala Leu Cys Val Ala Val Ala Ile
1 5 10 15

Val Ala Gly Ala Ser Ser Glu Ser Leu Gly Thr Glu Gln Arg Val Val
20 25 30

Gly Arg Ala Ala Glu Val Pro Gly Pro Glu Pro Gly Gln Gln Glu Gln
35 40 45

Leu Val Phe Gly Ser Gly Asp Ala Val Glu Leu Ser Cys Pro Pro Pro
50 55 60

```
Gly Gly Gly Pro Met Gly Pro Thr Val Trp Val Lys Asp Gly Thr Gly
65                  70                  75                  80

Leu Val Pro Ser Glu Arg Val Leu Val Gly Pro Gln Arg Leu Gln Val
                85                  90                  95

Leu Asn Ala Ser His Glu Asp Ser Gly Ala Tyr Ser Cys Arg Gln Arg
            100                 105                 110

Leu Thr Gln Arg Val Leu Cys His Phe Ser Val Arg Val Thr Asp Ala
        115                 120                 125

Pro Ser Ser Gly Asp Asp Glu Asp Gly Glu Asp Glu Ala Glu Asp Thr
    130                 135                 140

Gly Val Asp Thr Gly Ala Pro Tyr Trp Thr Arg Pro Glu Arg Met Asp
145                 150                 155                 160

Lys Lys Leu Leu Ala Val Pro Ala Ala Asn Thr Val Arg Phe Arg Cys
                165                 170                 175

Pro Ala Ala Gly Asn Pro Thr Pro Ser Ile Ser Trp Leu Lys Asn Gly
            180                 185                 190

Arg Glu Phe Arg Gly Glu His Arg Ile Gly Gly Ile Lys Leu Arg His
        195                 200                 205

Gln Gln Trp Ser Leu Val Met Glu Ser Val Val Pro Ser Asp Arg Gly
    210                 215                 220

Asn Tyr Thr Cys Val Val Glu Asn Lys Phe Gly Ser Ile Arg Gln Thr
225                 230                 235                 240

Tyr Thr Leu Asp Val Leu Glu Arg Ser Pro His Arg Pro Ile Leu Gln
                245                 250                 255

Ala Gly Leu Pro Ala Asn Gln Thr Ala Val Leu Gly Ser Asp Val Glu
            260                 265                 270

Phe His Cys Lys Val Tyr Ser Asp Ala Gln Pro His Ile Gln Trp Leu
        275                 280                 285

Lys His Val Glu Val Asn Gly Ser Lys Val Gly Pro Asp Gly Thr Pro
    290                 295                 300

Tyr Val Thr Val Leu Lys Thr Ala Gly Ala Asn Thr Thr Asp Lys Glu
305                 310                 315                 320
```

```
Leu Glu Val Leu Ser Leu His Asn Val Thr Phe Glu Asp Ala Gly Glu
                325                 330                 335

Tyr Thr Cys Leu Ala Gly Asn Ser Ile Gly Phe Ser His His Ser Ala
            340                 345                 350

Trp Leu Val Val Leu Pro Ala Glu Glu Glu Leu Val Glu Ala Asp Glu
        355                 360                 365

Ala Gly Ser Val Tyr Ala Gly Ile Leu Ser Tyr Gly Val Gly Phe Phe
    370                 375                 380

Leu Phe Ile Leu Val Val Ala Ala Val Thr Leu Cys Arg Leu Arg Ser
385                 390                 395                 400

Pro Pro Lys Lys Gly Leu Gly Ser Pro Thr Val His Lys Ile Ser Arg
                405                 410                 415

Phe Pro Leu Lys Arg Gln Val Ser Leu Glu Ser Asn Ala Ser Met Ser
            420                 425                 430

Ser Asn Thr Pro Leu Val Arg Ile Ala Arg Leu Ser Ser Gly Glu Gly
        435                 440                 445

Pro Thr Leu Ala Asn Val Ser Glu Leu Glu Leu Pro Ala Asp Pro Lys
    450                 455                 460

Trp Glu Leu Ser Arg Ala Arg Leu Thr Leu Gly Lys Pro Leu Gly Glu
465                 470                 475                 480

Gly Cys Phe Gly Gln Val Val Met Ala Glu Ala Ile Gly Ile Asp Lys
                485                 490                 495

Asp Arg Ala Ala Lys Pro Val Thr Val Ala Val Lys Met Leu Lys Asp
            500                 505                 510

Asp Ala Thr Asp Lys Asp Leu Ser Asp Leu Val Ser Glu Met Glu Met
        515                 520                 525

Met Lys Met Ile Gly Lys His Lys Asn Ile Ile Asn Leu Leu Gly Ala
    530                 535                 540

Cys Thr Gln Gly Gly Pro Leu Tyr Val Leu Val Glu Tyr Ala Ala Lys
545                 550                 555                 560

Gly Asn Leu Arg Glu Phe Leu Arg Ala Arg Arg Pro Pro Gly Leu Asp
```

565 570 575

Tyr Ser Phe Asp Thr Cys Lys Pro Pro Glu Glu Gln Leu Thr Phe Lys
580 585 590

Asp Leu Val Ser Cys Ala Tyr Gln Val Ala Arg Gly Met Glu Tyr Leu
595 600 605

Ala Ser Gln Lys Cys Ile His Arg Asp Leu Ala Ala Arg Asn Val Leu
610 615 620

Val Thr Glu Asp Asn Val Met Lys Ile Ala Asp Phe Gly Leu Ala Arg
625 630 635 640

Asp Val His Asn Leu Asp Tyr Tyr Lys Lys Thr Thr Asn Gly Arg Leu
645 650 655

Pro Val Lys Trp Met Ala Pro Glu Ala Leu Phe Asp Arg Val Tyr Thr
660 665 670

His Gln Ser Asp Val Trp Ser Phe Gly Val Leu Leu Trp Glu Ile Phe
675 680 685

Thr Leu Gly Gly Ser Pro Tyr Pro Gly Ile Pro Val Glu Glu Leu Phe
690 695 700

Lys Leu Leu Lys Glu Gly His Arg Met Asp Lys Pro Ala Asn Cys Thr
705 710 715 720

His Asp Leu Tyr Met Ile Met Arg Glu Cys Trp His Ala Ala Pro Ser
725 730 735

Gln Arg Pro Thr Phe Lys Gln Leu Val Glu Asp Leu Asp Arg Val Leu
740 745 750

Thr Val Thr Ser Thr Asp Glu Tyr Leu Asp Leu Ser Ala Pro Phe Glu
755 760 765

Gln Tyr Ser Pro Gly Gly Gln Asp Thr Pro Ser Ser Ser Ser Ser Gly
770 775 780

Asp Asp Ser Val Phe Ala His Asp Leu Leu Pro Pro Ala Pro Pro Ser
785 790 795 800

Ser Gly Gly Ser Arg Thr
805

<210> 66
<211> 330
<212> PRT
<213> Artificial sequence

<220>
<223> an artificially synthesized sequence

<400> 66

Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Lys Val Glu Pro Lys Ser Cys Asp Lys Thr His Thr Cys Pro Pro Cys
            100                 105                 110

Pro Ala Pro Glu Leu Leu Gly Gly Pro Ser Val Phe Leu Phe Pro Pro
        115                 120                 125

Lys Pro Lys Asp Thr Leu Met Ile Ser Arg Thr Pro Glu Val Thr Cys
    130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Asn Ser Thr Tyr Arg Val Val Ser Val Leu Thr Val Leu
            180                 185                 190

His Gln Asp Trp Leu Asn Gly Lys Glu Tyr Lys Cys Lys Val Ser Asn
        195                 200                 205

Lys Ala Leu Pro Ala Pro Ile Glu Lys Thr Ile Ser Lys Ala Lys Gly

210 215 220

Gln Pro Arg Glu Pro Gln Val Tyr Thr Leu Pro Pro Ser Arg Asp Glu
225 230 235 240

Leu Thr Lys Asn Gln Val Ser Leu Thr Cys Leu Val Lys Gly Phe Tyr
245 250 255

Pro Ser Asp Ile Ala Val Glu Trp Glu Ser Asn Gly Gln Pro Glu Asn
260 265 270

Asn Tyr Lys Thr Thr Pro Pro Val Leu Asp Ser Asp Gly Ser Phe Phe
275 280 285

Leu Tyr Ser Lys Leu Thr Val Asp Lys Ser Arg Trp Gln Gln Gly Asn
290 295 300

Val Phe Ser Cys Ser Val Met His Glu Ala Leu His Asn His Tyr Thr
305 310 315 320

Gln Lys Ser Leu Ser Leu Ser Pro Gly Lys
325 330

<210> 67
<211> 326
<212> PRT
<213> Artificial sequence

<220>
<223> an artificially synthesized sequence

<400> 67

Ala Ser Thr Lys Gly Pro Ser Val Phe Pro Leu Ala Pro Cys Ser Arg
1 5 10 15

Ser Thr Ser Glu Ser Thr Ala Ala Leu Gly Cys Leu Val Lys Asp Tyr
20 25 30

Phe Pro Glu Pro Val Thr Val Ser Trp Asn Ser Gly Ala Leu Thr Ser
35 40 45

Gly Val His Thr Phe Pro Ala Val Leu Gln Ser Ser Gly Leu Tyr Ser
50 55 60

Leu Ser Ser Val Val Thr Val Pro Ser Ser Asn Phe Gly Thr Gln Thr
65 70 75 80

Tyr Thr Cys Asn Val Asp His Lys Pro Ser Asn Thr Lys Val Asp Lys
85 90 95

```
Thr Val Glu Arg Lys Cys Cys Val Glu Cys Pro Pro Cys Pro Ala Pro
            100                 105                 110

Pro Val Ala Gly Pro Ser Val Phe Leu Phe Pro Pro Lys Pro Lys Asp
        115                 120                 125

Thr Leu Met Ile Ser Arg Thr Pro Glu Val Thr Cys Val Val Val Asp
    130                 135                 140

Val Ser His Glu Asp Pro Glu Val Gln Phe Asn Trp Tyr Val Asp Gly
145                 150                 155                 160

Val Glu Val His Asn Ala Lys Thr Lys Pro Arg Glu Glu Gln Phe Asn
                165                 170                 175

Ser Thr Phe Arg Val Val Ser Val Leu Thr Val Val His Gln Asp Trp
            180                 185                 190

Leu Asn Gly Lys Glu Tyr Lys Cys Lys Val Ser Asn Lys Gly Leu Pro
        195                 200                 205

Ala Pro Ile Glu Lys Thr Ile Ser Lys Thr Lys Gly Gln Pro Arg Glu
    210                 215                 220

Pro Gln Val Tyr Thr Leu Pro Pro Ser Arg Glu Glu Met Thr Lys Asn
225                 230                 235                 240

Gln Val Ser Leu Thr Cys Leu Val Lys Gly Phe Tyr Pro Ser Asp Ile
                245                 250                 255

Ala Val Glu Trp Glu Ser Asn Gly Gln Pro Glu Asn Asn Tyr Lys Thr
            260                 265                 270

Thr Pro Pro Met Leu Asp Ser Asp Gly Ser Phe Phe Leu Tyr Ser Lys
        275                 280                 285

Leu Thr Val Asp Lys Ser Arg Trp Gln Gln Gly Asn Val Phe Ser Cys
    290                 295                 300

Ser Val Met His Glu Ala Leu His Asn His Tyr Thr Gln Lys Ser Leu
305                 310                 315                 320

Ser Leu Ser Pro Gly Lys
                325
```

<210> 68

<211> 377
<212> PRT
<213> Artificial sequence

<220>
<223> an artificially synthesized sequence

<400> 68

```
Ala Ser Thr Lys Gly Pro Ser Val Phe Pro Leu Ala Pro Cys Ser Arg
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Thr Cys Asn Val Asn His Lys Pro Ser Asn Thr Lys Val Asp Lys
                85                  90                  95

Arg Val Glu Leu Lys Thr Pro Leu Gly Asp Thr Thr His Thr Cys Pro
            100                 105                 110

Arg Cys Pro Glu Pro Lys Ser Cys Asp Thr Pro Pro Pro Cys Pro Arg
        115                 120                 125

Cys Pro Glu Pro Lys Ser Cys Asp Thr Pro Pro Pro Cys Pro Arg Cys
    130                 135                 140

Pro Glu Pro Lys Ser Cys Asp Thr Pro Pro Pro Cys Pro Arg Cys Pro
145                 150                 155                 160

Ala Pro Glu Leu Leu Gly Gly Pro Ser Val Phe Leu Phe Pro Pro Lys
                165                 170                 175

Pro Lys Asp Thr Leu Met Ile Ser Arg Thr Pro Glu Val Thr Cys Val
            180                 185                 190

Val Val Asp Val Ser His Glu Asp Pro Glu Val Gln Phe Lys Trp Tyr
        195                 200                 205

Val Asp Gly Val Glu Val His Asn Ala Lys Thr Lys Pro Arg Glu Glu
    210                 215                 220
```

Gln Tyr Asn Ser Thr Phe Arg Val Val Ser Val Leu Thr Val Leu His
225 230 235 240

Gln Asp Trp Leu Asn Gly Lys Glu Tyr Lys Cys Lys Val Ser Asn Lys
245 250 255

Ala Leu Pro Ala Pro Ile Glu Lys Thr Ile Ser Lys Thr Lys Gly Gln
260 265 270

Pro Arg Glu Pro Gln Val Tyr Thr Leu Pro Pro Ser Arg Glu Glu Met
275 280 285

Thr Lys Asn Gln Val Ser Leu Thr Cys Leu Val Lys Gly Phe Tyr Pro
290 295 300

Ser Asp Ile Ala Val Glu Trp Glu Ser Ser Gly Gln Pro Glu Asn Asn
305 310 315 320

Tyr Asn Thr Thr Pro Pro Met Leu Asp Ser Asp Gly Ser Phe Phe Leu
325 330 335

Tyr Ser Lys Leu Thr Val Asp Lys Ser Arg Trp Gln Gln Gly Asn Ile
340 345 350

Phe Ser Cys Ser Val Met His Glu Ala Leu His Asn Arg Phe Thr Gln
355 360 365

Lys Ser Leu Ser Leu Ser Pro Gly Lys
370 375

<210> 69
<211> 327
<212> PRT
<213> Artificial sequence

<220>
<223> an artificially synthesized sequence

<400> 69

Ala Ser Thr Lys Gly Pro Ser Val Phe Pro Leu Ala Pro Cys Ser Arg
1 5 10 15

Ser Thr Ser Glu Ser Thr Ala Ala Leu Gly Cys Leu Val Lys Asp Tyr
20 25 30

Phe Pro Glu Pro Val Thr Val Ser Trp Asn Ser Gly Ala Leu Thr Ser
35 40 45

```
Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Lys Thr
65                  70                  75                  80

Tyr Thr Cys Asn Val Asp His Lys Pro Ser Asn Thr Lys Val Asp Lys
                85                  90                  95

Arg Val Glu Ser Lys Tyr Gly Pro Pro Cys Pro Ser Cys Pro Ala Pro
            100                 105                 110

Glu Phe Leu Gly Gly Pro Ser Val Phe Leu Phe Pro Pro Lys Pro Lys
        115                 120                 125

Asp Thr Leu Met Ile Ser Arg Thr Pro Glu Val Thr Cys Val Val Val
    130                 135                 140

Asp Val Ser Gln Glu Asp Pro Glu Val Gln Phe Asn Trp Tyr Val Asp
145                 150                 155                 160

Gly Val Glu Val His Asn Ala Lys Thr Lys Pro Arg Glu Glu Gln Phe
                165                 170                 175

Asn Ser Thr Tyr Arg Val Val Ser Val Leu Thr Val Leu His Gln Asp
            180                 185                 190

Trp Leu Asn Gly Lys Glu Tyr Lys Cys Lys Val Ser Asn Lys Gly Leu
        195                 200                 205

Pro Ser Ser Ile Glu Lys Thr Ile Ser Lys Ala Lys Gly Gln Pro Arg
    210                 215                 220

Glu Pro Gln Val Tyr Thr Leu Pro Pro Ser Gln Glu Glu Met Thr Lys
225                 230                 235                 240

Asn Gln Val Ser Leu Thr Cys Leu Val Lys Gly Phe Tyr Pro Ser Asp
                245                 250                 255

Ile Ala Val Glu Trp Glu Ser Asn Gly Gln Pro Glu Asn Asn Tyr Lys
            260                 265                 270

Thr Thr Pro Pro Val Leu Asp Ser Asp Gly Ser Phe Phe Leu Tyr Ser
        275                 280                 285

Arg Leu Thr Val Asp Lys Ser Arg Trp Gln Glu Gly Asn Val Phe Ser
    290                 295                 300
```

```
Cys Ser Val Met His Glu Ala Leu His Asn His Tyr Thr Gln Lys Ser
305                 310                 315                 320

Leu Ser Leu Ser Leu Gly Lys
                325
```